# EUROPEAN PATENT SPECIFICATION

(11) **EP 3 986 430 B1**
(45) Date of publication and mention of the grant of the patent: **06.08.2025**
(21) Application number: 20825845.9
(22) Date of filing: 19.06.2020
(51) Int. Cl.: A61K 35/51, A61K 35/545, A61P 17/02, A61P 9/02, C12N 5/0797, A61K 35/50, A61K 35/12, A61K 31/728, A61K 38/17

(54) **METHODS OF PROMOTING VASCULOGENESIS**
VERFAHREN ZUR FÖRDERUNG DER VASKULOGENESE
MÉTHODES DE PROMOTION DE LA VASCULOGENÈSE

(30) Priority: 20.06.2019 US 201962864379 P
(43) Date of publication of application: 27.04.2022
(73) Proprietor: BioTissue Holdings Inc., Miami, FL 33126 (US)
(72) Inventor: TSENG, Scheffer, Miami, Florida 33126 (US); YOUNG, Frank, Miami, Florida 33126 (US); CHEN, Szu Yu, Miami, Florida 33126 (US); ZHU, Ying-Tieng, Miami, Florida 33126 (US)
(74) Representative: HGF
(86) International application number: PCT/US2020/038698
(87) International publication number: WO 2020/257626

(56) References cited:
- WO-A1-2020/097251
- US-A1- 2015 166 624
- US-A1- 2015 275 180
- ZHU YING-TING ET AL: "HC-HA/PTX3 Purified From Human Amniotic Membrane Reverts Human Corneal Fibroblasts and Myofibroblasts to Keratocytes by Activating BMP Signaling", INVESTIGATIVE OPTHALMOLOGY & VISUAL SCIENCE, vol. 61, no. 5, 28 May 2020 (2020-05-28), US, pages 62, XP093046178, ISSN: 1552-5783, DOI: 10.1167/iovs.61.5.62
- S. ZHANG ET AL: "Constitutive Expression of Pentraxin 3 (PTX3) Protein by Human Amniotic Membrane Cells Leads to Formation of the Heavy Chain (HC)-Hyaluronan (HA)-PTX3 Complex", JOURNAL OF BIOLOGICAL CHEMISTRY, vol. 289, no. 19, 9 May 2014 (2014-05-09), US, pages 13531 - 13542, XP055330585, ISSN: 0021-9258, DOI: 10.1074/jbc.M113.525287
- SCHEFFER TSENG C.G.: "Niche Regulation of Limbal Epithelial Stem Cells: Relationship between Inflammation and Regeneration", THE OCULAR SURFACE, vol. 14, no. 2, 1 April 2016 (2016-04-01), XP093046194
- CHEN SZU-YU ET AL: "HC-HA/PTX3 Purified From Amniotic Membrane Promotes BMP Signaling in Limbal Niche Cells to Maintain Quiescence of Limbal Epithelial Progenitor/Stem Cells", STEM CELLS, vol. 33, no. 11, 16 July 2015 (2015-07-16), pages 3341 - 3355, XP055938568, ISSN: 1066-5099, Retrieved from the Internet <URL:https://onlinelibrary.wiley.com/doi/full-xml/10.1002/stem.2091> DOI: 10.1002/stem.2091
- TSENG ET AL: "Reprogramming By A Novel Matrix Component HC-HA/PTX3 Purified From Amniotic Membrane", ASIA-ARVO. FEB 16-19, 2015; YOKOHAMA, JAPAN. PLENARY SESSION 6, RECENT TRENDS IN REGENERATIVE MEDICINE; EVOLUTIONS IN OPHTHALMOLOGY: FROM THE STUDY OF AGING AND REGENERATIVE MEDICINE TO QUALITY OF VISION,, 18 February 2015 (2015-02-18), pages 158, XP009529379
- SCHEFFER C. G. TSENG: "HC-HA/PTX3 Purified From Amniotic Membrane as Novel Regenerative Matrix: Insight Into Relationship Between Inflammation and Regeneration", INVESTIGATIVE OPTHALMOLOGY & VISUAL SCIENCE, vol. 57, no. 5, 26 April 2016 (2016-04-26), US, pages ORSFh1, XP055706420, ISSN: 1552-5783, DOI: 10.1167/iovs.15-17637

## Description

### STATEMENT REGARDING FEDEARLLY SPONSORED RESEARCH

This invention was made with the support of the United States government under Contract number RO1 EY06819 by National Institutes of Health.

### Background

US2015166624A1 discloses methods for the production of native and reconstituted hyaluronan (HA) complexes containing pentraxin-3 (PTX3) and heavy chain 1 (HC1) of inter alpha inhibitor (IαI). Also disclosed are methods of reprogramming a cell having a first phenotype comprising contacting the cell with HC-HA/PTX3 for a time sufficient to reprogram the first phenotype of the cell to a second phenotype.
US2015/0275180 discloses use of hyaluronic acid as a carrier for butyric and retinoic acid (HBR) differentiation agents in a chemical epigenetic reprogramming to induce pluripotency.
WO2020097251A1 discloses a method of reprogramming a cell having a first phenotype, comprising: contacting the cell with HC-HA/PTX3 for a time sufficient to reprogram the first phenotype of the cell to a second phenotype.

### Summary of Disclosure

In a first aspect of the invention there is provided a fetal support tissue product for use in the therapeutic promotion of vasculogenesis and/or neurovasculogenesis of a tissue, wherein neurovasculogenesis comprises neurogenesis, wherein the tissue comprises endothelial cells and pericytes, wherein the fetal support tissue product comprises native heavy chain-hyaluronan/pentraxin 3 (HC-HA/PTX3) complex, reconstituted HC-HA/PTX3 complex, or a combination thereof, wherein the fetal support tissue product reprograms the pericytes to a first progenitor phenotype and reprograms the endothelial cells to a second progenitor phenotype.
In certain embodiments, the tissue in need of vasculogenesis and/or neurovasculogenesis comprises an ulcer, a wound, a perforation, a burn, a surgical incision, an injury, a fistula, a degenerated tissue, a bone, a tendon, a nerve, a ligament, an intervertebral disc, cardiac tissue, cartilage, an ischemic tissue, ischemic condition comprising cardiac ischemia, ischemic colitis, mesenteric ischemia, brain ischemia, acute limb ischemia, cyanosis, gangrene, neuropathic, or a neurotrophic condition.
In certain embodiments, the tissue further comprises neural crest progenitor cells.
In certain embodiments, wherein the use prevents necrosis of the tissue.
In certain embodiments, the fetal support tissue product comprises umbilical cord that is substantially free of a vein or artery.
In certain embodiments, the use promotes apoptosis, necrosis, or a combination thereof.
In certain embodiments, the fetal support tissue product comprises native HC-HA/PTX3 complex (nHC-HA/PTX3).
In certain embodiments, the use comprises use of reconstituted HC-HA/PTX3 complex (rcHC-HA/PTX3).
In certain embodiments, the rcHC-HA/PTX3 complex consists of high molecular weight (HMW) HA, HC1, HC2 and PTX3.
In certain embodiments, the fetal support tissue product, the nHC-HA/PTX3 complex or the rcHC-HA/PTX3 complex comprises a pharmaceutically acceptable excipient, carrier, or combination thereof.
In certain embodiments, the fetal support tissue product is from placenta, placental amniotic membrane, umbilical cord, umbilical cord amniotic membrane, chorion, amnion-chorion, amniotic stroma, amniotic jelly, Wharton's jelly, amniotic fluid, or a combination thereof.
In certain embodiments, the fetal support tissue product is isolated from a fetal support tissue that is frozen or previously frozen.
In certain embodiments, the fetal support tissue product is ground, pulverized, morselized, a graft, a sheet, micronized, a powder, a homogenate, or an extract.
In certain embodiments, wherein the fetal support tissue product comprises umbilical cord amniotic membrane (UCAM).
In certain embodiments, UCAM further comprises Wharton's jelly.
The invention is defined in the claims. Any of the following figures that do not fall within the scope of the claims are provided for illustrative or comparative purposes only.

### BRIEF DESCRIPTION OF THE DRAWINGS

The novel features of the disclosure set forth with particularity in the appended claims. A better understanding of the features and advantages of the disclosure will be obtained by reference to the following detailed description that sets forth illustrative embodiments, in which the principles of the disclosure are utilized, and the accompanying drawings of which:

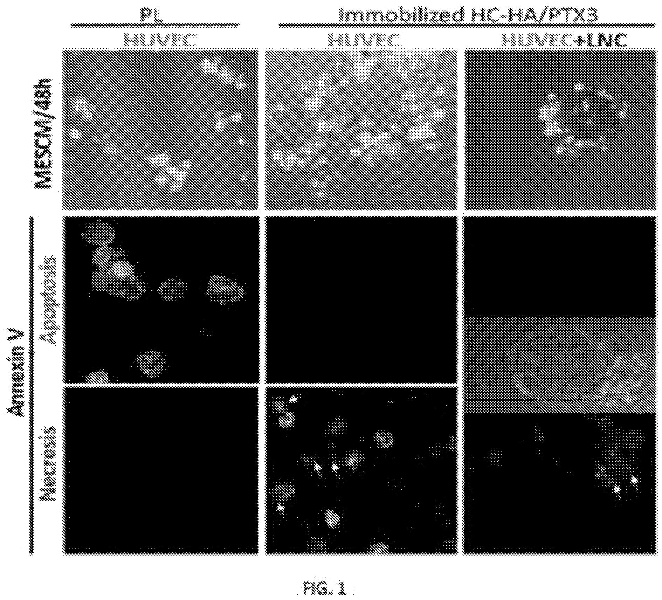

**FIG. 1** illustrates apoptotic and necrotic effect of immobilized HC-HA/PTX3 on HUVEC with or without LNCs.

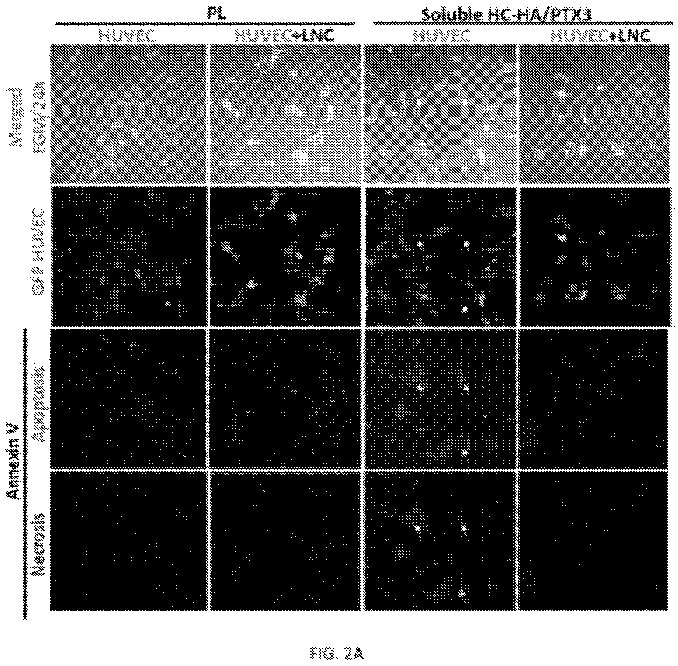

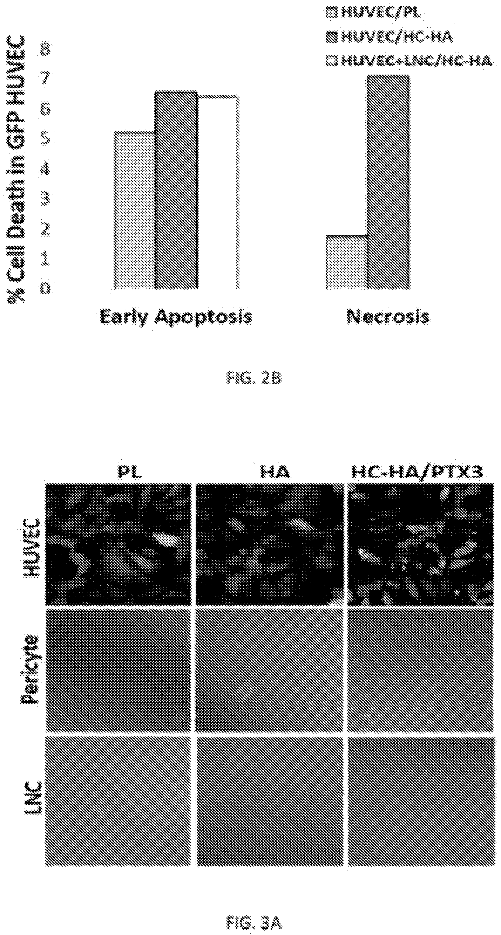

**FIGS. 2A-2B** illustrate apoptosis effect of soluble HC-HA/PTX3/4P on GFP HUVEC with or without P4 LNCs on plastic. **FIG. 2A** illustrates immunofluorescence staining to detect apoptosis and necrosis. **FIG. 2B** illustrates percentage of apoptosis and necrosis.

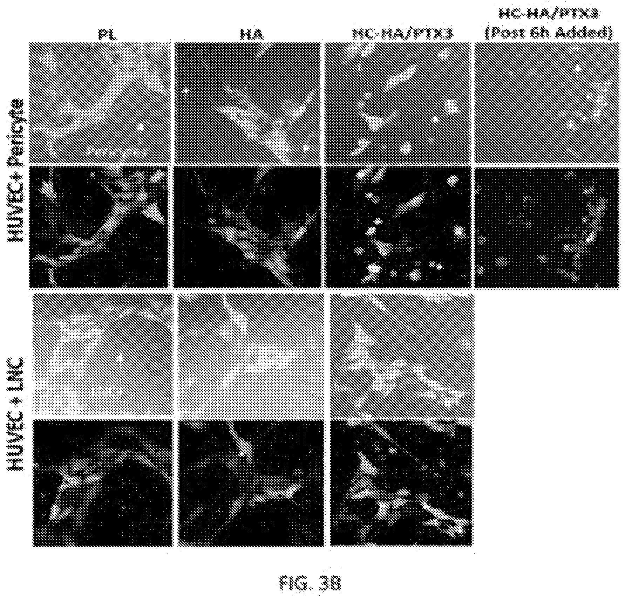

**FIGS. 3A-3B** illustrate immunofluorescence staining to detect apoptosis. **FIG. 3A** illustrates immunofluorescence staining to detect apoptosis in HUVEC, pericyte, and LNC. **FIG. 3B** illustrates immunofluorescence staining following simultaneous or sequential addition of HC-HA/PTX3 to detect apoptosis in HUVEC + pericyte and HUVEC + LNC.

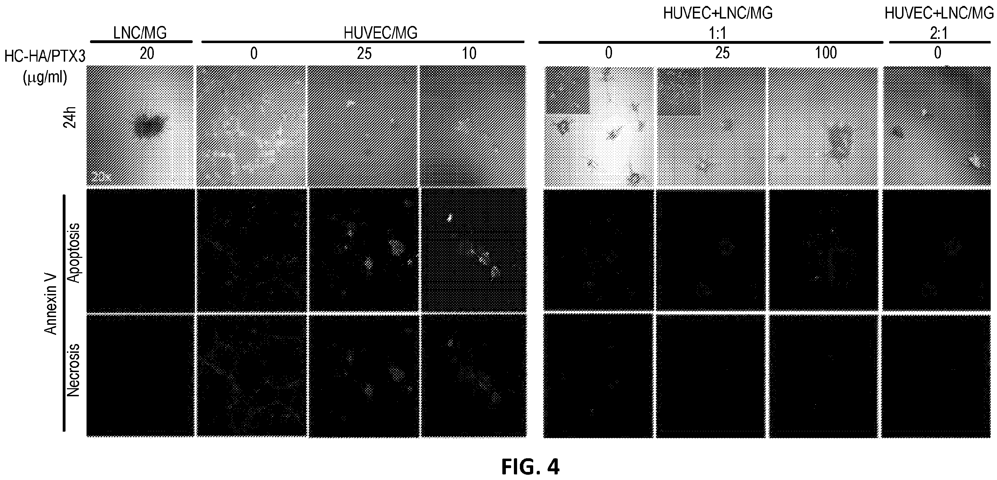

**FIG. 4** illustrates apoptosis effect of soluble HC-HA/PTX3/4P on GFP-HUVEC with or without LNC on Matrigel^{™}.

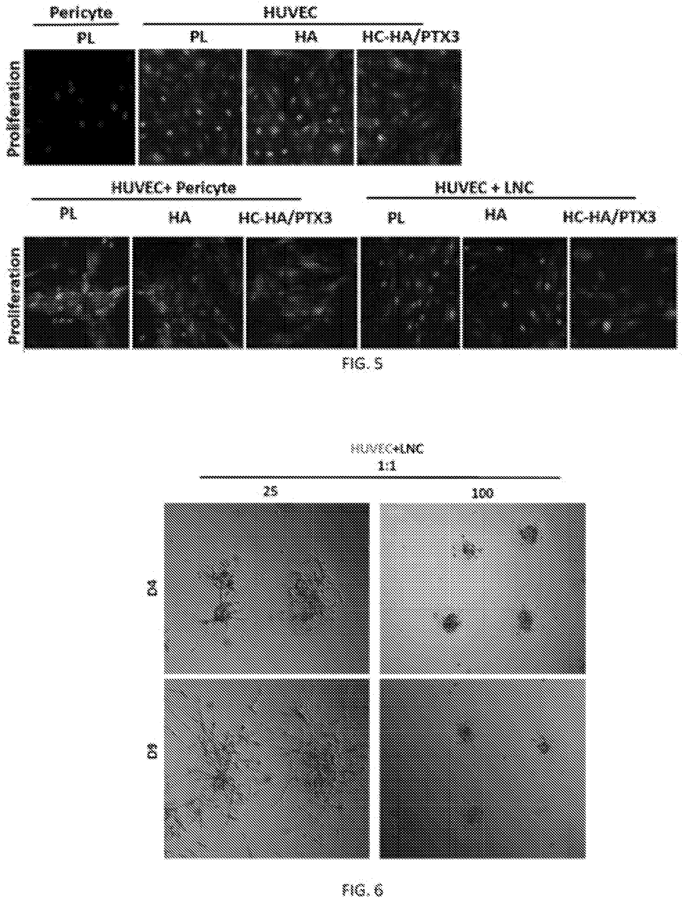

**FIG. 5** illustrates soluble HC-HA/PTX3 promotes quiescence of LNC when co-cultured with GFP-HUVEC on coated Matrigel^{™}.
**FIG. 6** illustrates the reunion of GFP-HUVEC and LNC resulting in growth of sprout-like LNC at a low dosage of HC-HA/PTX3 (2ug/ml) but inhibited growth at a higher dosage (100 ug/ml).

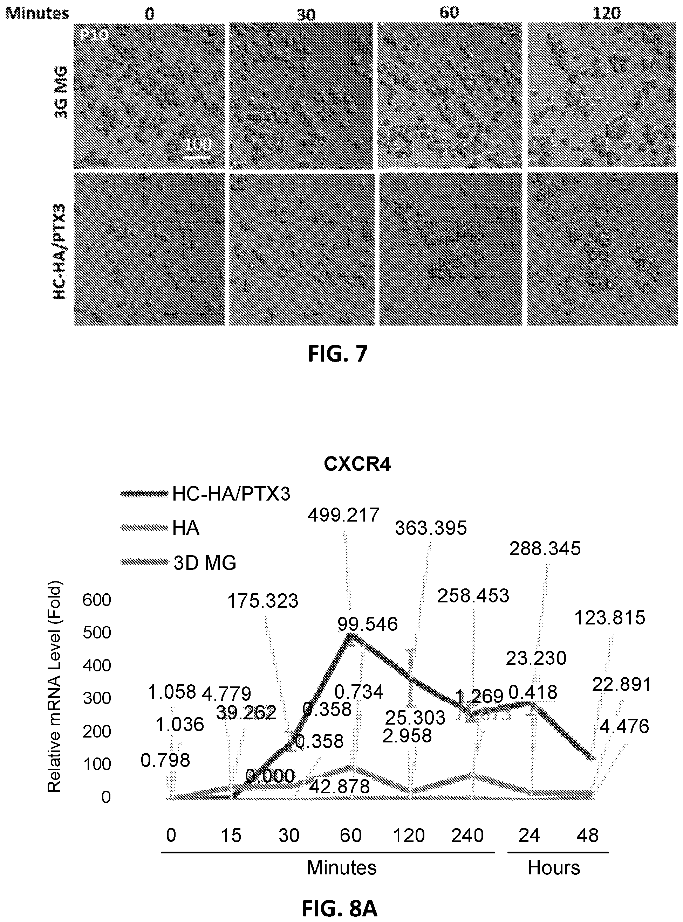

**FIG. 7** illustrates HC-HA/PTX3 promotes the early sphere formation at 60 min in P10 LNC.

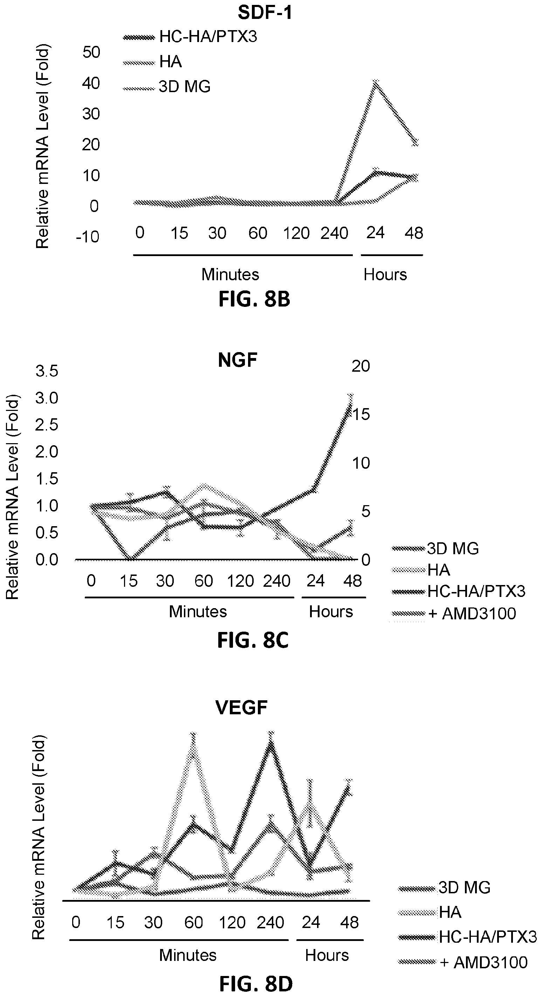

**FIGS. 8A-8D** illustrate time course mRNA expression on HC-HA/PTX3, HA, or 3D Matrigel^{™}. **FIG. 8A** illustrates time course mRNA expression of CXCR4. **FIG. 8B** illustrate time course mRNA expression of SDF-1. **FIG. 8C** illustrate time course mRNA expression of NGF. **FIG. 8D** illustrate time course mRNA expression of VEGF.

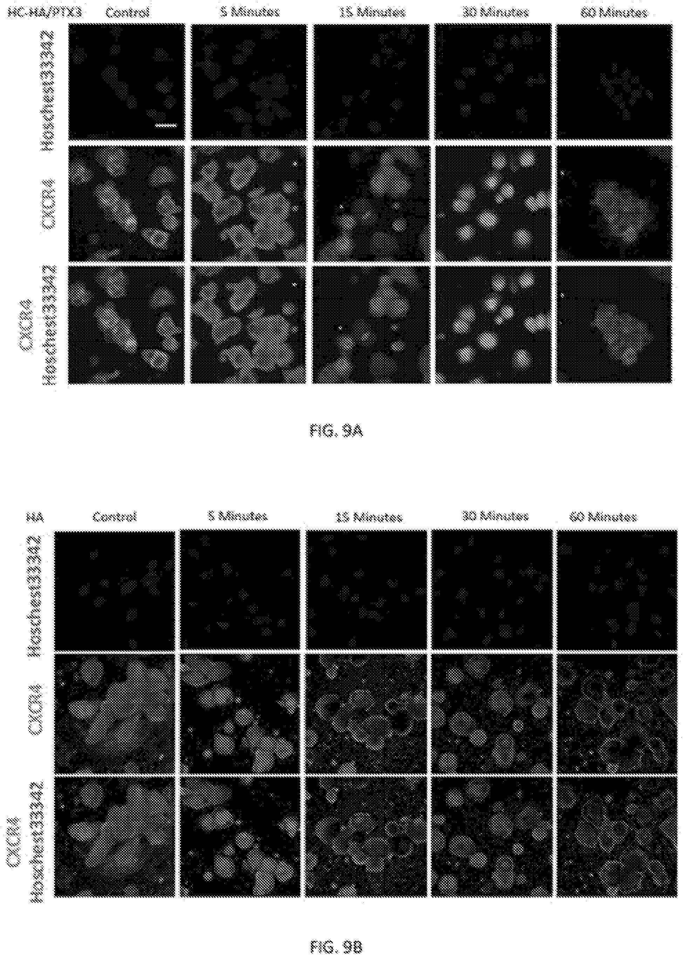

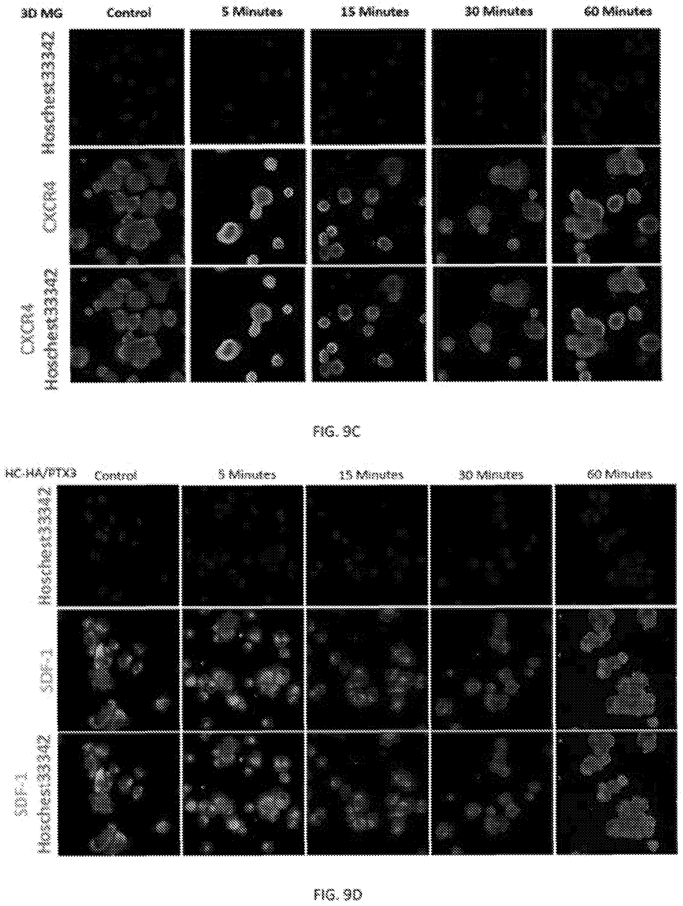

**FIGS. 9A-9D** illustrate immunofluorescence staining confirming cytoplasmic/nucleus expression of CXCR4 and SDF-1. **FIG. 9A** illustrates immunofluorescence staining of CXCR4 following exposure to HC-HA/PTX3. **FIG. 9B** illustrates immunofluorescence staining of CXCR4 following exposure to HA. **FIG. 9C** illustrates immunofluorescence staining of CXCR4 on 3D Matrigel^{™} (3D MG). **FIG. 9D** illustrates immunofluorescence staining of SDF-1 following exposure to HC-HA/PTX3.

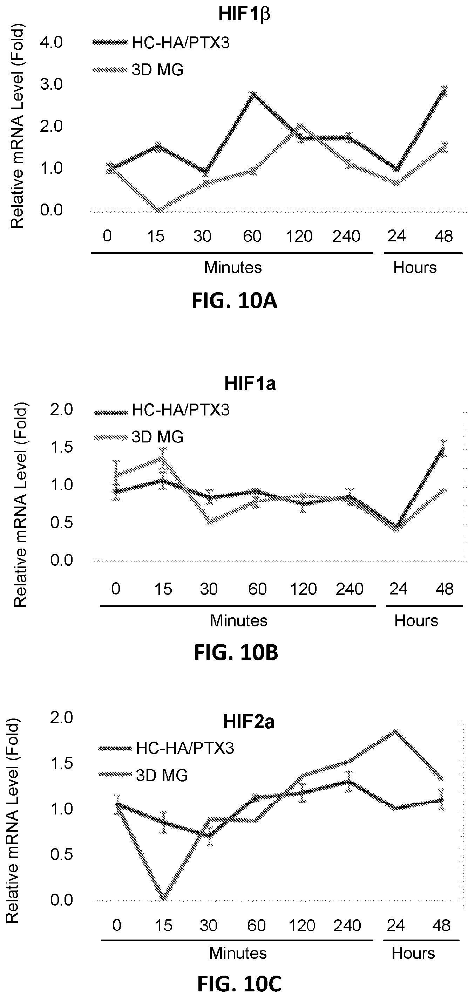

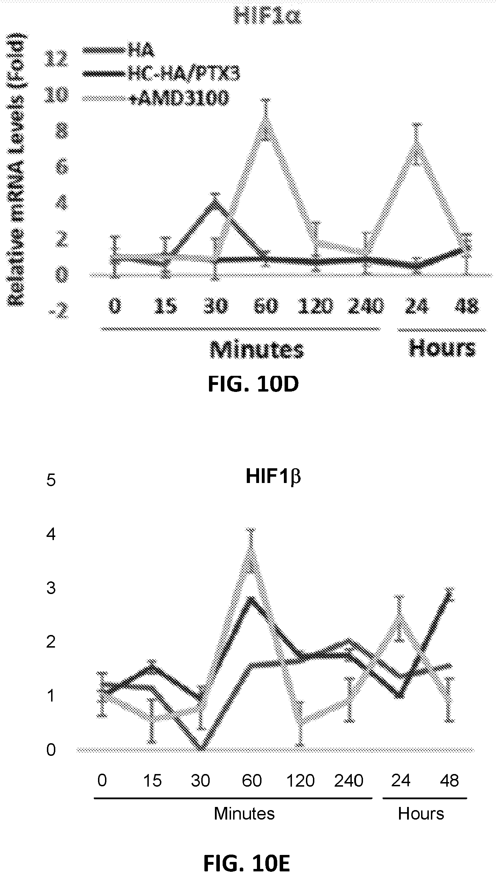

**FIGS. 10A-10E** illustrates time course mRNA expression pattern of HIF signaling. **FIG. 10A** illustrates a time course mRNA expression pattern of HIF1β. **FIG. 10B** illustrates a time course mRNA expression pattern of HIF1α. **FIG. 10C** illustrates a time course mRNA expression pattern of HIF2α. **FIG. 10D** illustrates a time course mRNA expression pattern of HIF1α. **FIG. 10E** illustrates a time course mRNA expression pattern of HIF 1β .

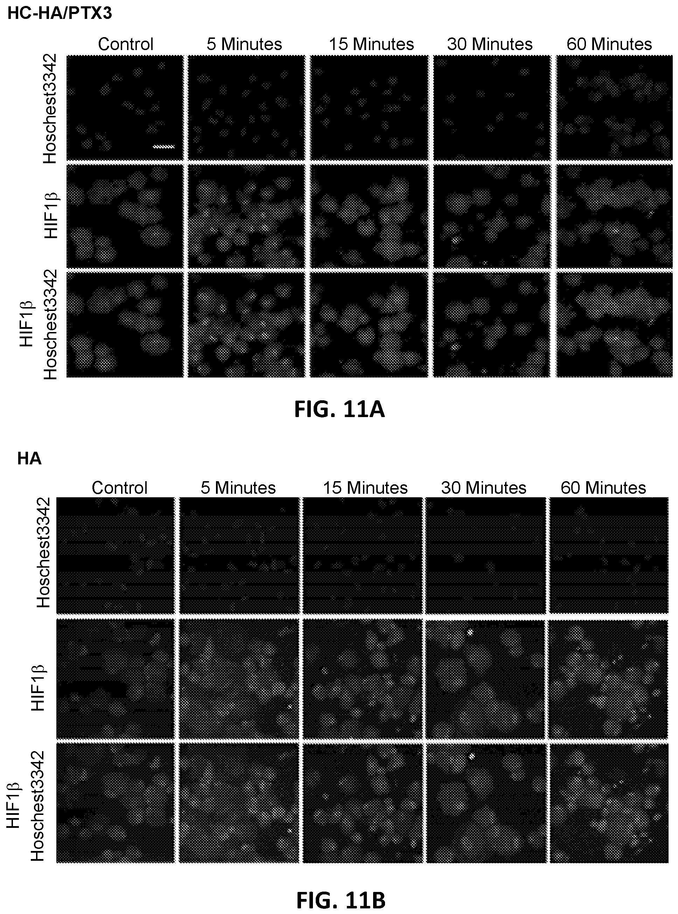

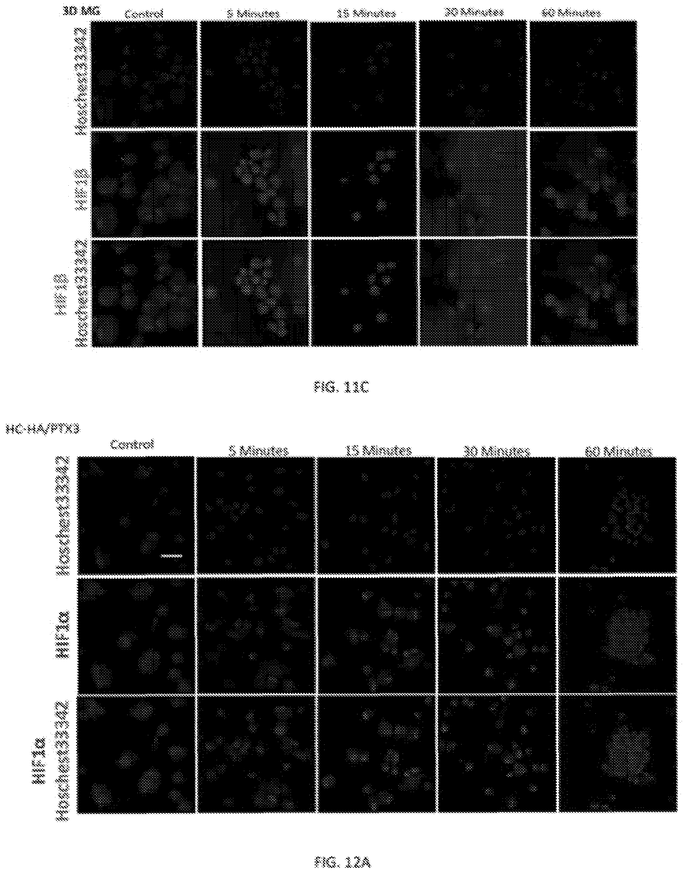

**FIGS. 11A-11C** illustrate immunofluorescence (IF) staining of HIF1β. **FIG. 11A** illustrates IF staining of HIF1β in the presence of HC-HA/PTX3. **FIG. 11B** illustrates IF staining of HIF1β in the presence of HA. **FIG. 11C** illustrates IF staining of HIF1β on 3D Matrigel^{™} (3D MG).

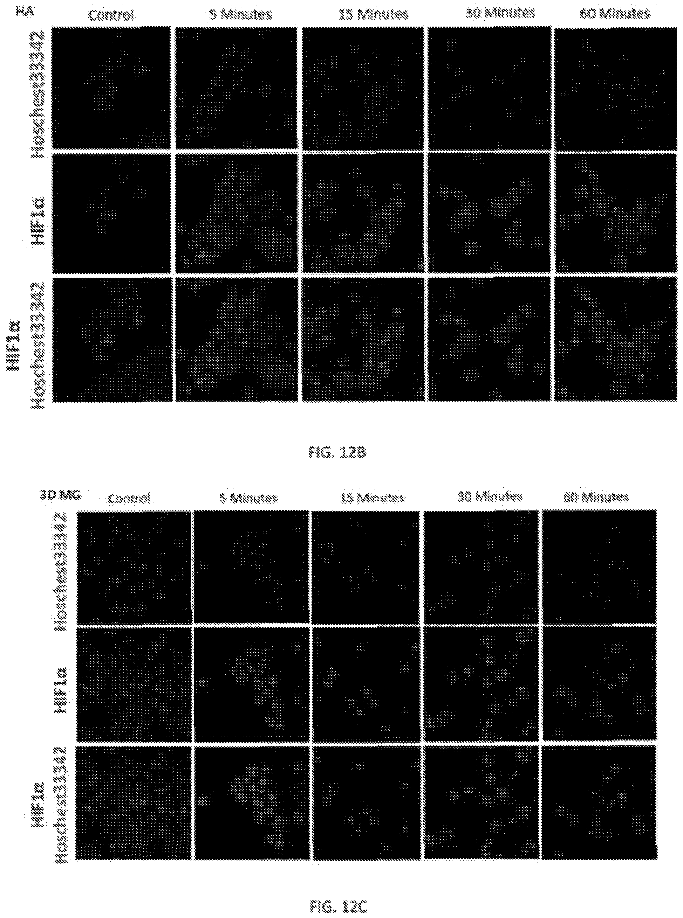

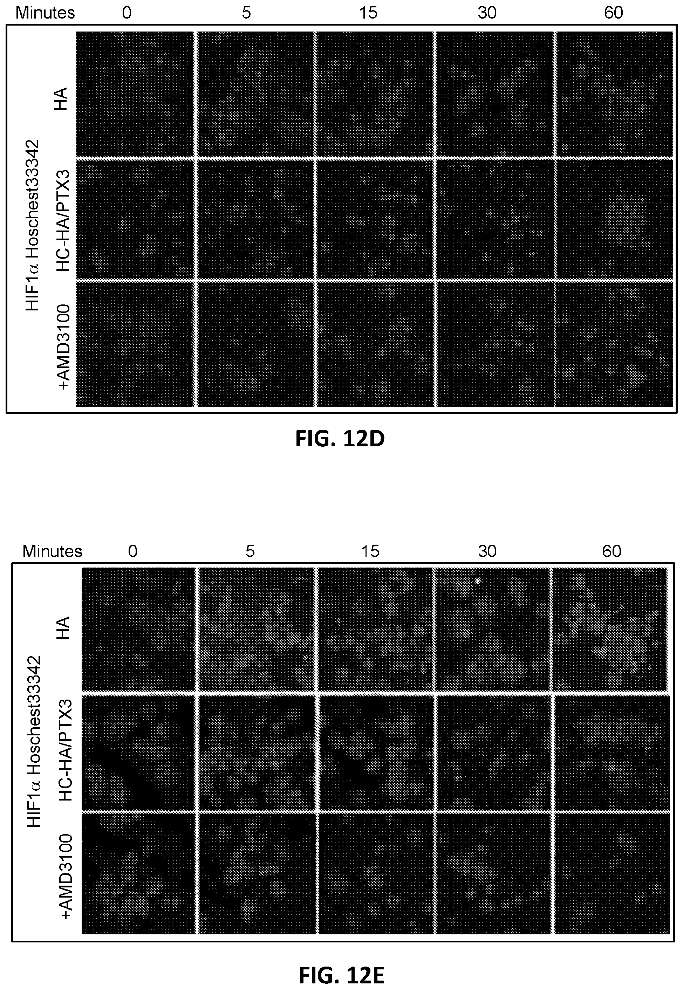

**FIGS. 12A-12E** illustrate immunofluorescence (IF) staining. **FIG. 12A** illustrates IF staining of HIF1α in the presence of HC-HA/PTX3. **FIG. 12B** illustrates IF staining of HIF1α in the presence of HA. **FIG. 12C** illustrates IF staining of HIF1α on 3D Matrigel^{™} (3D MG). **FIG. 12C** illustrates IF staining of HIF1α. **FIG. 12D** illustrates IF staining of HIF1β.

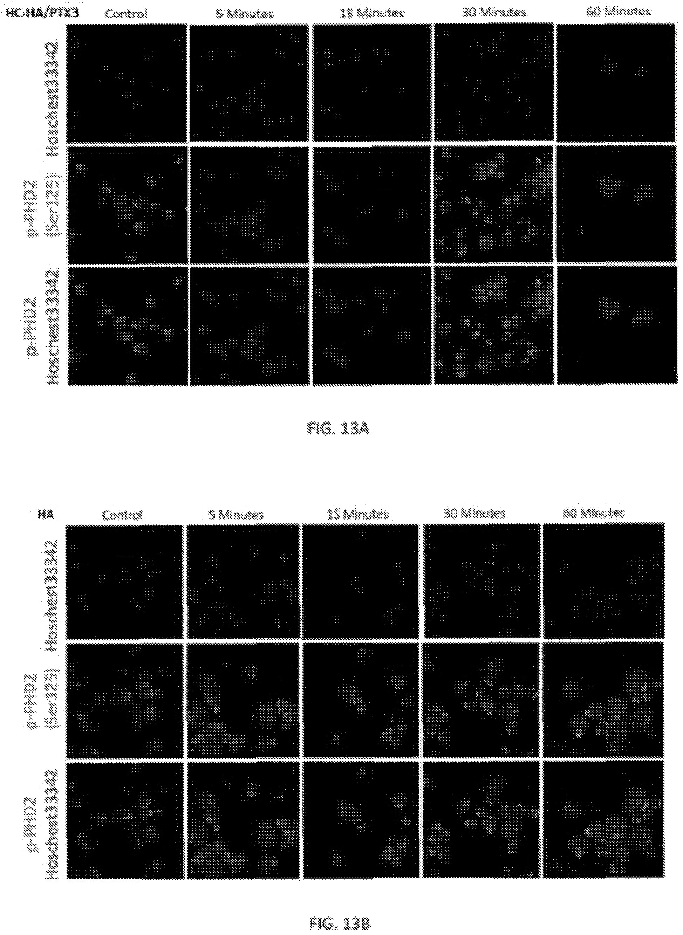

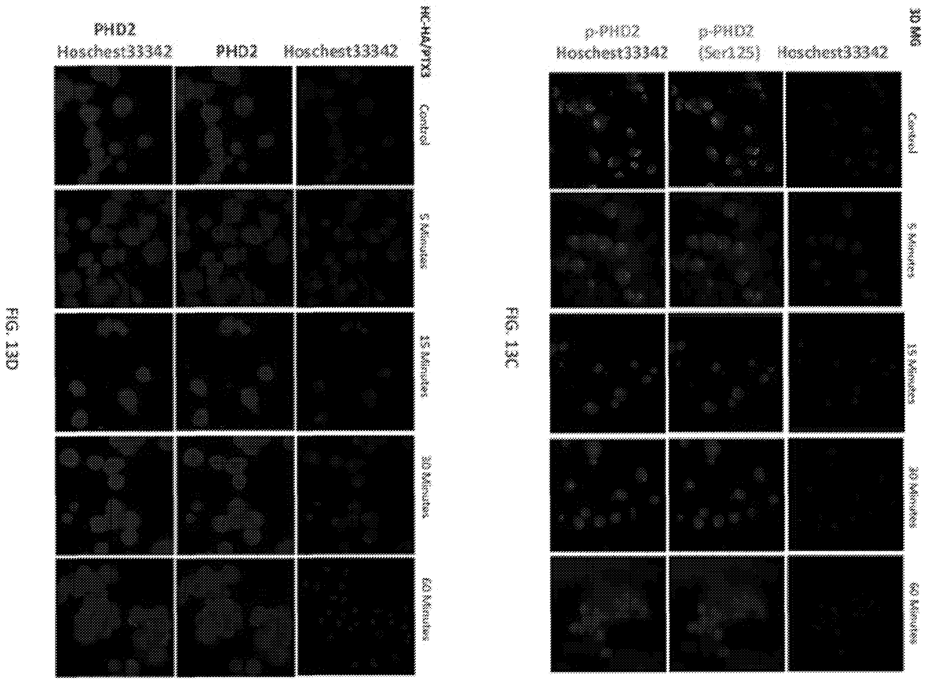

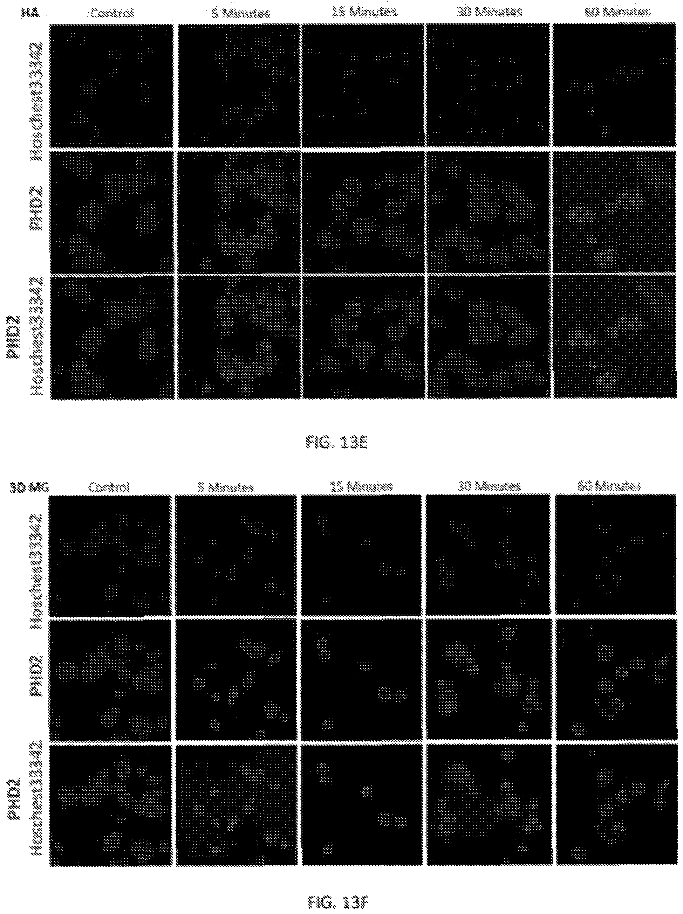

**FIGS. 13A-13F** illustrate immunofluorescence (IF) staining of phosphorylated PHD2 (p-PHD2, Ser125). **FIG. 13A** illustrates immunofluorescence staining of p-PHD2 in the presence of HC-HA/PTX3. **FIG. 13B** illustrates IF staining of phosphor-PHD2 (p-PHD2) in the presence of HA. **FIG. 13C** illustrates IF staining of p-PHD2 on 3D Matrigel^{™} (3D MG). **FIG. 13D** illustrates immunofluorescence staining of PHD2 in the presence of HC-HA/PTX3. **FIG. 13E** illustrates IF staining of PHD2 in the presence of HA. **FIG. 13F** illustrates IF staining of PHD2 on 3D Matrigel^{™} (3D MG).

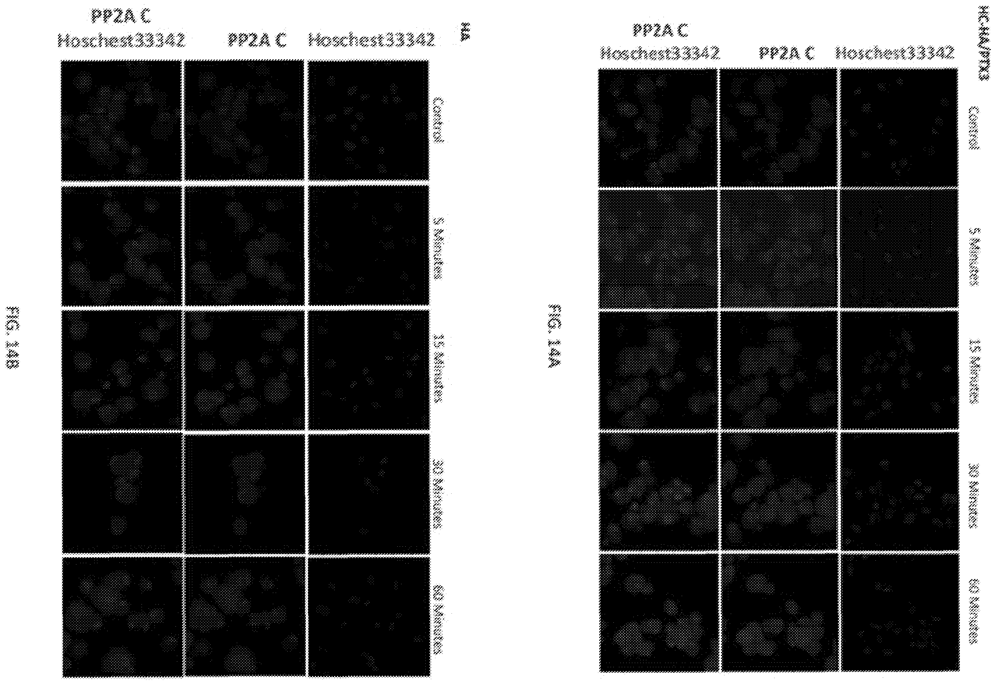

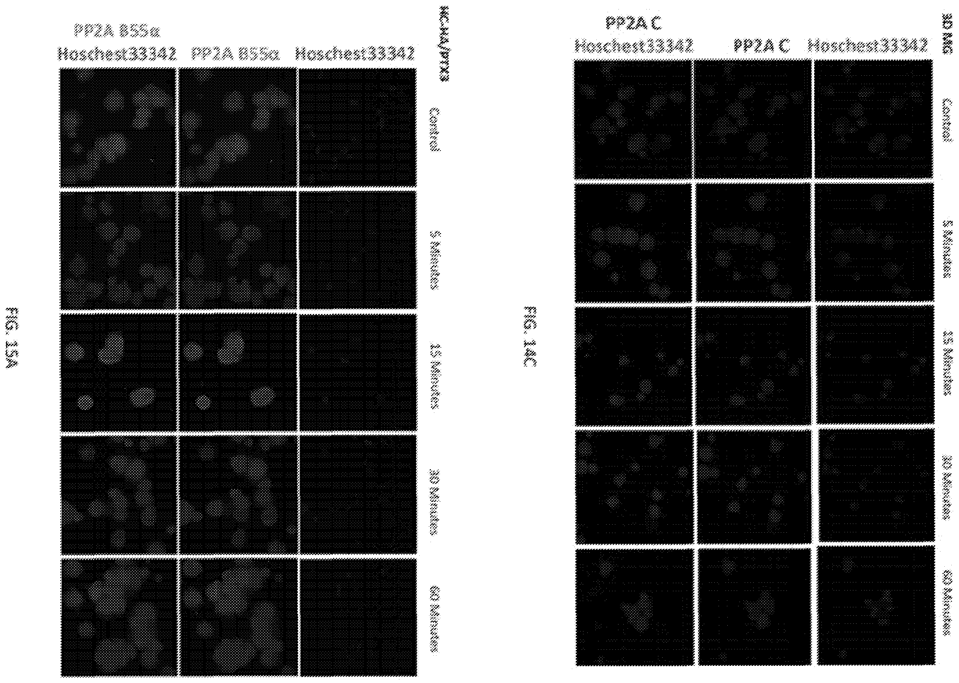

**FIGS. 14A-14C** illustrate immunofluorescence (IF) staining of PP2A C subunit. **FIG. 14A** illustrates immunofluorescence staining of PP2A C subunit in the presence of HC-HA/PTX3. **FIG. 14B** illustrates IF staining of PP2A C subunit in the presence of HA. **FIG. 13C** illustrates IF staining of PP2A C subunit on 3D Matrigel^{™} (3D MG).

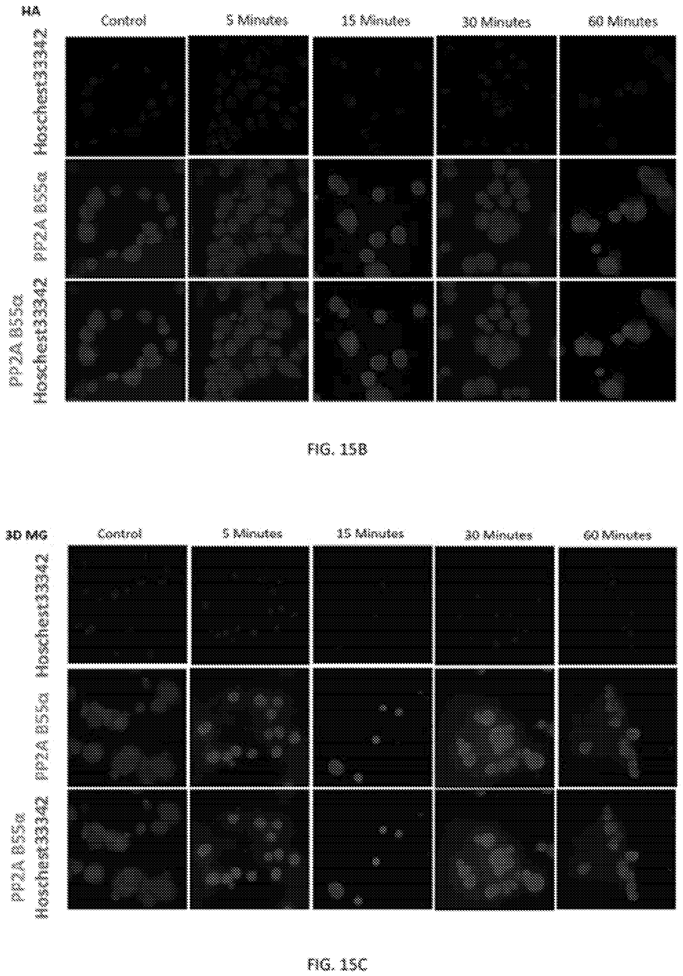

**FIGS. 15A-15C** illustrate immunofluorescence (IF) staining of PP2A B55α. **FIG. 15A** illustrates immunofluorescence staining of PP2A B55α in the presence of HC-HA/PTX3. **FIG. 15B** illustrates IF staining of PP2A B55α in the presence of HA. **FIG. 15C** illustrates IF staining of PP2A B55α on 3D Matrigel^{™} (3D MG).

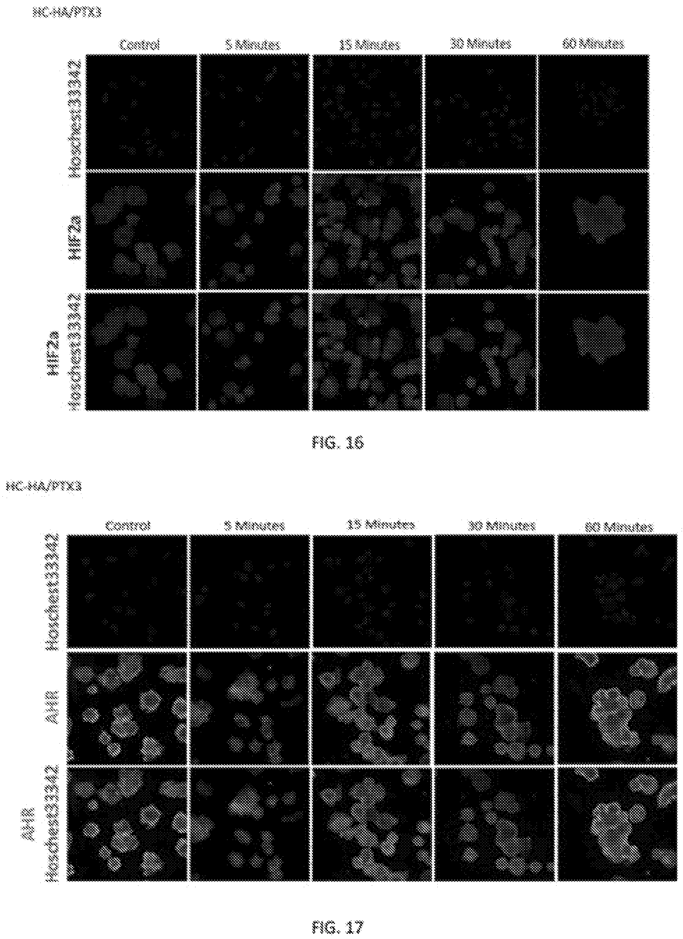

**FIG. 16** illustrates IF staining of HIF2α in the presence of HC-HA/PTX3.
**FIG. 17** illustrates IF staining of aryl hydrocarbon receptor (AHR) in the presence of HC-HA/PTX3.

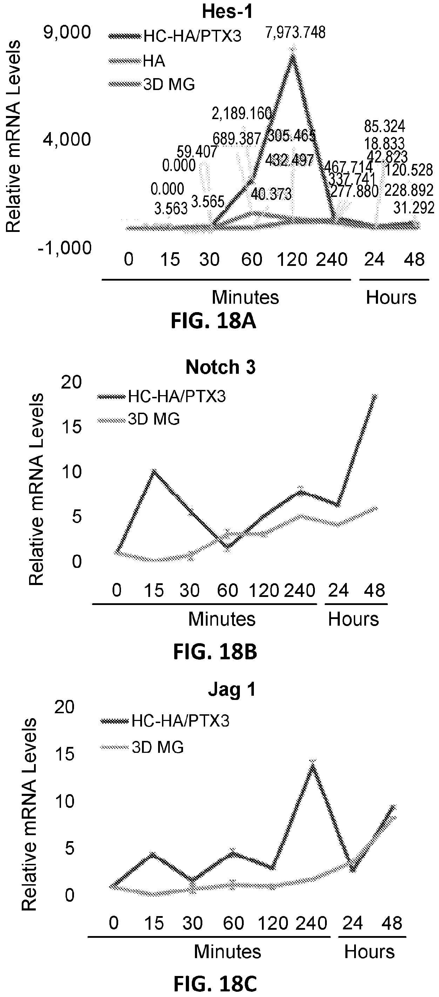

**FIGS. 18A-18C** illustrates time course mRNA expression pattern of Hes-1, Notch3, and Jag1. **FIG. 18A** illustrates a time course mRNA expression pattern of Hes-1. **FIG. 18B** illustrates a time course mRNA expression pattern of Notch3. **FIG. 18C** illustrates a time course mRNA expression pattern of Jag1.

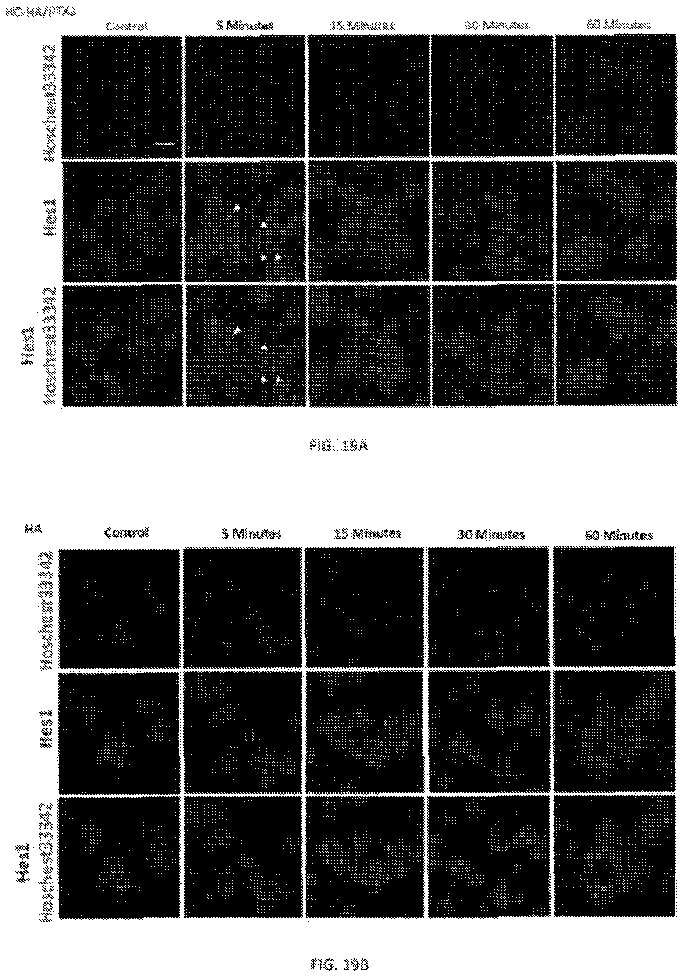

**FIGS. 19A-19B** illustrate immunofluorescence (IF) staining of Hes1. **FIG. 19A** illustrates IF staining of Hes1 in the presence of HC-HA/PTX3. **FIG. 19B** illustrates IF staining of Hes1 in the presence of HA.

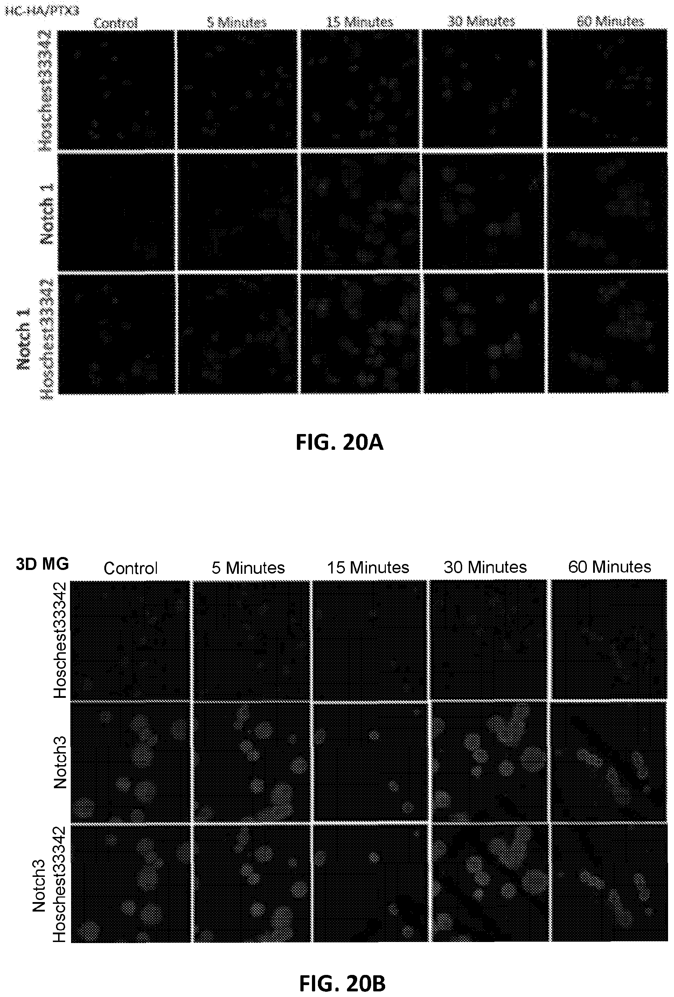

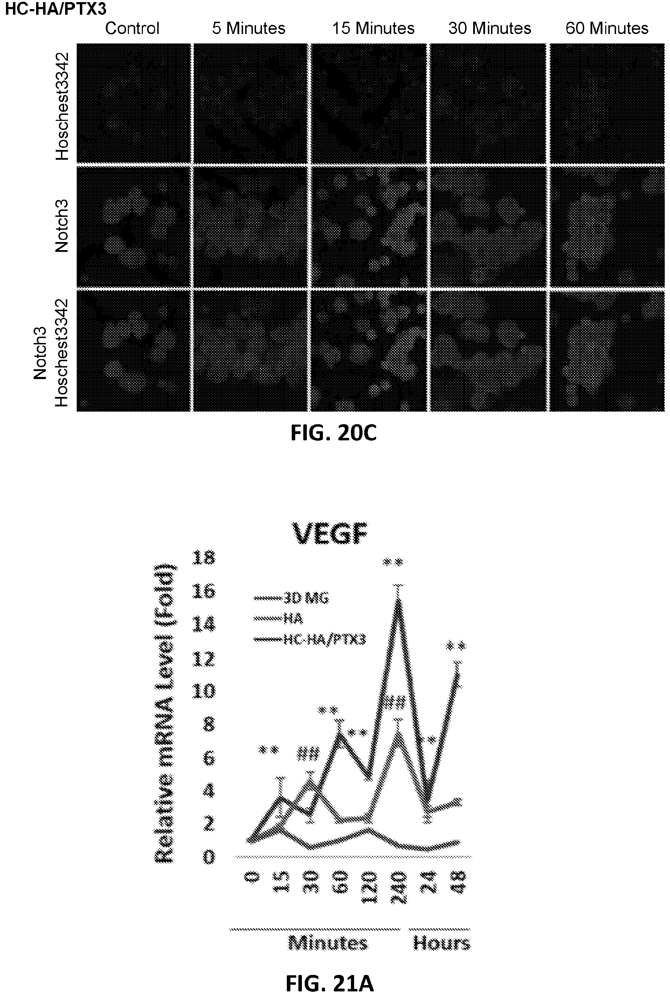

**FIGS. 20A-20C** illustrate immunofluorescence (IF) staining of Notch 1 or Notch3 . **FIG. 20A** illustrates IF staining of Notch1 in the presence of HC-HA/PTX3. **FIG. 20B** illustrates IF staining of Notch3 in the presence of 3D Matrigel (MG). **FIG. 20C** illustrates IF staining of Notch3 in the presence of HC-HA/PTX3.

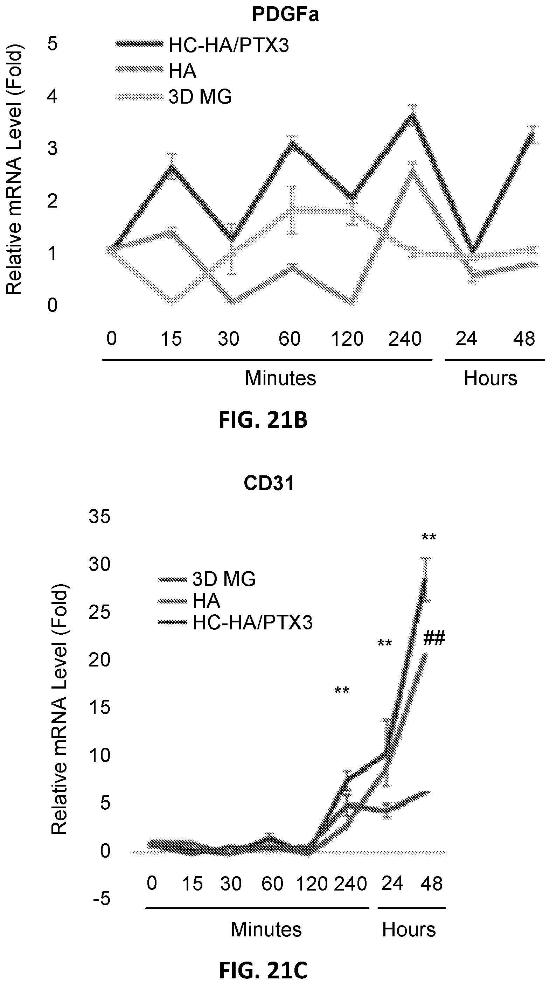

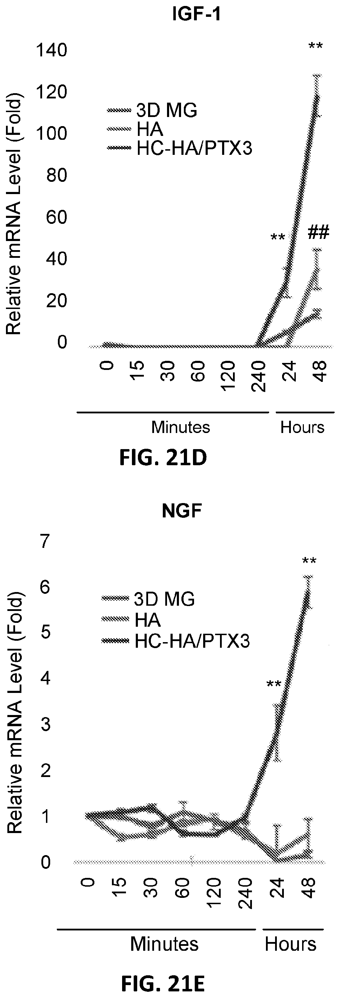

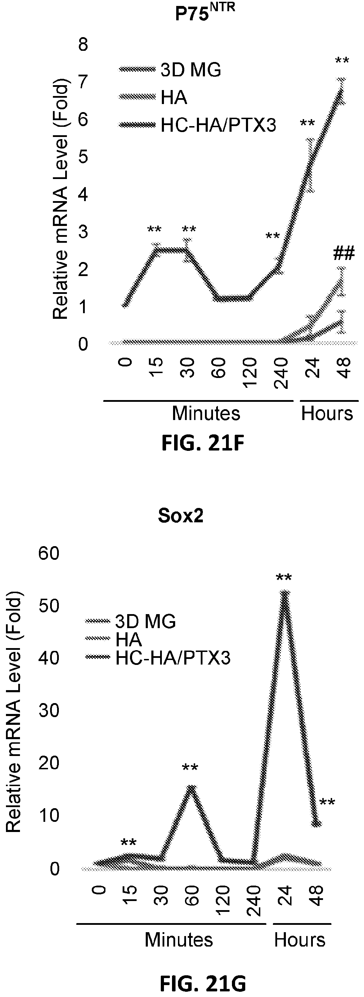

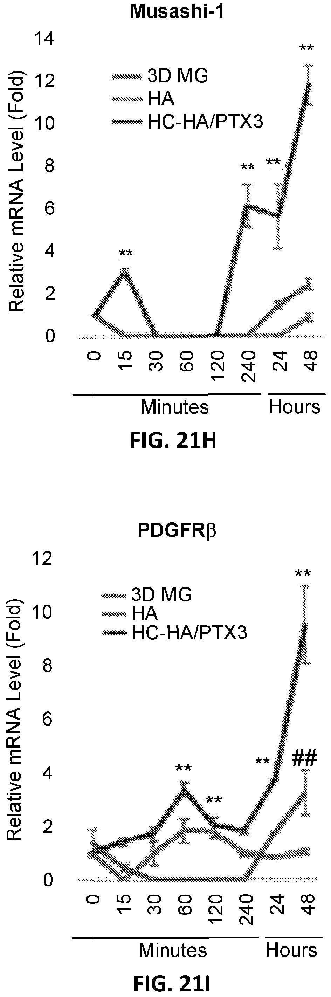

**FIGS. 21A-21F** illustrates time course mRNA expression pattern of VEGF, PDGFα, CD31, IGF-1, NGF, and p75^{NTR}. **FIG. 21A** illustrates a time course mRNA expression pattern of VEGF. **FIG. 21B** illustrates a time course mRNA expression pattern of PDGFα. **FIG. 21C** illustrates a time course mRNA expression pattern of CD31. **FIG. 21D** illustrates a time course mRNA expression pattern of IGF-1. **FIG. 21E** illustrates a time course mRNA expression pattern of NGF. **FIG. 21F** illustrates a time course mRNA expression pattern of p75^{NTR}. **FIG. 21G** illustrates a time course mRNA expression pattern of Sox2. **FIG. 21H** illustrates a time course mRNA expression pattern of Musashi-1. **FIG. 21I** illustrates a time course mRNA expression pattern of PDGFRβ.

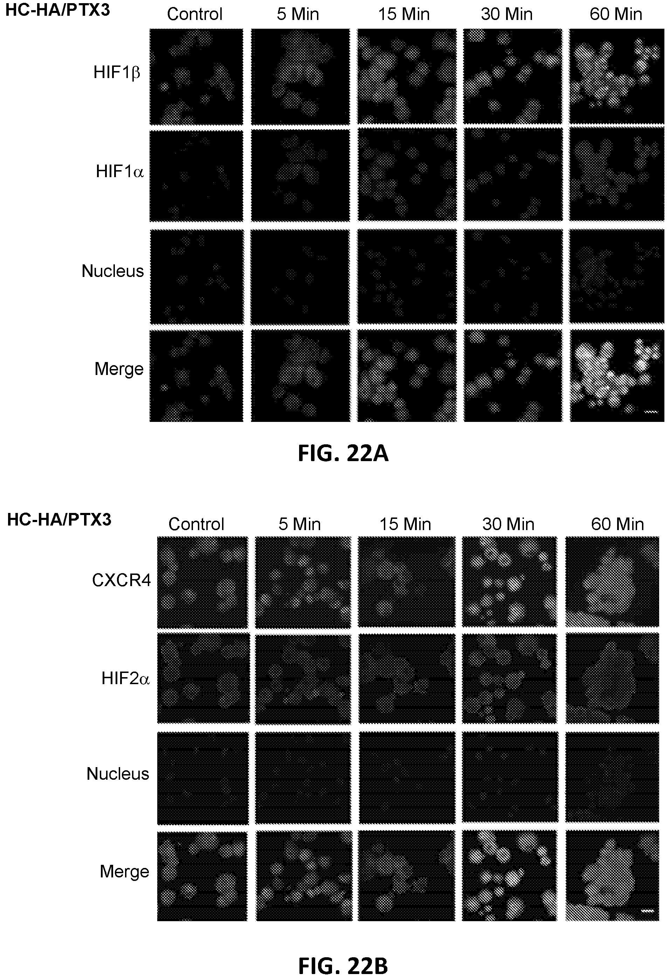

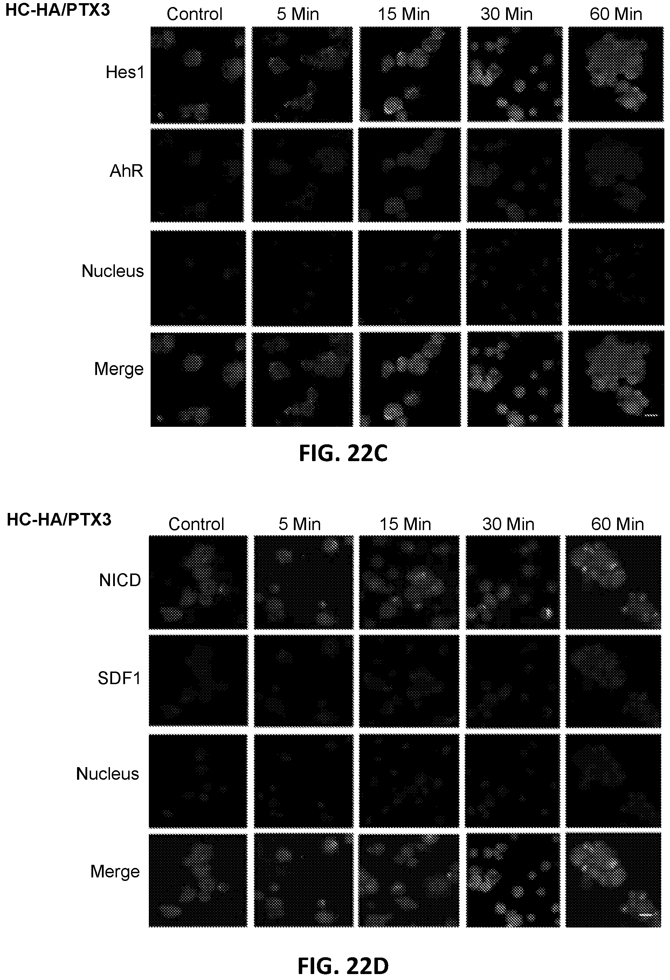

**FIGS. 22A-22D** illustrate immunofluorescence (IF) staining of HIF1α, HIF1β, CXCR4, HIF2α, Hes1, AHR, NICD, and SDF1 in P4 LNC in the presence of HC-HA/PTX3 . **FIG. 22A** illustrates immunofluorescence (IF) staining of HIF1α and HIF1β. **FIG. 22B** illustrates immunofluorescence (IF) staining of CXCR4 and HIF2α. **FIG. 22C** illustrates immunofluorescence (IF) staining of Hes1 and AHR. **FIG. 22D** illustrates immunofluorescence (IF) staining of NICD and SDF1.

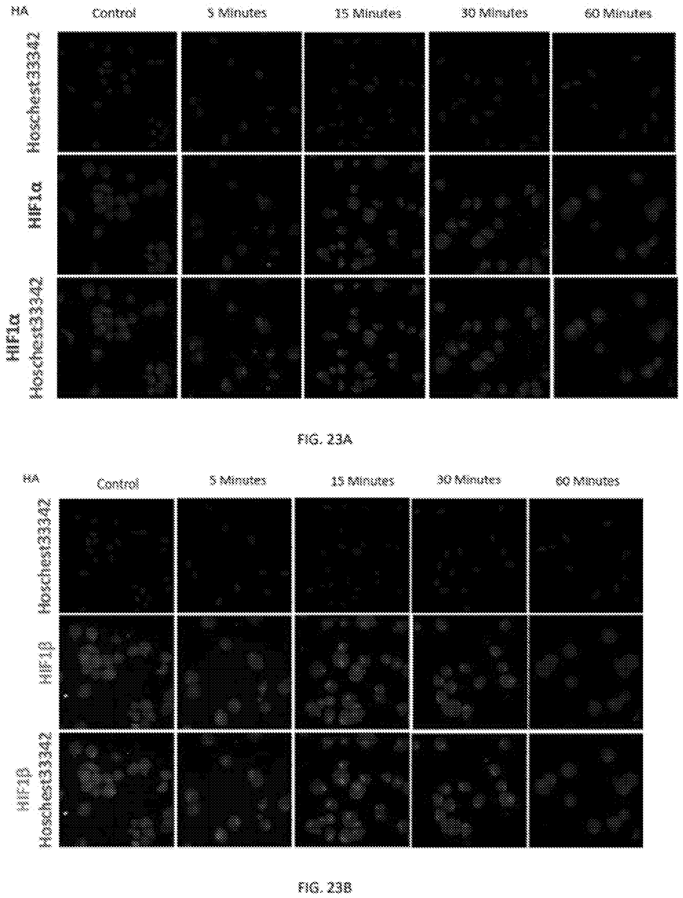

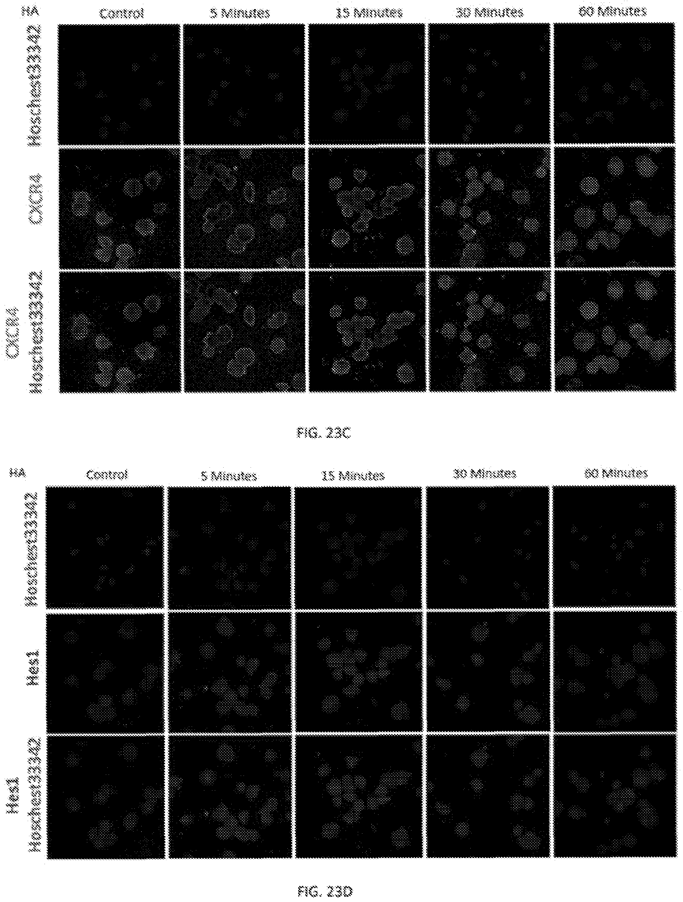

**FIGS. 23A-23D** illustrate immunofluorescence (IF) staining of HIF1α, HIF1B, CXCR4, and Hes1 in the presence of HA. **FIG. 23A** illustrates immunofluorescence (IF) staining of HIF1α. **FIG. 23B** illustrates immunofluorescence (IF) staining of HIF 1β. **FIG. 23C** illustrates immunofluorescence (IF) staining of CXCR4. **FIG. 23D** illustrates immunofluorescence (IF) staining of Hes1.

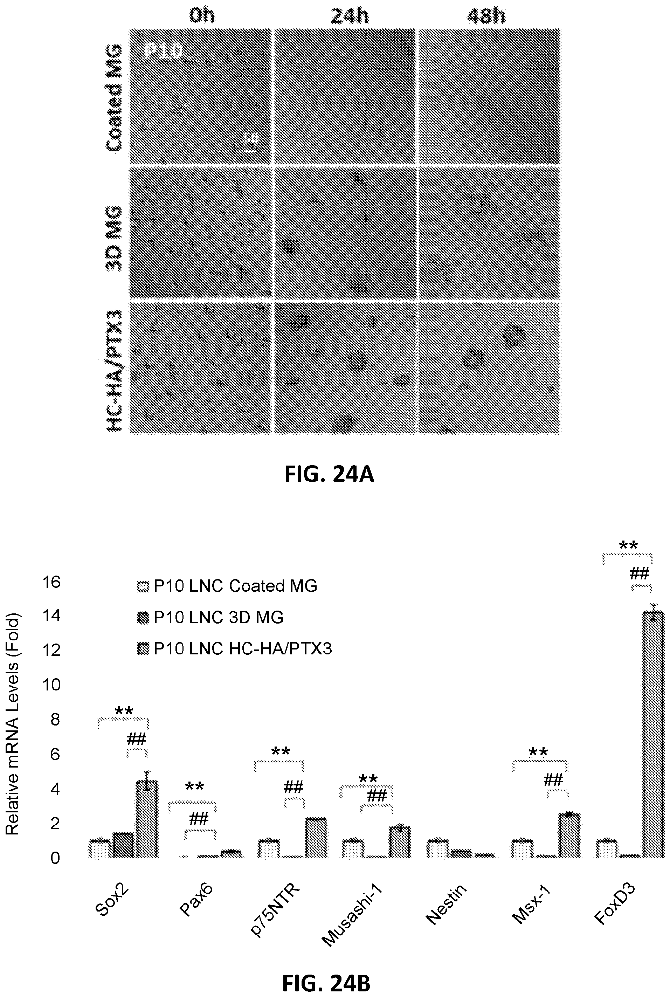

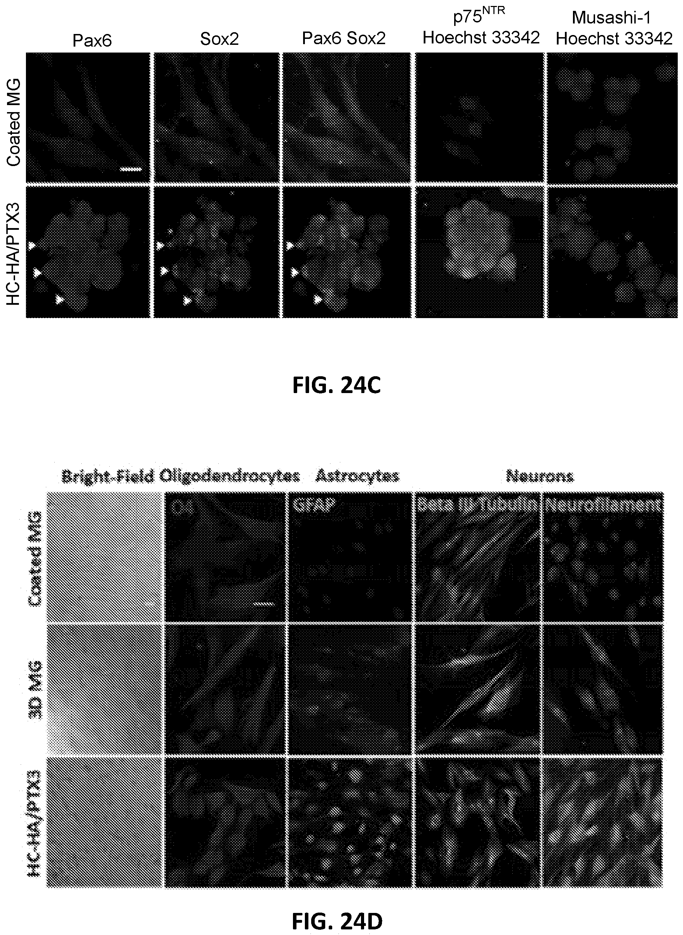

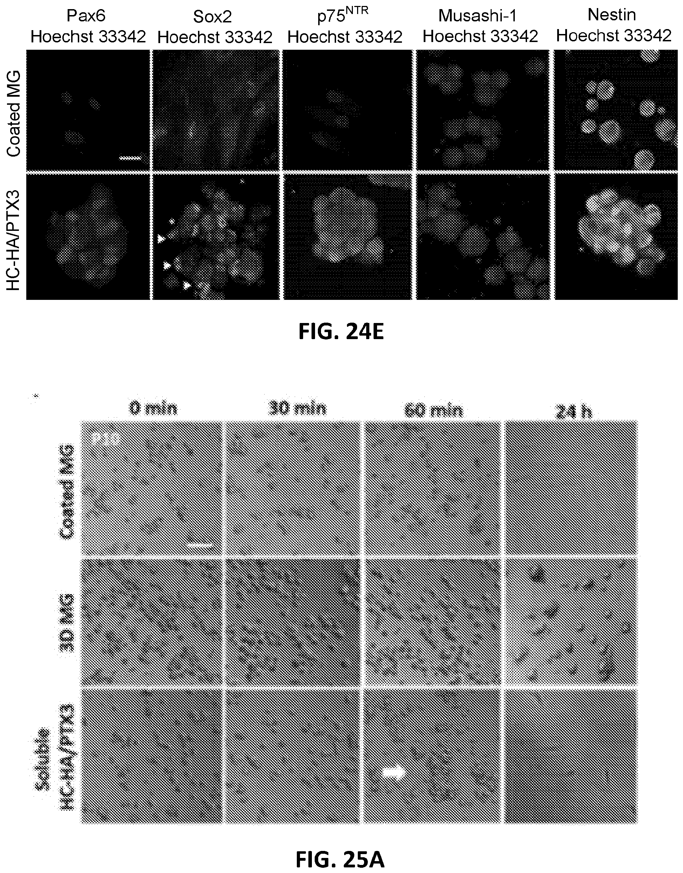

**FIGS. 24A-24E** illustrate immobilized HC-HA/PTX3, but not on 3D Matrigel^{™}, promotes neural crest progenitors with neuroglial potential in P10 LNC. 1x10⁵/ml P10 LNC were seeded on 5% coated MG, 3D MG or immobilized HC-HA/PTX3 in Covalink-NH 96 plate for 48 h in Modified Embryonic Stem Cell Medium (MESCM). **FIG. 24A** shows results sphere formation at 24 h and 48 h determined from phase contrast microscopy. White scale bar = 50 µm. **FIG. 24B** illustrates quantitative RT-PCR analysis was used to compare the mRNA levels of neural crest markers for pax6, p75^{NTR}, Musashi-1, Nestin, Msx-1, FoxD3 of P10 LNC on HC-HA/PTX3 when compare to respective gene expressions on coated MG (## p<0.05, n=3) or 3D MG (** p<0.05, n=3). **FIG. 24C** illustrates immunofluorescence staining showed the cytolocalization of neural crest progenitor markers for pax6, Sox2, p75^{NTR} and Musashi-1. Nuclear counterstaining by Hoechst 33342. White scale bars = 25 µm. The differentiation potential for cells derived from cell aggregates were assessed after being cultured in the respective induction media by phase microscopy and immunofluorescence staining of neurofilament M (NFM), O4, and glial fibrillary acidic protein (GFAP), respectively (**FIG. 24D**). Nuclear counterstaining by Hoechst 33342. Scale bars = 50 µm. **FIG. 24E** illustrates immunofluorescence staining to pax6, Sox2, p75^{NTR}, Musashi-1, and Nestin.

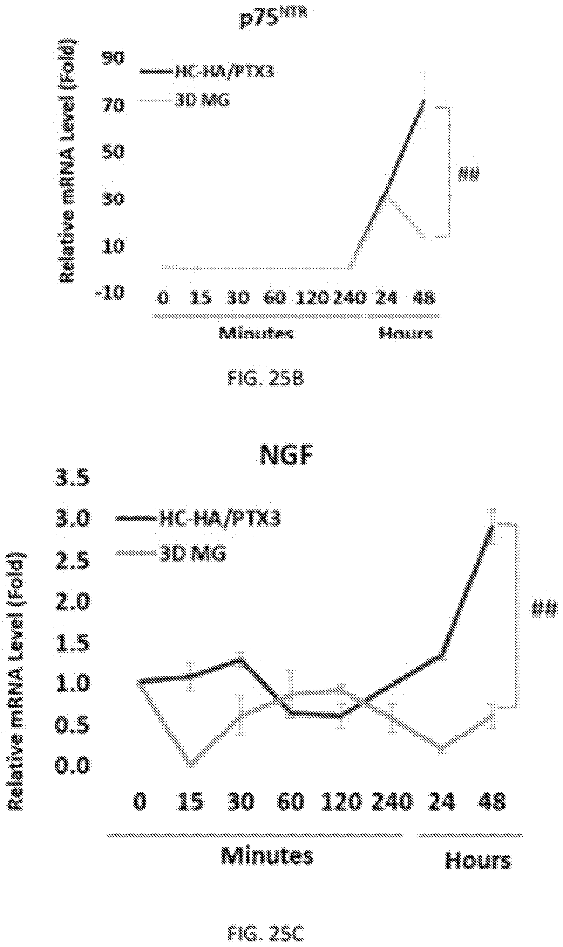

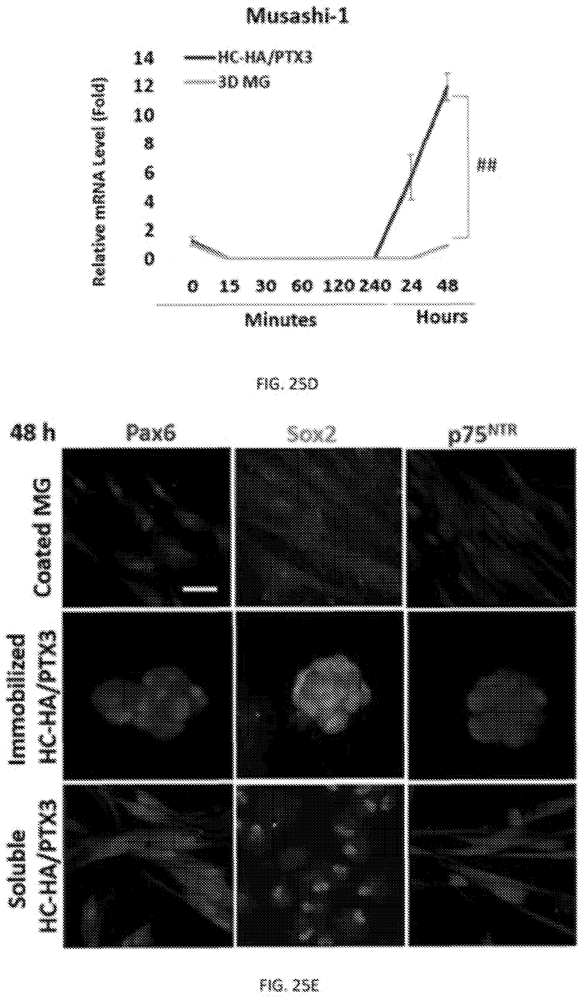

**FIGS. 25A-25E** illustrate soluble HC-HA/PTX3 promoted early cell aggregation and Pax6+ neural crest progenitors in P10 LNC. 1x10⁵/ml of P10 limbal niche cells were seeded on soluble HC-HA/PTX3, 3D MG or coated MG in MESCM. **FIG. 25A** illustrates phase contrast microscopy images of cell morphology and aggregation (marked by a white arrow). White scale bar = 100 µm. Quantitative RT-PCR analysis at different time course on 3D MG and HC-HA/PTX3 were used to compare to the mRNA of p75^{NTR} (**FIG. 25B**), NGF (**FIG. 25C**), and Musashi-1 (**FIG. 25D**) in P10 LNC. (## p < 0.01, n=3). **FIG. 25E** illustrates immunofluorescence staining confirmed the expression of Pax6, p75^{NTR} and Sox2 on coated MG, immobilized HC-HA/PTX3 or soluble HC-HA/PTX3 at 48 h. Bar scale: 50 µm. Nuclear counterstaining by Hoechst 33342.

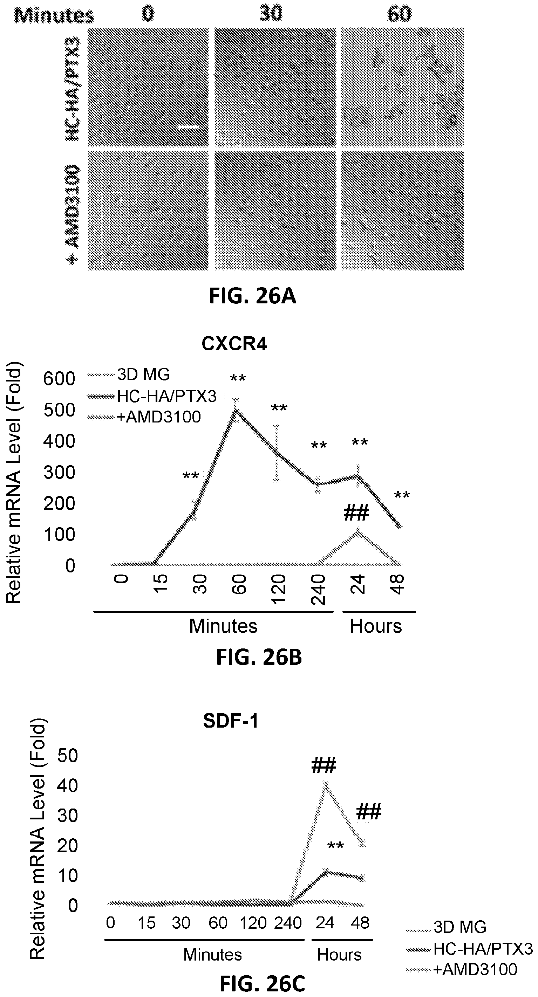

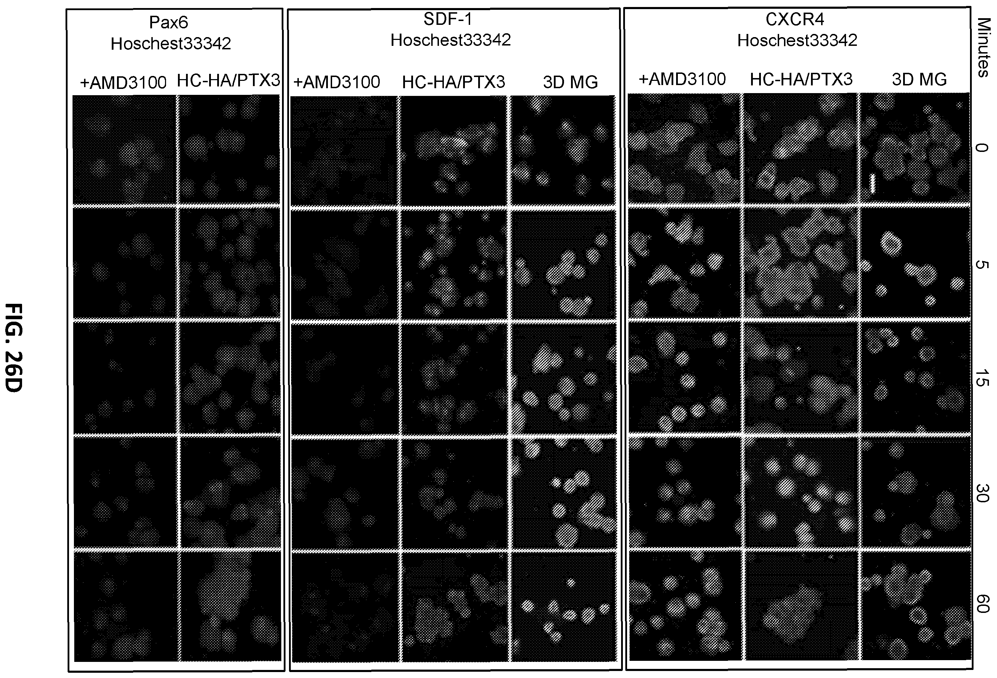

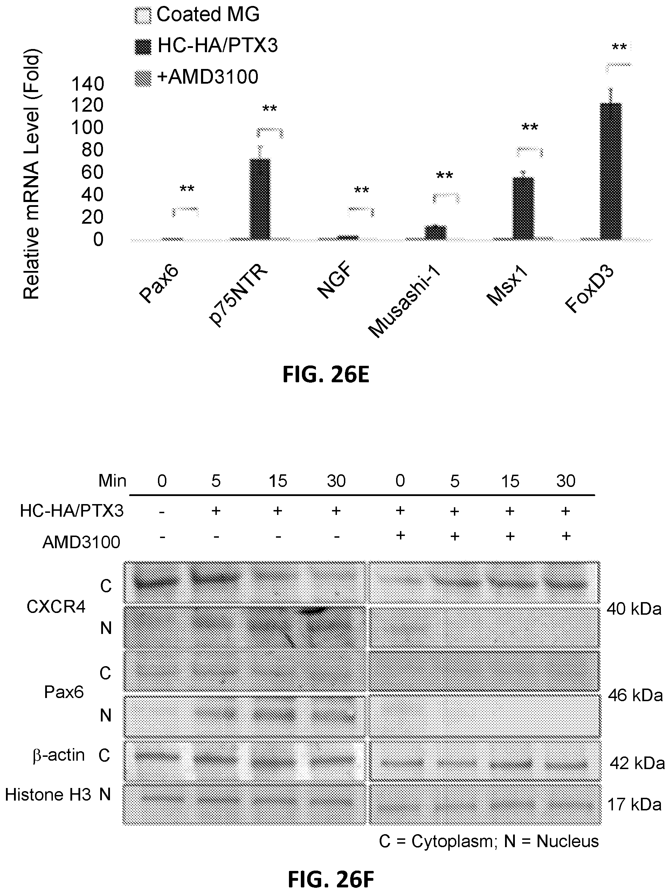

**FIGS. 26A-26F** illustrate cell aggregation and nuclear Pax6 expression promoted by soluble HC-HA/PTX3 is mediated by CXCR4/SDF-1 signalingP10 LNC were seeded in 3D MG or on coated MG with or without soluble HC-HA/PTX3 and pretreated with or without AMD3100 in MESCM for 5, 15, 30, 60 min or 48 h. Cell aggregation was assessed by phase contrast microscopy (**FIG. 26A**, bar = 100 µm). CXCR4/SDF-1 signaling was determined by qRT-PCR to compare the mRNA transcript levels of SDF-1 (**FIG. 26C**) and CXCR4 (**FIG. 26B**) using the expression level in 3D Matrigel at time 0 set as 1 (** p<0.01 or ^{##} p<0.01, n=3). Phenotypic characterization was performed by qRT-PCR for the mRNA transcript levels of Pax6, p75^{NTR}, NGF, Musashi-1, Msx-1, and FoxD3 using the expression level of coated MG set as 1 (**FIG. 26E**, ** p<0.01) and by immunofluorescence staining of CXCR4, SDF-1, and Pax6 (**FIG. 26D**, nuclear counterstaining by Hoechst 33342, Bar = 50 µm). Protein expression of cytoplasmic or nuclear extract fraction of Pax6 and CXCR4 were confirmed by western blot using β-actin or Histone H3 as the loading control. (**FIG. 26F**).

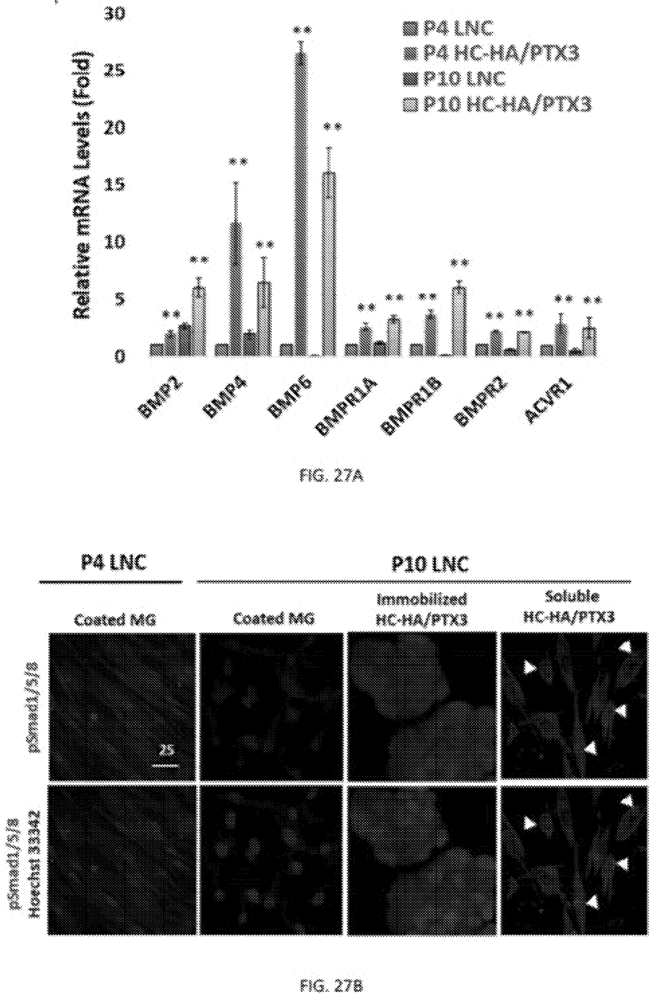

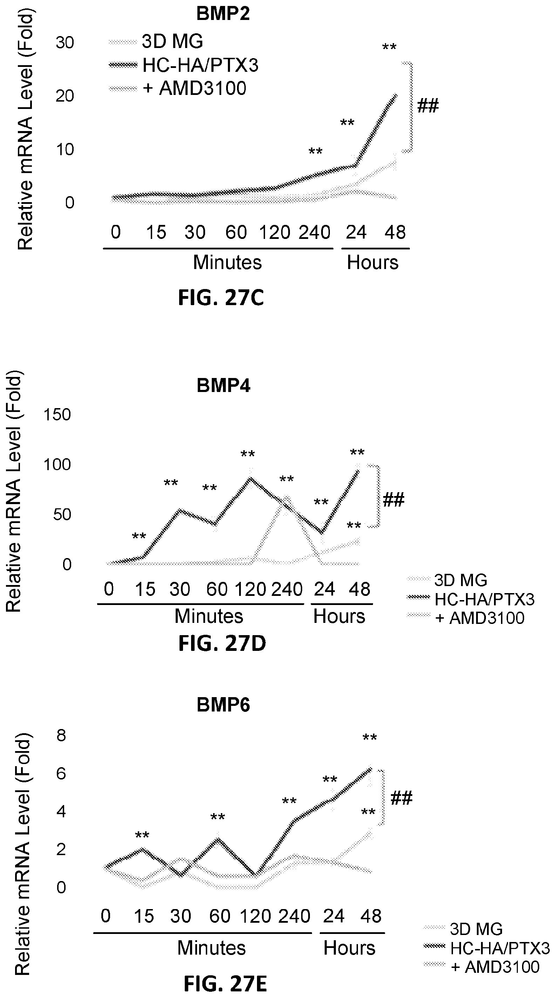

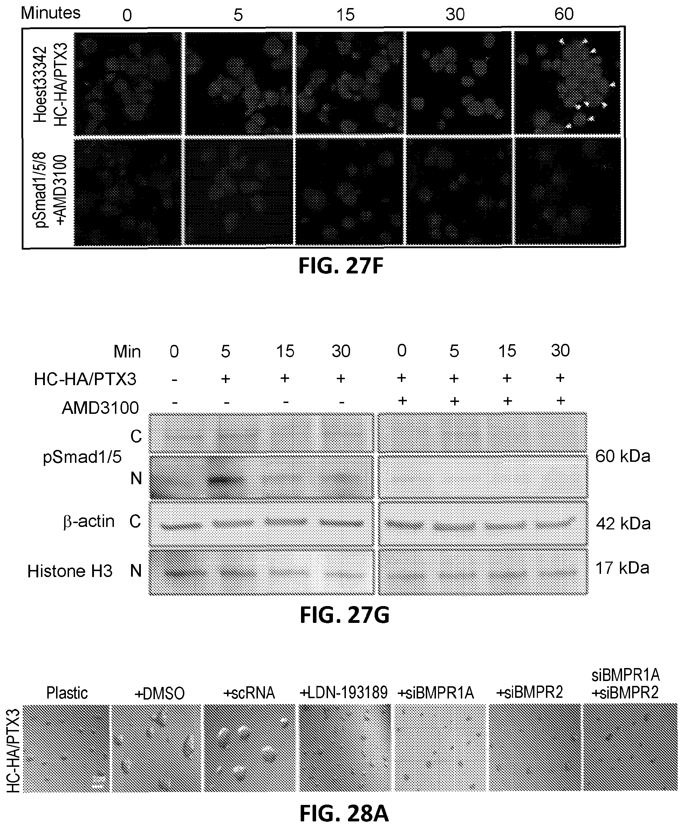

**FIGS. 27A-27G** illustrate HC-HA/PTX3 promotes cell aggregation and BMP Signaling in P10 LNC; however, BMP ligands alone on Plastic does not promote BMP signaling with reduced cell aggregation. Early (P4) of limbal niche cells were expanded on the plastic with or without addition of BMP ligands or HC-HA/PTX3 in Modified Embryonic Stem Cell Medium (MESCM) for 24 h. Late (P10) passaged of limbal niche cells were seeded on 3D MG or immobilized HC-HA/PTX3 in MESCM for 5, 15, 30, 60 and 120 minutes. Cell aggregates in HC-HA/PTX3 or plastic treating with BMP ligands were compared P4 LNC on at 24 h and immunofluorescence staining of nuclear pSmad1/5/8 were compared. Phase white scale bars = 100 µm. **FIG. 27A** illustrates transcript expression of BMP ligands and receptors, BMP2, BMP4, BMP6, BMPR1A, BMPR2 and ACVR1 on coated MG or HC-HA/PTX3 by RT-qPCR were used to compare in P4 and P10 LNC. **FIG. 27B** illustrates immunofluorescence staining of nuclear pSmad1/5/8 in P4 and P10 LNC on coated Matrigel^{™}, HC-HA/PTX3 or soluble HC-HA/PTX3 were compared. IF white scale bars = 25 µm. Quantitative RT-PCR analysis at different time course on 3D MG and HC-HA/PTX3 were used to compare the mRNA expression of BMP2 (**FIG. 27C**), BMP4 (**FIG. 27D**), and BMP6 (**FIG. 27E**) in P10 LNC. (** p <0.01, n=3; ## P < 0.01, n=3). **FIG. 27F** illustrates immunofluorescence staining of nuclear pSmad1/5/8. **FIG. 27G** illustrates protein expression of nuclear and cytoplasmic extract fractions of pSmad1/5 as confirmed by western blot using β-actin and Histone H3 as the loading control.

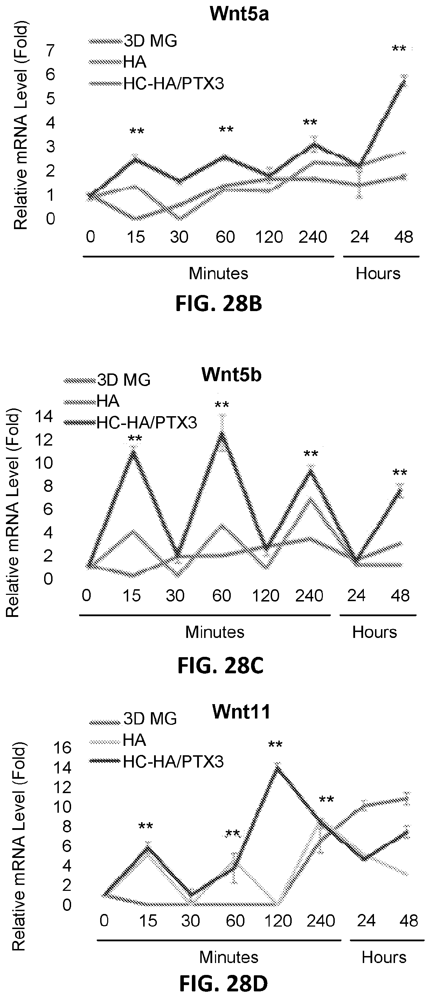

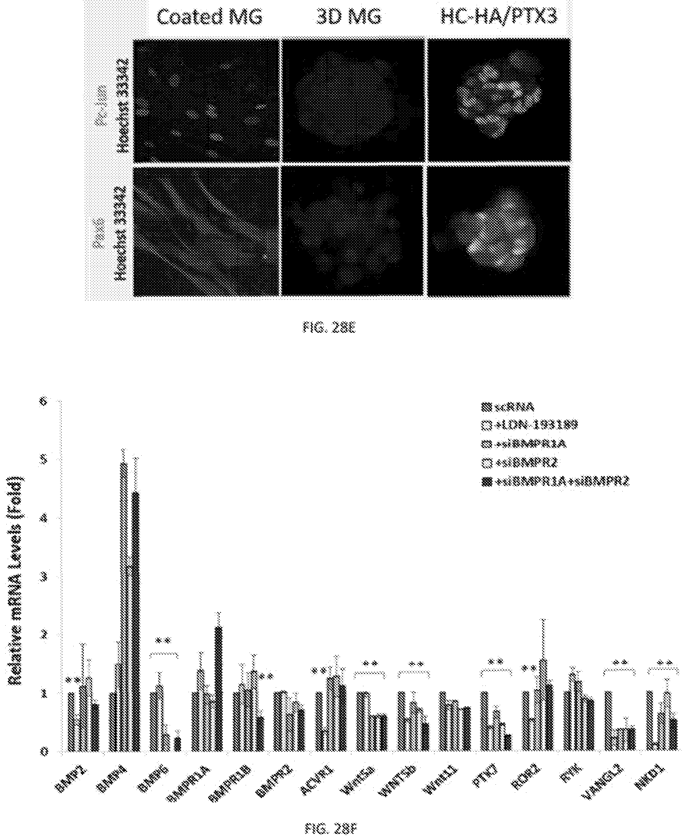

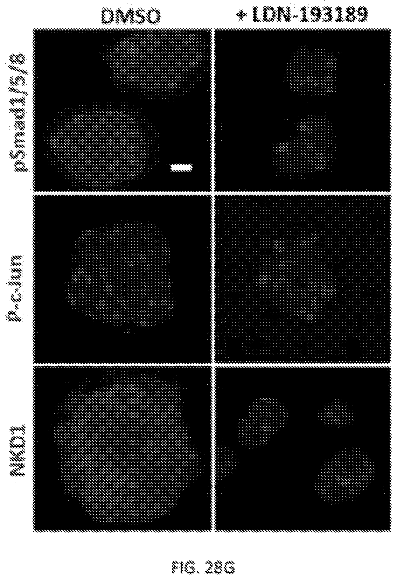

**FIGS. 28A-28G** illustrate immobilized HC-HA/PTX3 Promotes BMP Signaling, required for Cell Aggregation and the Initiation of PCP Signaling in P4 LNC. 1x10⁵/ml of P4 LNC were pre-treated with LDN-193189 for 1 h or transfection reagent containing 50 µl of DMEM mixed with HiPerfect siRNA transfection reagent and scrambled RNA, siBMPR1A, siBMPR2 or siBMPR1A/siBMPR2 for 72h before seeding in immobilized HC-HA/PTX3 on Covalink-NH 96 plate for 48 h in Modified Embryonic Stem Cell Medium. **FIG. 28A** illustrates the resulting cell aggregates imaged by phase contrast microscopy at 24 h. **FIG. 28B** illustrates qRT-PCR of the transcript expression of Wnt5a. **FIG. 28C** illustrates qRT-PCR of the transcript expression of Wnt5b. **FIG. 28D** illustrates qRT-PCR of the transcript expression of Wnt11. **FIG. 28E** illustrates immunostaining of pc-Jun, and Pax6 in P10 LNC seeded on immobilized HC-HA/PTX3, coated Matrigel^{™}, or 3D Matrigel^{™}. **FIG. 28F** illustrates qRT-PCR of the transcript expression of BMP ligands and receptors (and PCP ligands and receptors). **FIG. 28G** illustrates immunostaining of pSmad1/5/8, (p-c-Jun and NKD1) were performed to confirm the status of canonical BMP signaling (and PCP signaling). Nuclear counterstaining by Hoechst 33342. Scale bars = 25 µm.

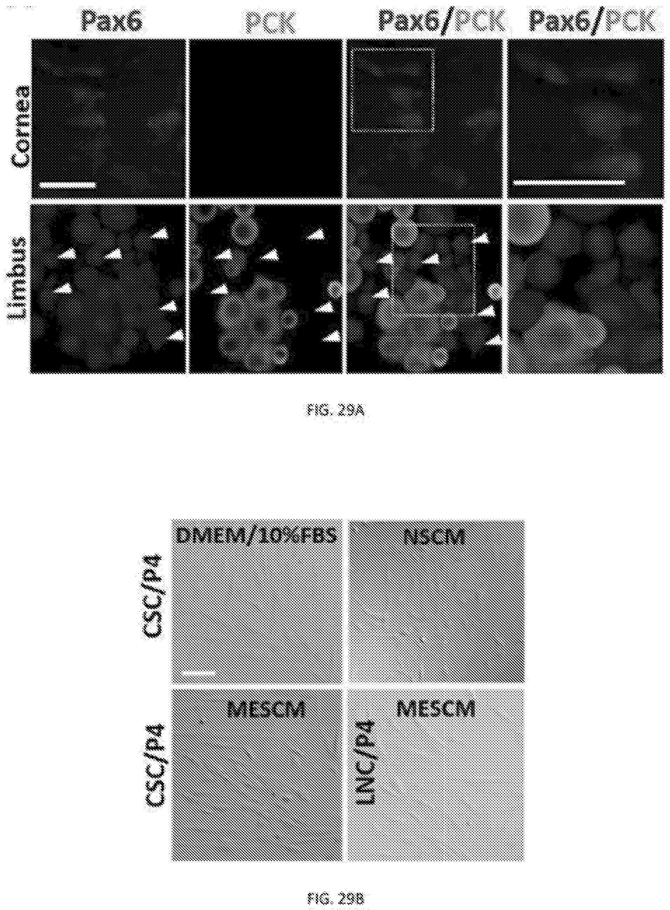

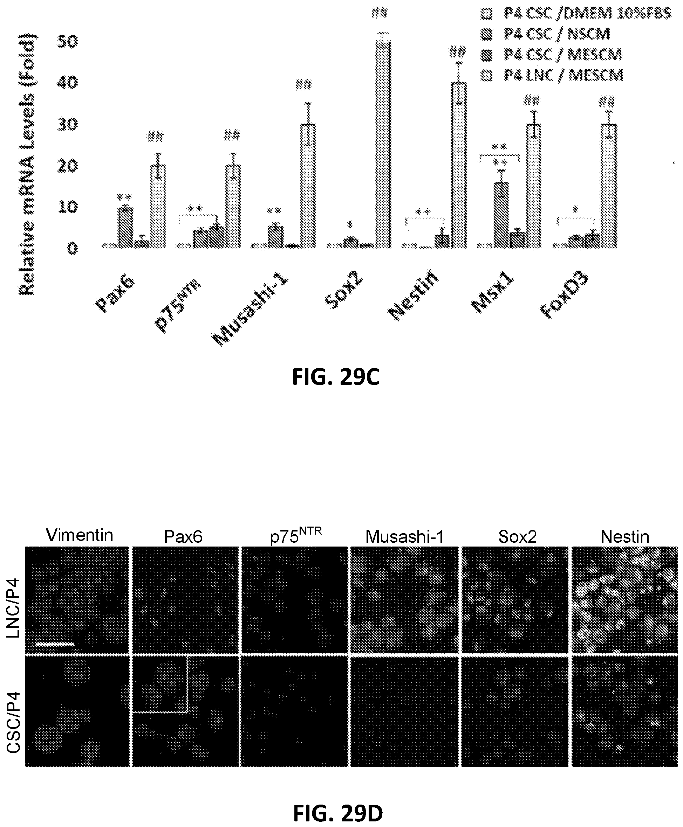

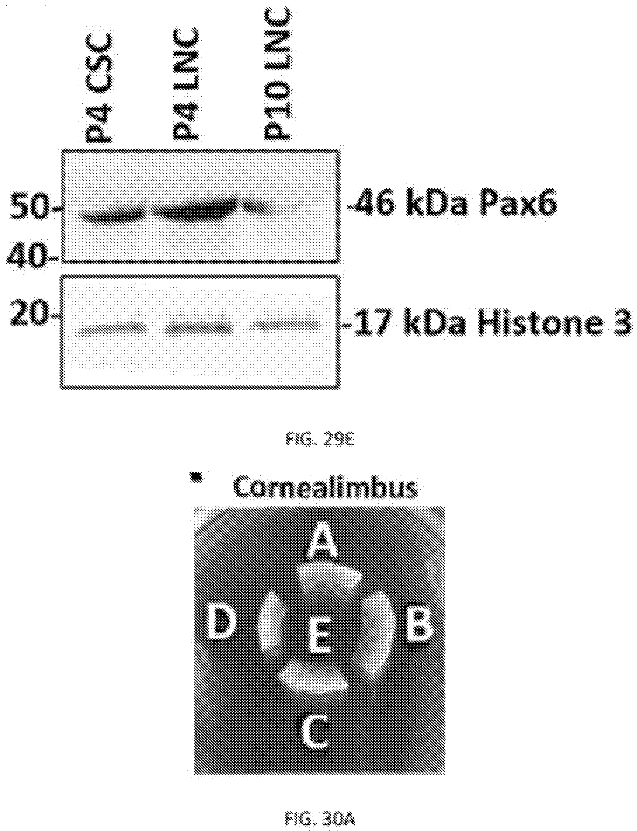

**FIGS. 29A-29E** illustrate unique nuclear 46 kDa Pax6 in limbal niche cells (LNC). **FIG. 29A** illustrates freshly isolated PCK (-) LNC (arrows) and PCK (+) limbal epithelial cells from the limbal tissue exhibited positive nuclear staining of Pax6 while freshly isolated PCK (-) CSC from epithelially denuded corneal stroma exhibited cytoplasmic staining of Pax6. LNC and CSC were expanded in the same manner on coated Matrigel^{™} in MESCM up to passage 4 (P4) while CSC were also cultured on plastic in neural stem cell medium (NSCM) or DMEM/10% FBS. **FIG. 29B** illustrates a comparison made on day 6 of cell morphology by phase microscopy. **FIG. 29C** illustrates transcript expression by RT-qPCR of neural crest markers (Pax6, p75^{NTR}, Musashi-1, Sox2, Nestin, Msx2, and FoxD3) in P4 LNC was compared to that of P4 CSC under the identical culture conditions (^{##}p<0.05, n-3). Bars from left to right: P4 CSC/DMEM; P4 CSC/NSCM; P4 CSC/MESCM; P4 LNC/ MESCM. **FIG. 29D** illustrates immunofluorescence staining showing the cytolocalization of vimentin, Pax6, p75^{NTR}, Musashi-1, Sox2, and Nestin in P4 LNC and P4 CSC on coated Matrigel^{™} in MESCM (nuclear counterstaining by Hoeschst 33342) Scale bars = 100 µm. **FIG. 29E** illustrates protein expression of Pax6 from P4 CSC, P4 LNC, and P10 LNC were confirmed by western blot using Histone 3 as a loading control.

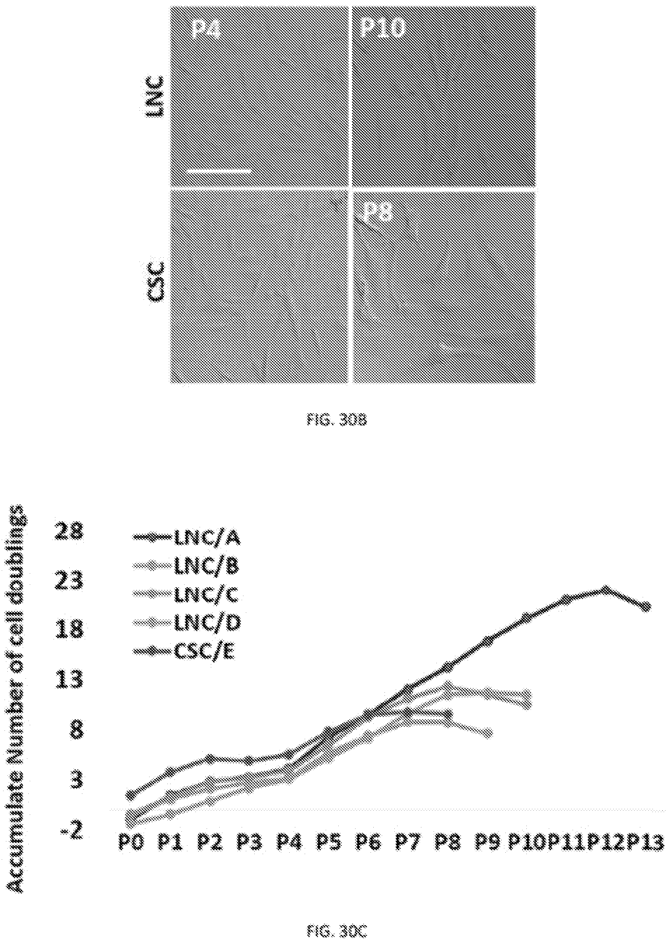

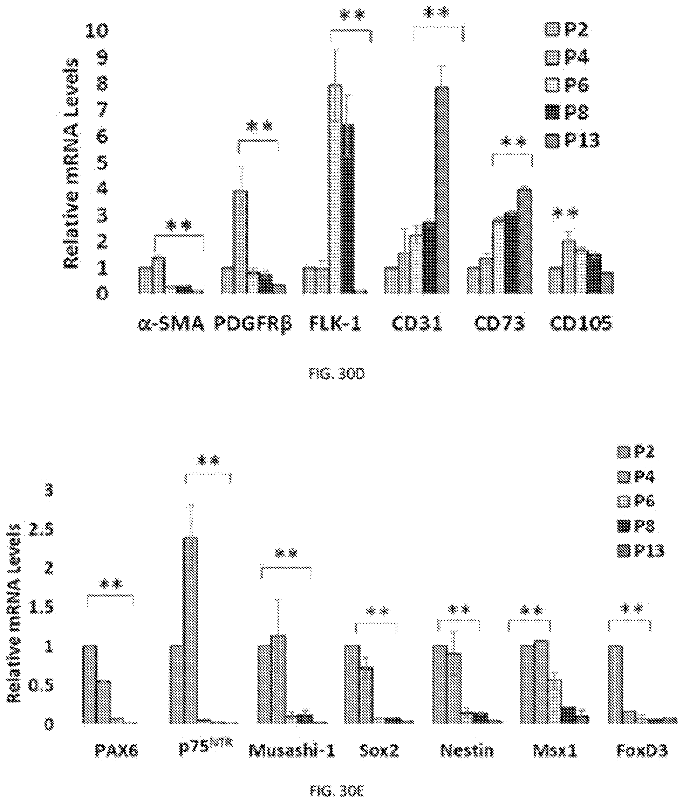

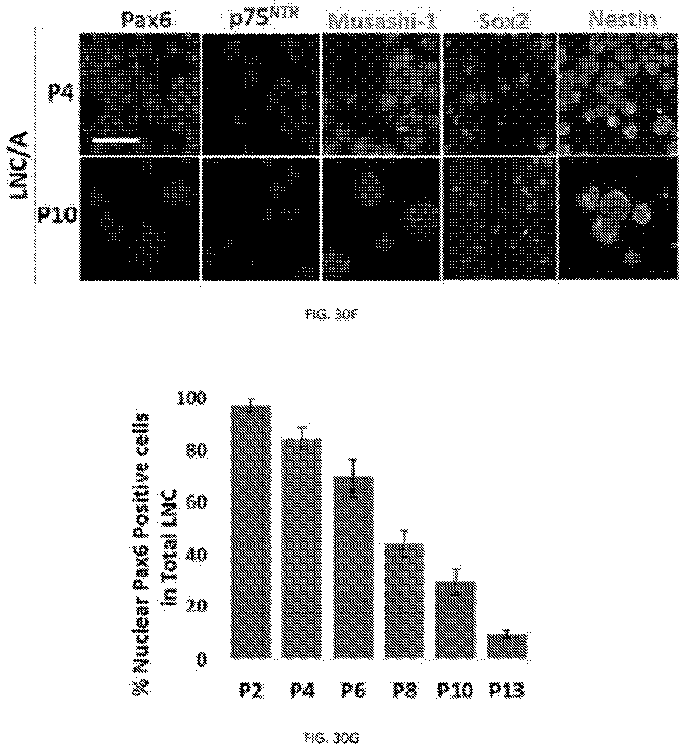

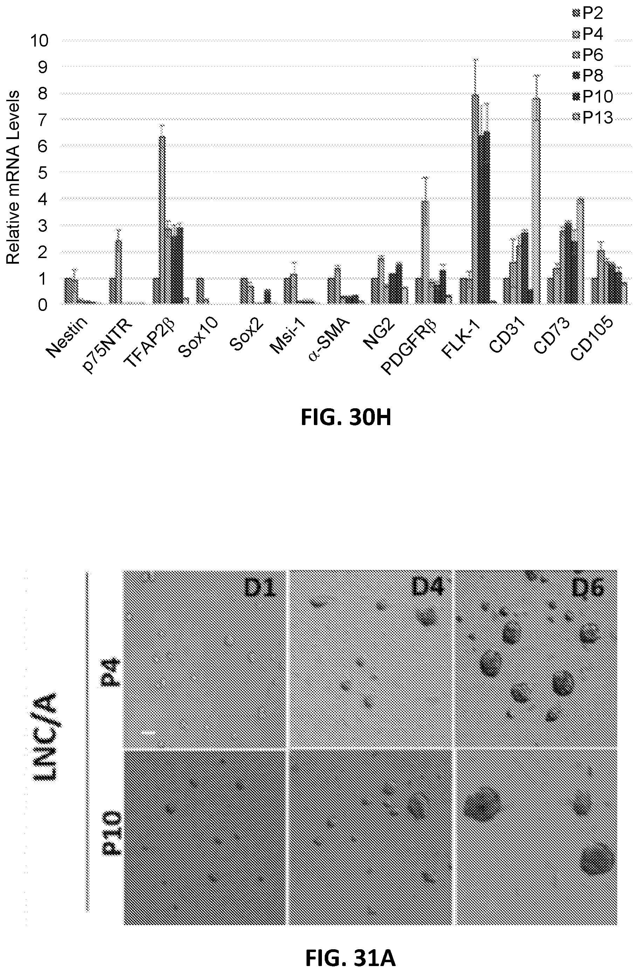

**FIGS. 30A-30H** illustrate loss of nuclear Pax6 staining in LNC after serial passages. LNC and CSC were isolated from four quadrants (labeled as A-D) and central cornea (labeled as E) of the same donor, as illustrated in **FIG. 30A****.** These LNC and CSC were serially passaged to measure cumulative doubling time on coated Matrigel^{™} in MESCM, as illustrated in **FIG. 30C. FIG. 30B** illustrates a comparison of cell morphology as determined by phase microscopy on day 6. **FIG. 30D** illustrates transcript expression of angiogenic markers (α-SMA, PDGFRβ, FLK-1, CD31), mesenchymal stem cell markers (CD73 and CD105) determined by RT-qPCR using the transcript expression level of each marker in P2 set at 1 (** p<0.01, n=3). Bars from left to right: P2, P4, P6, P8, P13. **FIG. 30E** illustrates transcript expression of neural crest markers (Pax6, p75^{NTR}, Musashi-1, Sox2, Nestin, FoxD3, and Msx1) determined by RT-qPCR using the transcript expression level of each marker in P2 set at 1 (** p<0.01, n=3). Bars from left to right: P2, P4, P6, P8, P13. **FIG. 30F** illustrates immunofluorescence staining showed the cytolocalization of Pax6, p75^{NTR}, Musashi-1, Sox2, and Nestin. Scale bars = 100 µm. **FIG. 30G** illustrates the percentage of cells with nuclear Pax6 staining in total LNC from region A declined during the serial passages. **FIG. 30H** illustrates transcript expression of various markers determined by RT-qPCR using the transcript expression level of each marker in P2 set at 1.

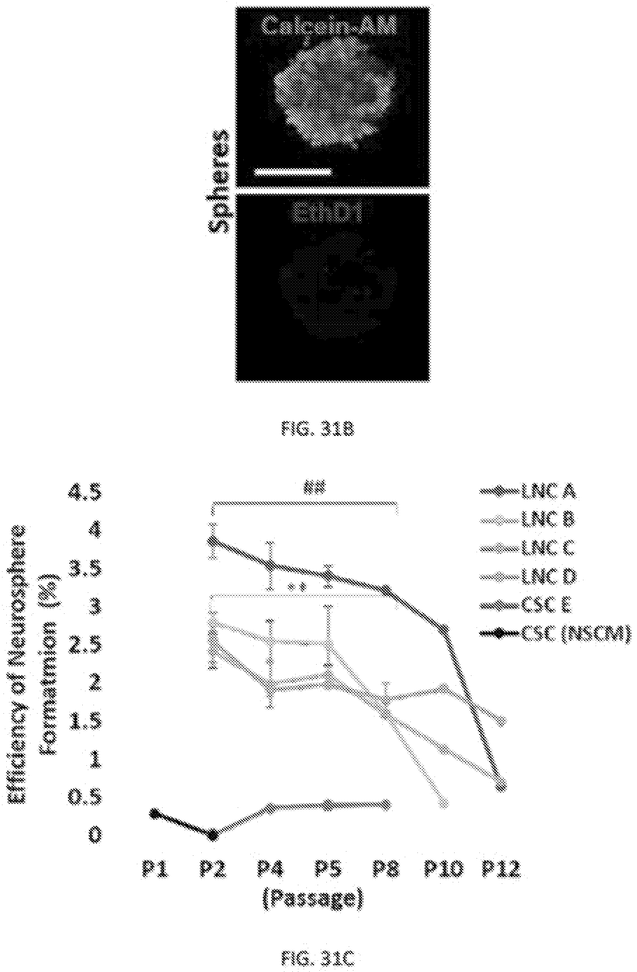

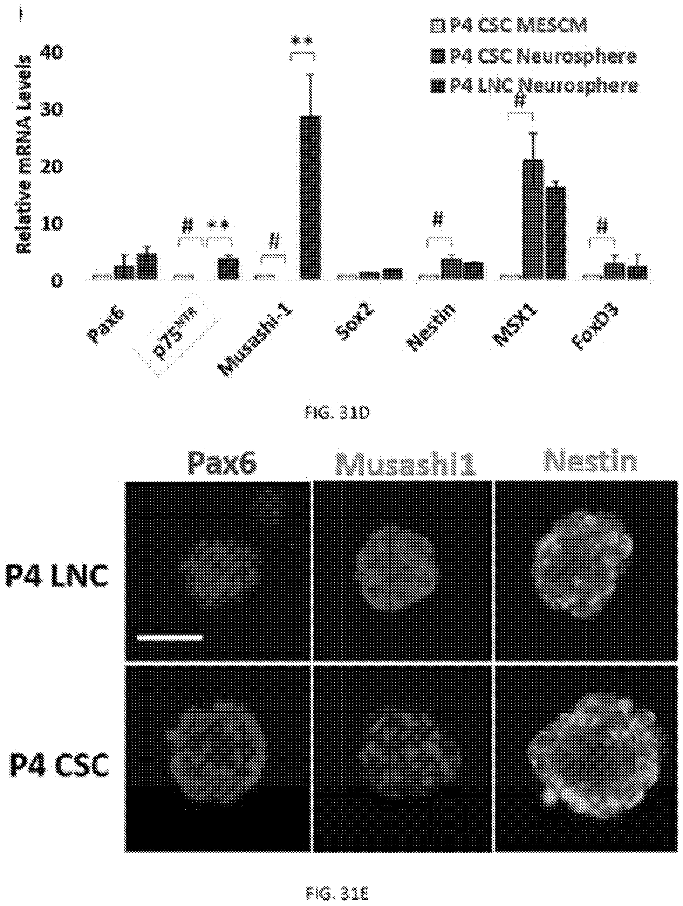

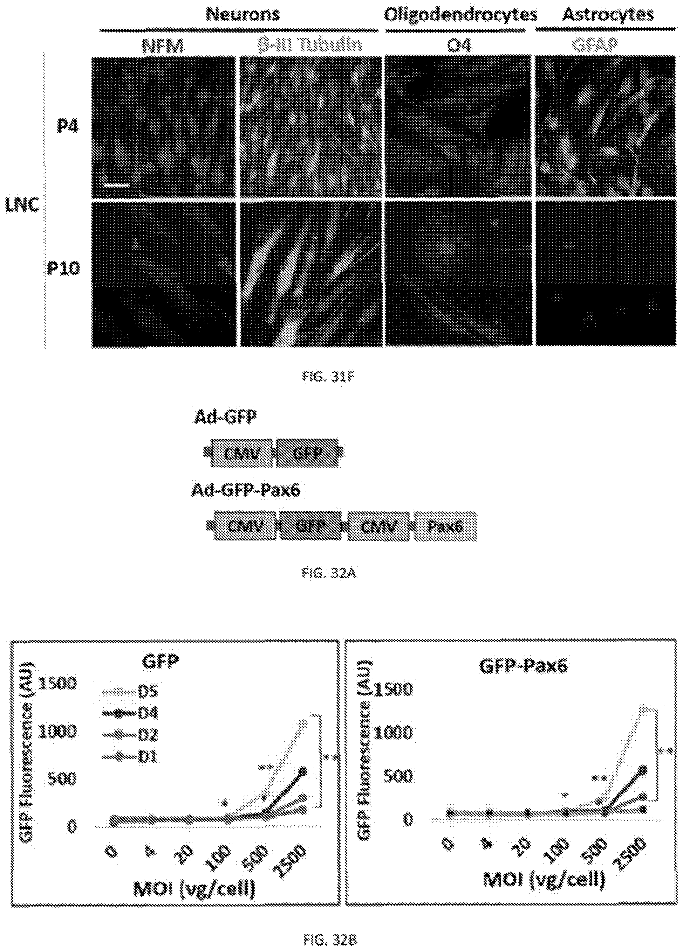

**FIGS 31A-31F** illustrates neural potential of LNC and CSC declines after serial passages. For each passage, 5 x 10³/cm² LNC cells were seeded on a 12 well plate coated with poly-HEMA in NSCM neurosphere medium to generate neurospheres for 6 days **(****FIG. 31A**; scale bar = 50 µm). **FIG. 31B** illustrates a live and dead assay showed the sphere formed by P4 LNC was alive on day 6 without dead cells. Scale bar =200 µm. The neurosphere-forming efficiency (%) was measured from LNC expanded from four different limbal regions and was compared with that of CSC region at each passage (**FIG. 31C**; ^{##} p<0.001 (LNC A); ** p<0.001 (LNC B)). The transcript level of neural crest markers such as Pax6, p75^{NTR}, Musashi-1, Sox2, Nestin, Msx1, and FoxD3 in neurospheres formed by P4 CSC was compared with those by P4 LNC or P4 CSC seeded on coated Matrigel^{™} in MESCM which the transcript expression was set as 1 (**FIG. 31D****,** ** p=0.0001; # p=0.001, n=3, respectively). Bars from left to right: P4 CSC MESCM, P4 CSC Neurosphere, P4 LNC Neurosphere. **FIG. 31E** illustrates immunofluorescence staining showing cytolocalization of Pax6, Musashi-1, and Nestin in neurospheres derived from P4 CSC and P4 LNC. Scale bar =100 µm. **FIG. 31F** illustrates P4 or P10 LNC were assessed for their potential of differentiation into neurons, oligodendrocytes, and astrocytes by immunofluorescence staining of neurofilament M (NFM) and β-III tubulin, O₄, and Glial fibrillay acidic protein (GFAP), respectively. Scale bar = 50 µm. Nuclear counterstaining by Hoeschst 33342.

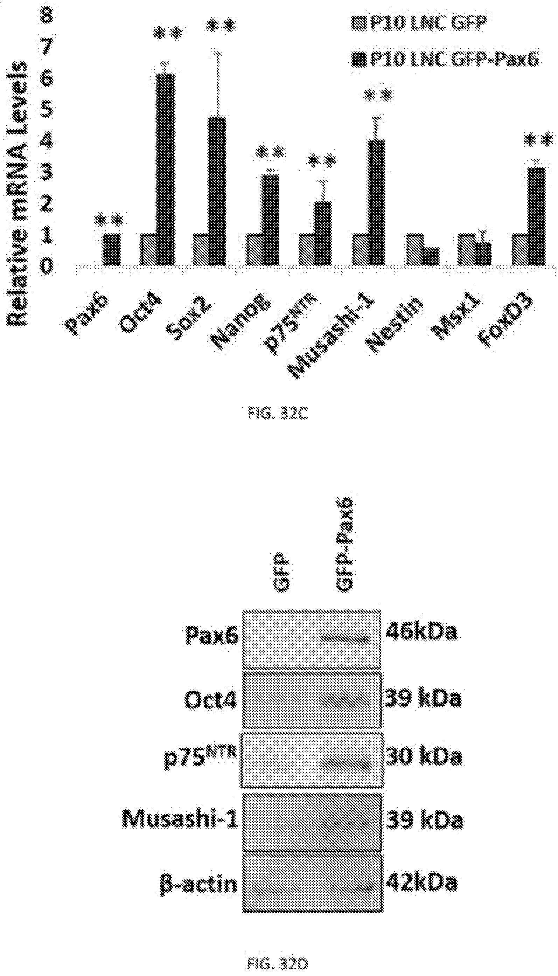

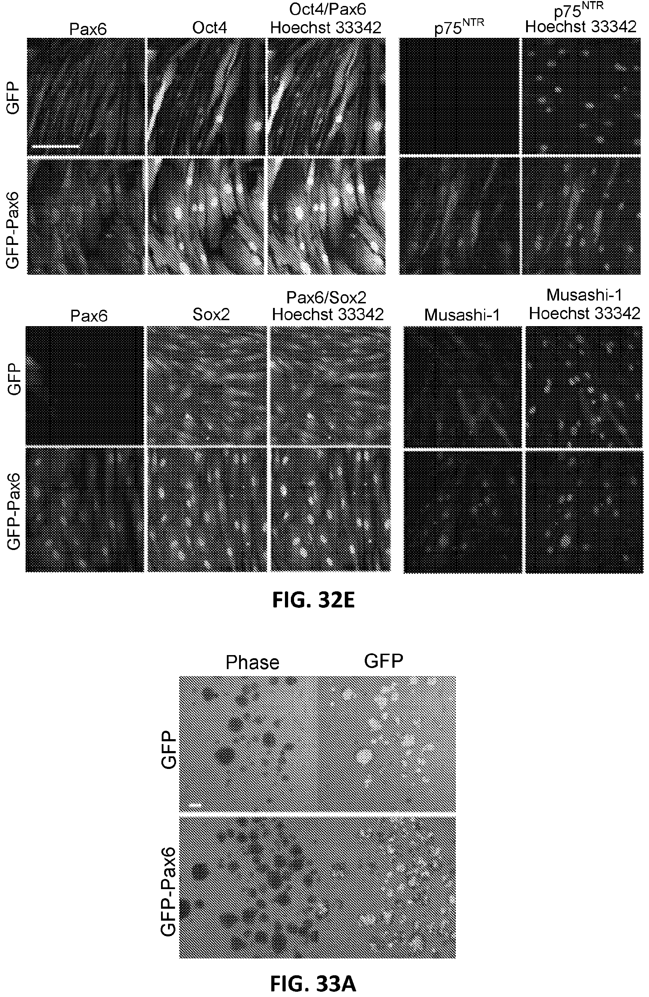

**FIGS. 32A-32E** illustrates forced expression of Pax6 upregulates expression of neural crest markers in P10LNC. **FIG. 32A** illustrates an Ad-GFP (GFP) plasmid or an Ad-GFP-Pax6 (GFP-Pax6) plasmid. Plasmids were transfected in P10 LNC cultured on coated Matrigel^{™} in MESCM after their respective multiplicity of infection (MOI) was pre-determined during a period of 5 days (**FIG. 32B****,** * p<0.1, **p<0.05, n=3). Following the respective transfection, RT-PCR analysis was used to compare the transcript levels of ESC markers (Oct4, Sox2, and Nanog) and neural crest markers (P7S^{NTR}, Musashi-1, Nestin, Msx1, and FoxD3) (**FIG. 32C****,** **p<0.05, n=3). FIG. 32D illustrates a Western blot analysis was used to compare the protein expression of 46 kDa Pax6, Oct4, p7S^{NTR}, and Musashi-1 using β-actin as the loading control. Cytolocalization of Pax6 and Oct4, Pax6 and Sox2, as well as p75^{NTR} and Musashi-1 were determined by either double or single immunofluorescence staining (**FIG. 32E**). Nuclear counterstaining by Hoechst 33342. Scale bar = 100 µm.

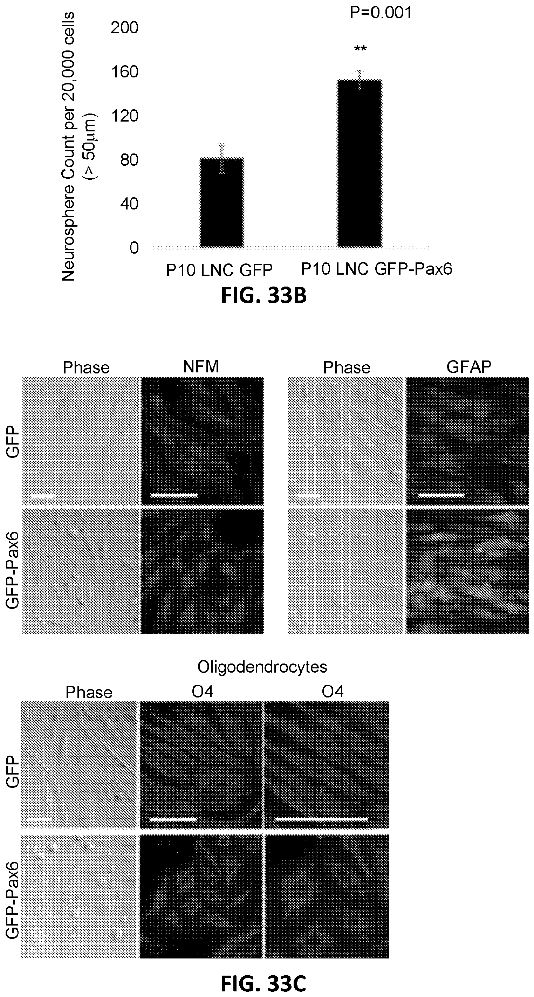

**FIGS. 33A-33C** illustrate forced expression of Pax6 upregulates expression of neural crest markers in P10LNC. P10 LNC on coated Matrigel^{™} in MESCM was transfected with Ad-GFP (GFP) or Ad-GFP-Pax6 (GFP-Pax6) plasmid at MOI 100 for 4 days, then the medium was switched to NSCM neurosphere medium for 7 days. Neurospheres were imaged by confocal microscopy with or without fluorescence for GFP (**FIG. 33A**). The total number of neurospheres with a size greater than 50 µm in diameter were compared (FIG. 33B, *p=0.001, n=3). The differentiation potential for cells derived from neurospheres was assessed after cells were cultured in different induction media and observed by phase microscopy and immunofluorescence staining of neurofilament M (NFM), O₄, and glial fibrillary acidic protein (GFAP) (**FIG. 33C****,** nuclear counterstaining by Hoechst 33342, scale bars = 50 µm).

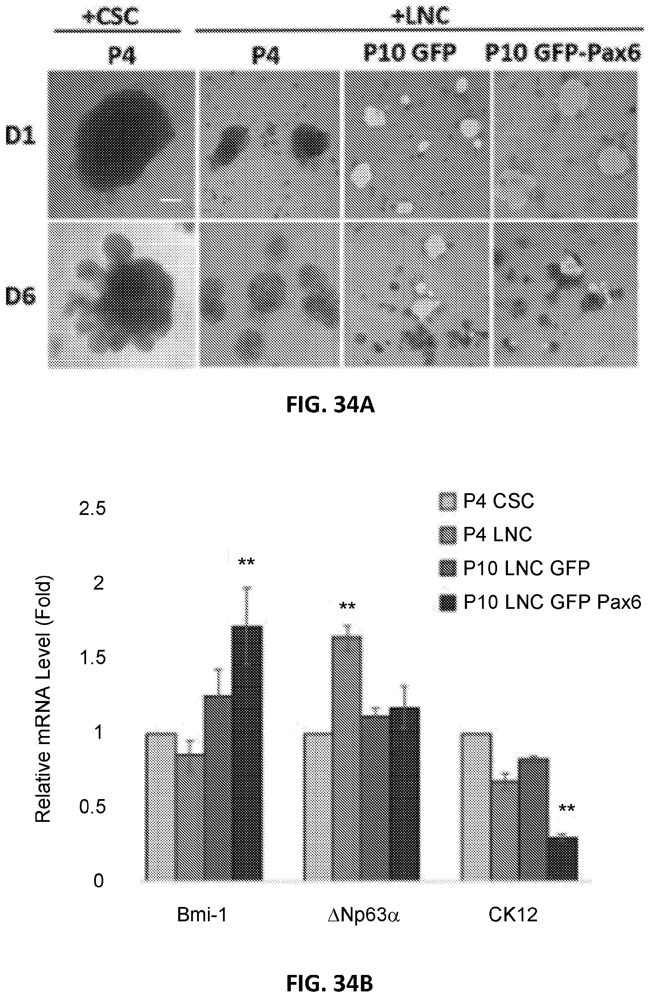

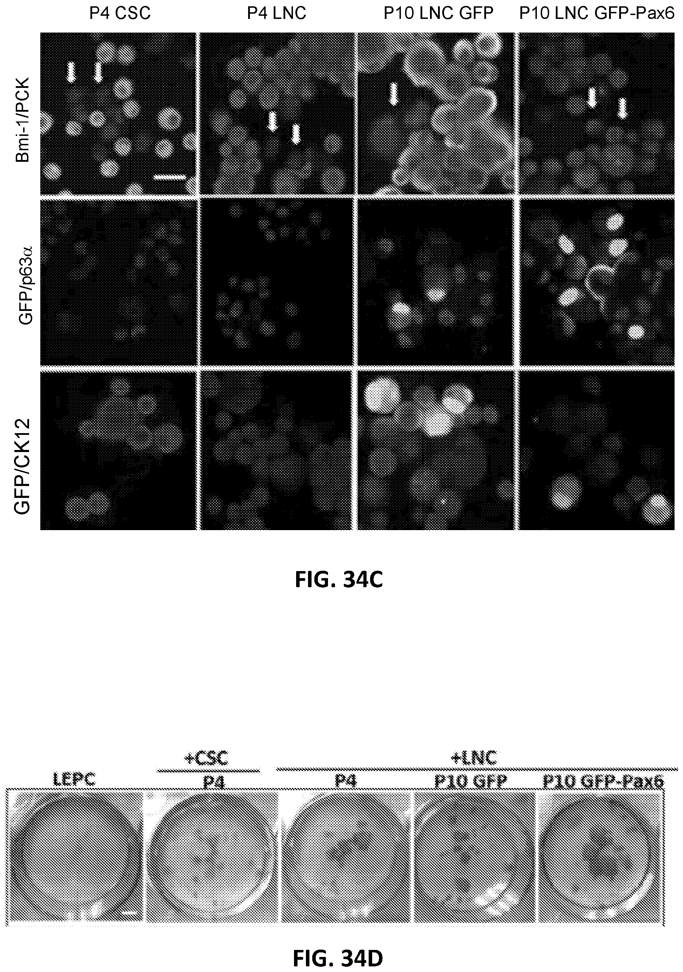

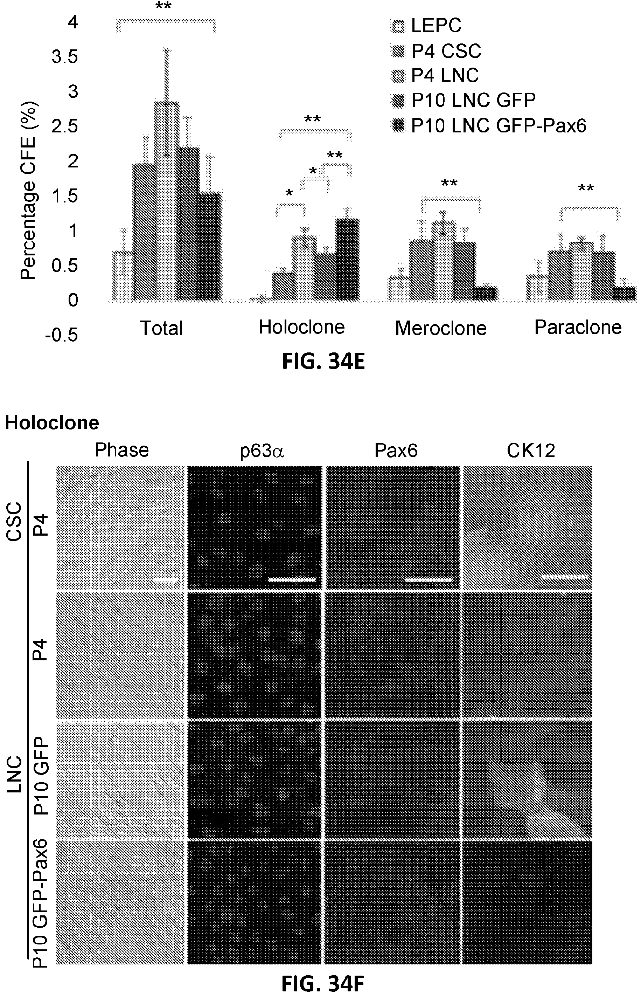

**FIGS. 34A-34F** illustrate P10 LNC with forced expression of Pax6 promoted selfrenewal of LEPC. *In vitro* reunion assay was performed between P10 LNC transfected with Ad-GFP or Ad-GFP-Pax6 plasmid at MOI 100 and LEPC in comparison with the positive control of P4 LNC and the negative control of P4 CSC. Sphere morphology was imaged by phase and GFP fluorescence under confocal microscopy at Day 1 and Day 6 (**FIG. 34A**; scale bar = 50 µm). The resultant reunion spheres were analyzed by qRT-PCR for transcript expression of Bmi-1 (**p = 0.003, n=3), ΔNp63α (**p=0.06, n=3), and cytokeratin 12 (CK12) (**p=0.000004, n=3) when compared with P4 CSC as the control (**FIG. 34B**). Double immunostaining was performed for Bmi-1/PCK, GFP/p63α, and GFP/CK12 for PCK (+) cells (**FIG. 34C**, white arrows indicate PCK (-) cells; scale bar = 50 µm.). *In vitro* clonal assay for LEPC with or without reunion with P10 LNC transfected with Ad-GFP or Ad-GFP-Pax6, P4 LNC or P4 CSC was performed on 3T3 fibroblast feeder layers. The clonal growth was assessed by rhodamine B staining (**FIG. 34D**; scale bar = 0.5mm.) while the colony-forming efficiency (%) for total, holoclone, meroclone, and paraclone was compared (**FIG. 34E**, *p<0.05; **<0.01). The epithelial morphology of holoclone was further characterized by phase image and immunostaining of p63α, Pax6, and CD12 (**FIG. 34F****;** scale bar = 50 µm.). Nuclear counterstaining by Hoechst 33342.

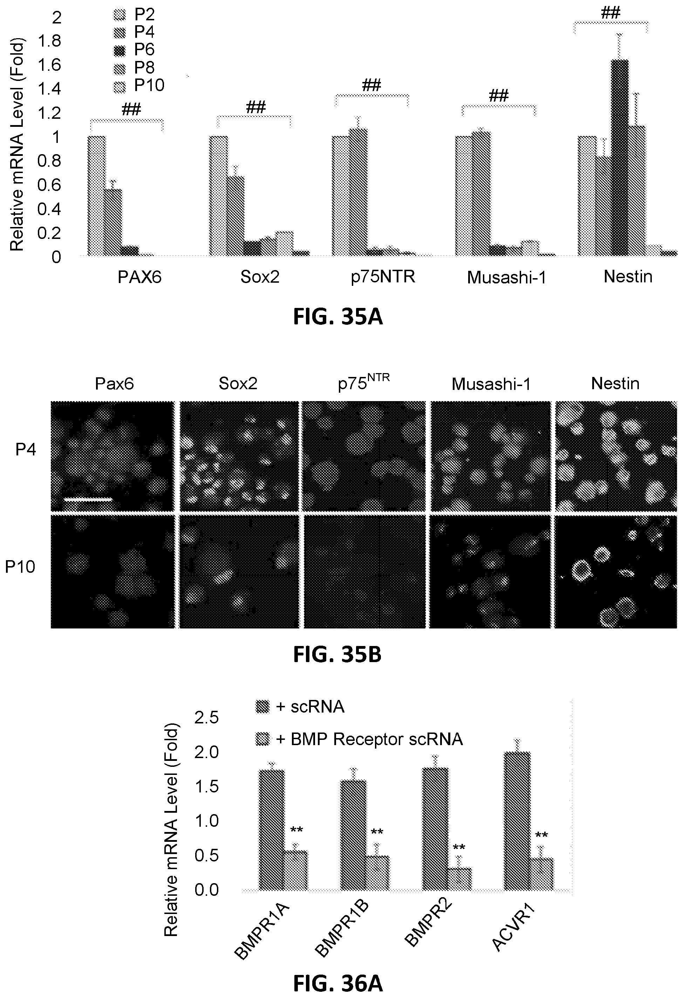

**FIGS. 35A-35B** illustrate progressive loss of nuclear Pax6 neural crest progenitor status in LNC after serial passage. P10 LNC were on 5% coated MG in MESCM and serially passaged. The phenotype of P10 LNC was determined by quantitative RT-PCR for mRNA levels of neural crest markers such as Pax6, Sox2, p75^{NTR}, Musashi-1, and Nestin using the expression level at passage 2 (P2) set as 1 (**FIG. 35A**, ## p<0.01, n=3) and immunofluorescence staining of Pax6, Sox2, p75^{NTR}, Musashi-1, and Nestin between P4 and P10 LNC (**FIG. 35B**, Bar = 100 µm).

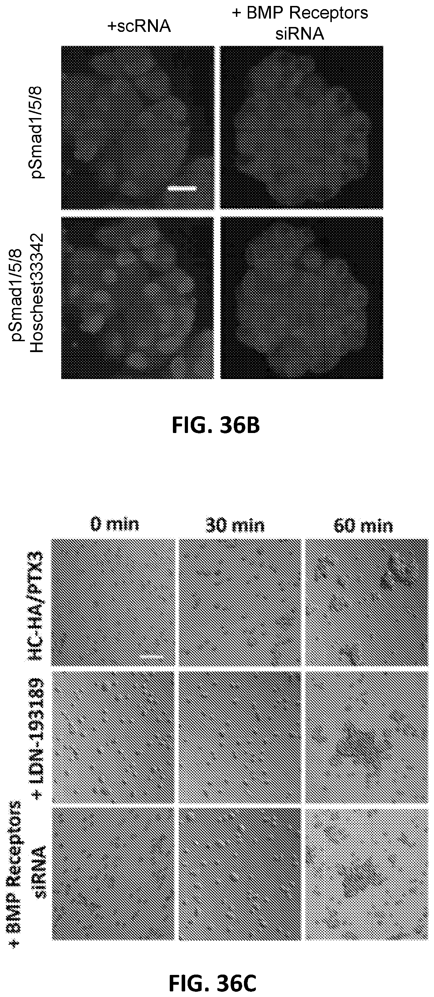

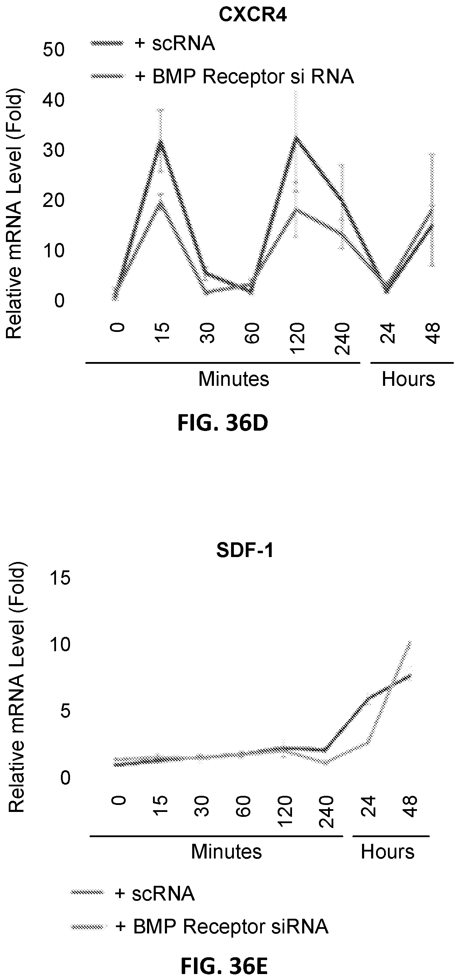

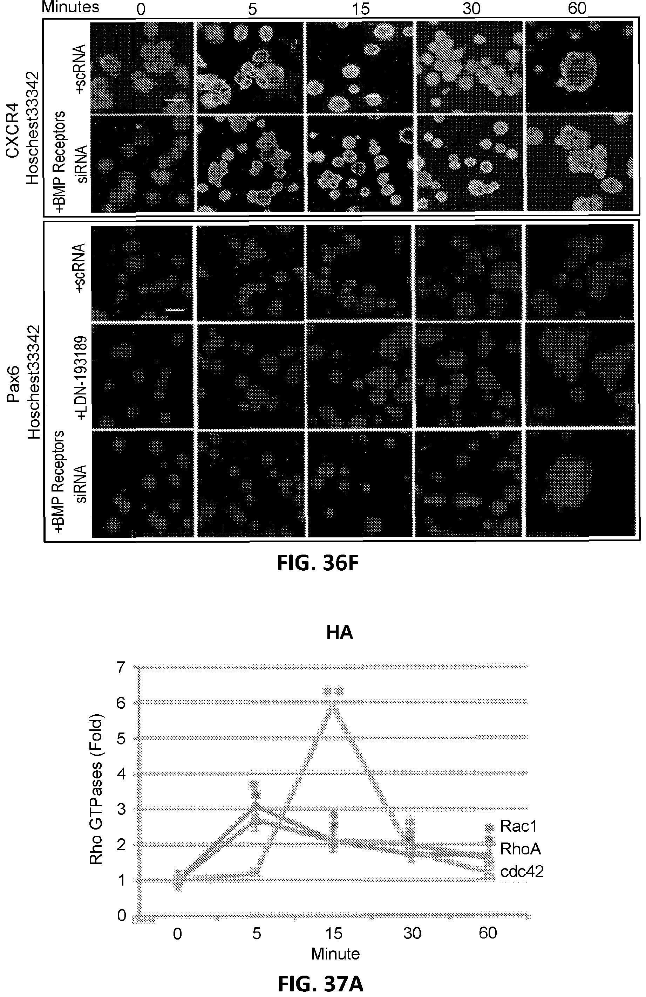

**FIGS. 36A-36F** illustrate cell aggregation and CXCR4/SDF-1 signaling promoted by HC-HA/PTX3 is not affected by BMP signaling. P10 LNC on coated MG in MESCM were pretreated with or without transfection with siRNAs for BMPR1A, BMPR1B, BMPR2 and ACVR1 before being seeded on coated MG with or without soluble HC-HA/PTX3 in MESCM. The transfection efficiency was verified by qRT-PCR when compared to scrambled RNA (scRNA) as the control (FIG. 36A, ** p <0.01, n=3). BMP signaling was measured by immunofluorescence staining to pSmad1/5/8 (FIG. 36B) and cell aggregation was detected by phase contrast microscopy (FIG. 36C, bar = 100 µm). CXCR4/SDF-1 signaling was assessed by qRT-PCR for the expression of CXCR4 (FIG. 36D) and SDF-1 (FIG. 36E) transcripts using the expression level by cells with HC-HA/PTX3 + scRNA at time 0 set as 1. (* p > 0.05, n = 3; + scRNA represented by darker line) and by immunofluorescence staining to CXCR4 and Pax6 (FIG. 36F, nuclear counterstaining by Hoechst 33342, bar = 25 µm).

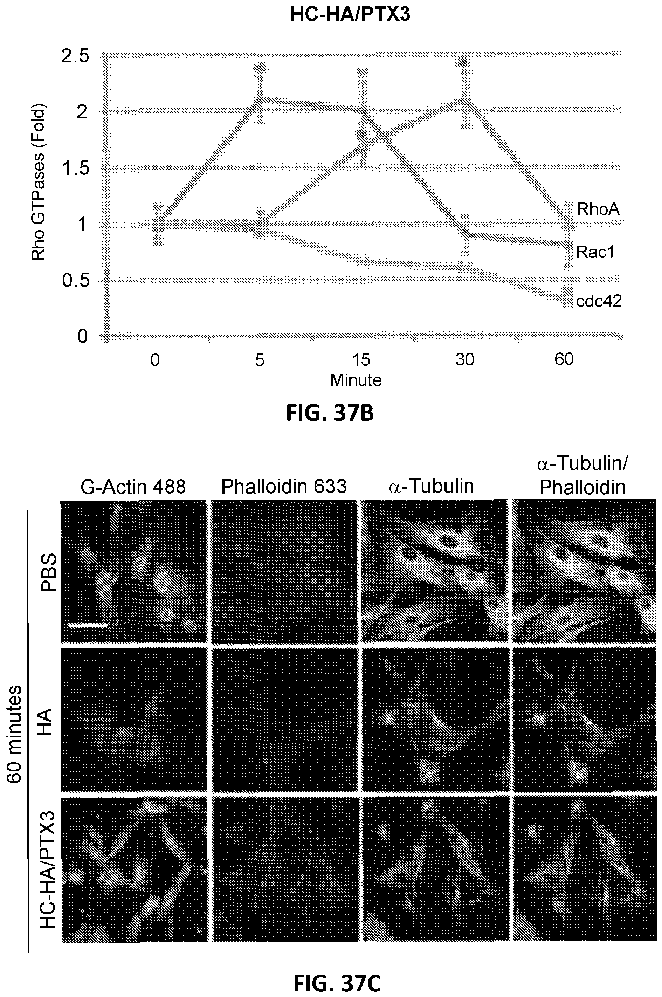

FIGS. 37A-37C illustrate cytoskeletal change by HA and HC-HA/PTX3 in LNCs correlates with Rho GTPase RhoA, Rac1 and Cdc42 effectors within 60 minutes. **FIG. 37A** illustrates phase images of LNC treated with HC-HA/PTX. **FIG. 37B** illustrates graphs of RhoA, Rac1, and Cdc42 activities after treatment with HA and HC-HA/PTX3. **FIG. 37C** illustrates double immunostaining of DNase I/Phalloidin (G-actin/F-actin).

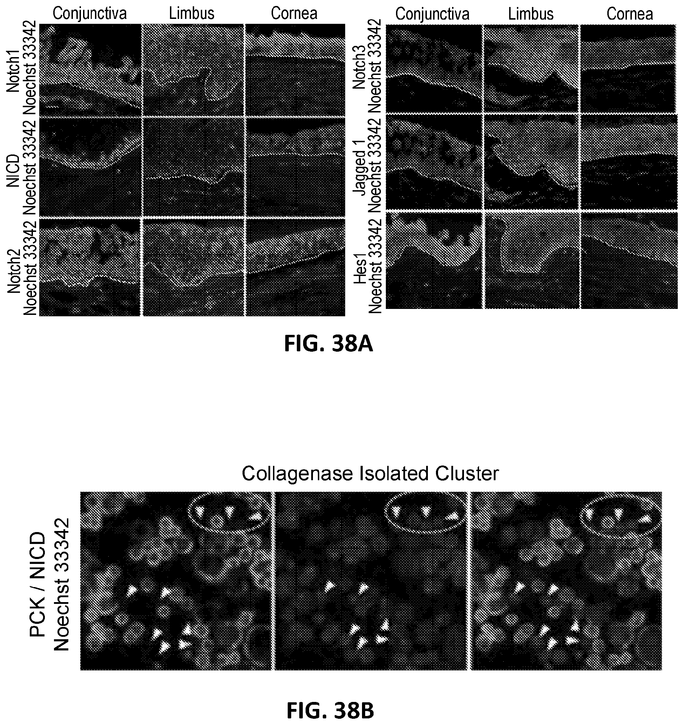

**FIGS. 38A-38B** illustrate expression of Notch ligands and receptors in human cornea, limbus, and conjunctiva. **FIG. 38A** illustrates in vivo signaling of notch receptors (Notch 1, Notch1 intracellular domain (NICD), Notch2, and Notch 3) and Notch ligands (Jagged 1, Delta). **FIG. 38B** illustrates in vivo notch signaling in freshly collagenase isolated clusters.

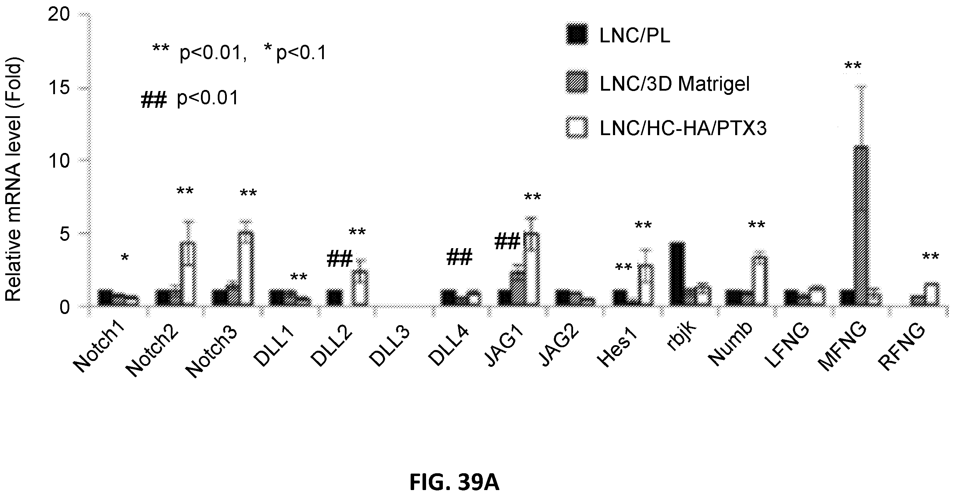

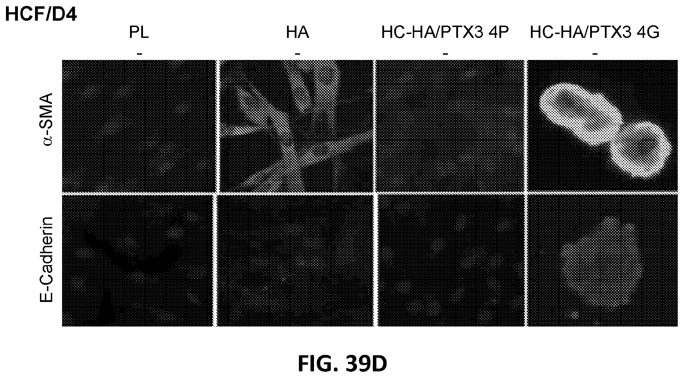

**FIGS. 39A-39E** illustrate expression of Notch signal on plastic, 3D Matrigel, and HC-HA/PTX3.

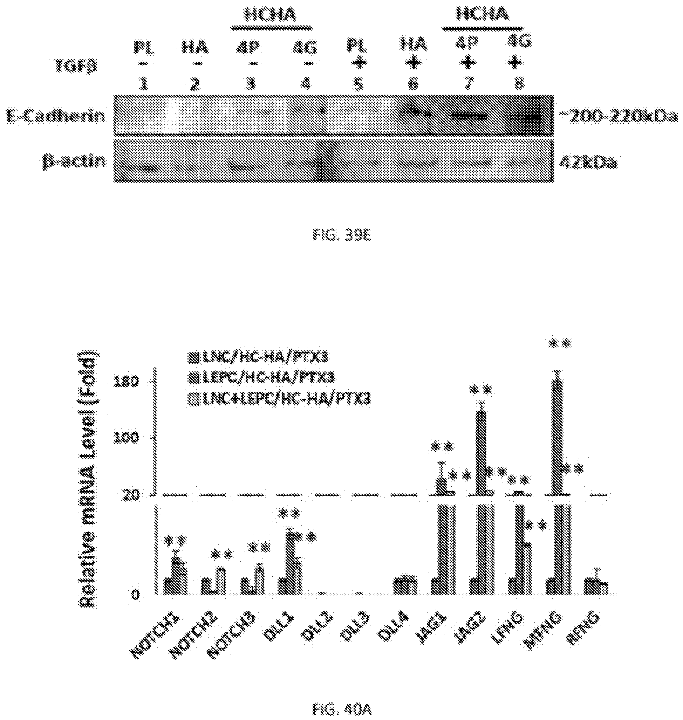

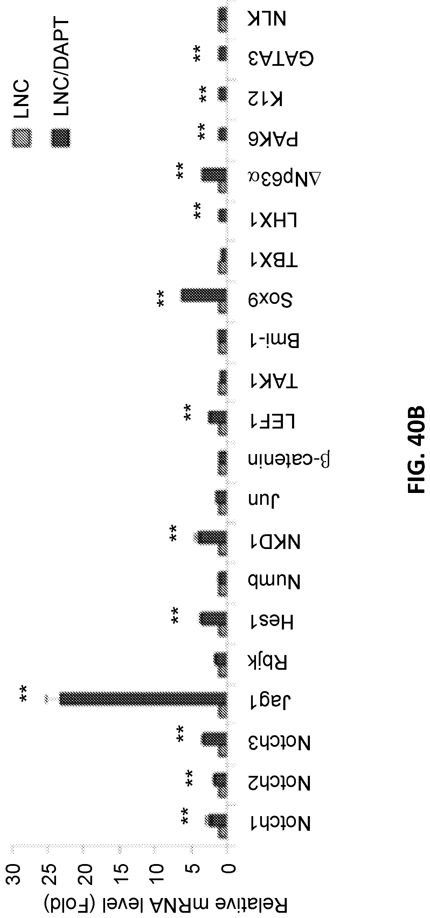

**FIGS. 40A-40B** illustrate expression of canonical Notch signaling in LEPC and LNC on immobilized HC-HA/PTX3 at 48 hours.

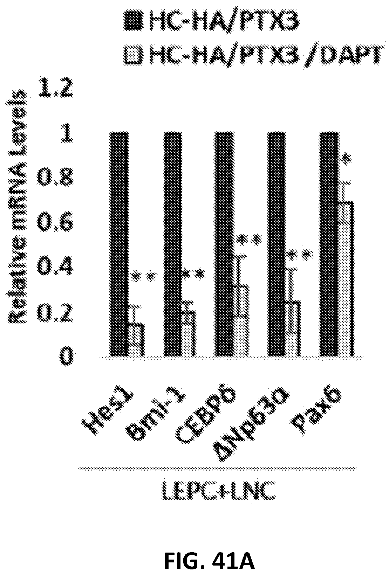

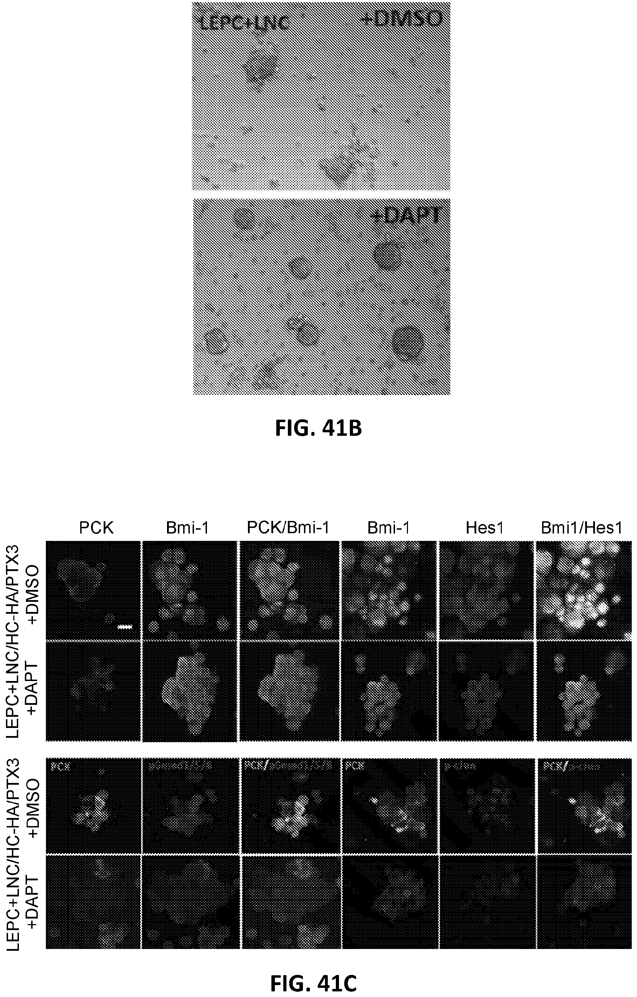

**FIGS. 41A-41C** illustrate blocking Notch signaling inhibits BMP and non-canonical Wnt in LEPC and LNC on immobilized HC-HA/PTX3 at 48 hours. **FIG. 41A** illustrates a graph of mRNA levels of various genes following treatment of HC-HA/PTX3 and HC-HA/PTX3/DAPT in LNCS renunioned with LEPC. **FIG. 41B** illustrates immunostaining with various markers.

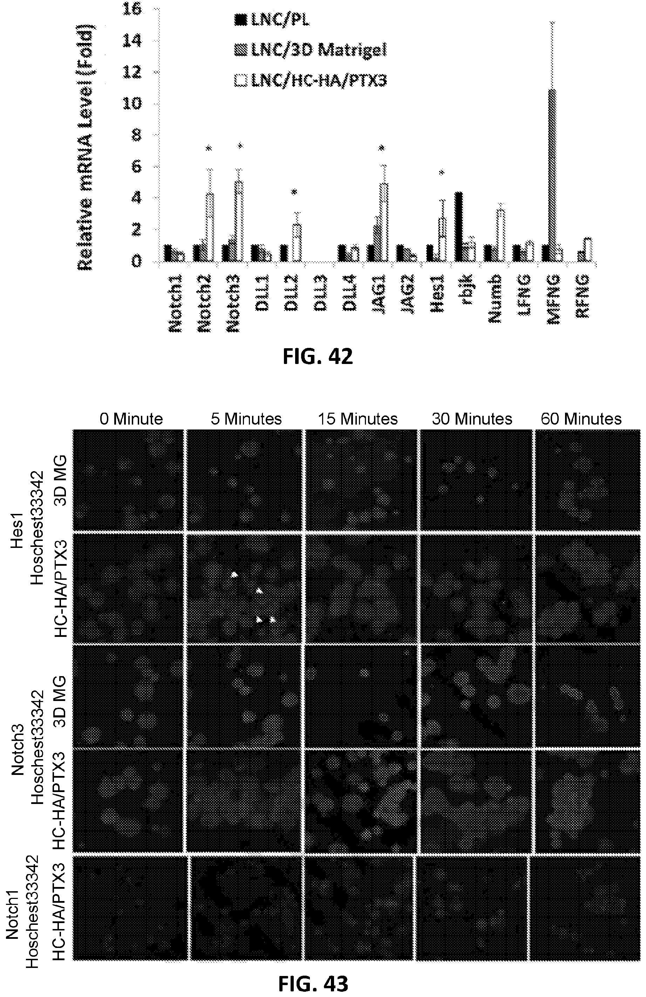

**FIG. 42** illustrates Notch signaling in LNC on plastic, 3D Matrigel or immobilized HC-HA/PTX3 at 48 hours.
**FIG. 43** illustrate immunofluorescence (IF) staining of Hes1, Notch3, and Notch1 in the presence of HC-HA/PTX3 or 3D Matrigel (MG).

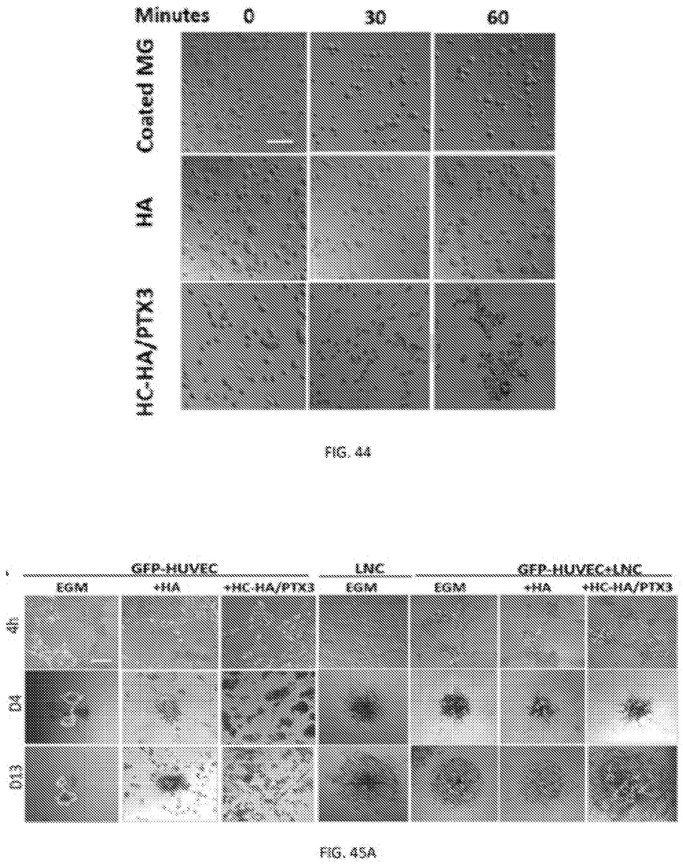

**FIG. 44** illustrates phase contrast microcopy image showing cell aggregation was promoted by soluble HC-HA/PTX3 as early as 60 minutes but not in HA or coated Matrigel (MG).

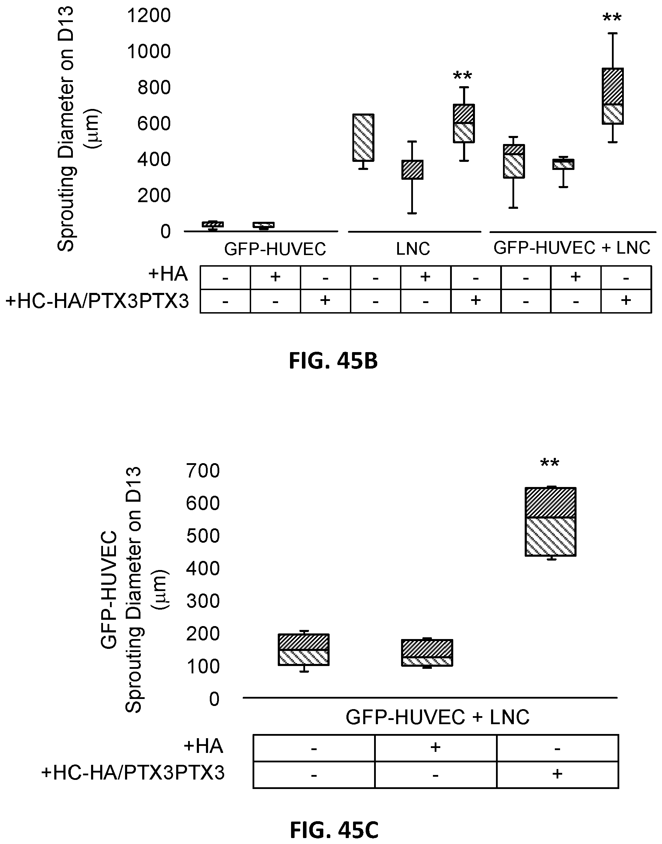

**FIGS. 45A-45C** illustrate soluble HC-HA/PTX3, but not HA or 3D MG alone, promotes angiogenesis sprouting. **FIG. 45A** illustrates phase contrast microscopy images showing cell morphology reunion aggregates at 4h. **FIGS. 45B-45C** illustrates a graph of diameter of sprouting outgrowth measured from the two sides of invading edges on D13.

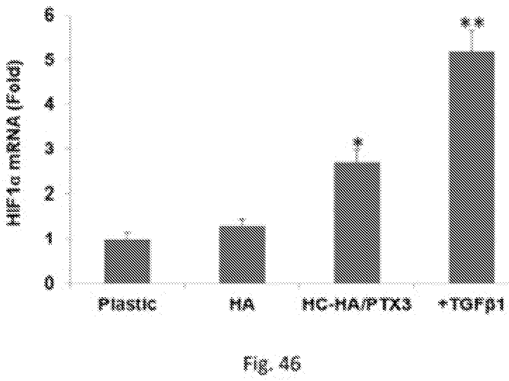

**FIG. 46** illustrates a graph of HIF1α mRNA expression in human corneal fibroblasts (HCF) that were seeded on plastic with or without immobilized HA, HC-HA/PTX3 complex and then treated with or without TGFβ1.

### DETAILED DESCRIPTION OF THE DISCLOSURE

The references to methods of treatment by therapy or surgery or in vivo diagnosis methods described below are to be interpreted as references to fetal support tissue products as claimed for use in such methods.

Blood vessels comprise endothelial cells, which form the inner lining of the vessel wall, and pericytes, which are found on the surface of the vessel. Blood vessels are generated by two different processes, angiogenesis which involves the formation of new vessels from existing vessels, and vasculogenesis, which involves the de novo formation of vessels. Normal angiogenesis is a complex, multi-step process including the creation the gradient formation of matrix-bound growth factor (GF) (e.g. VEGF-A, bFGF, PDGF-BB), migration and proliferation of endothelial cells (EC), dissolution of the extracellular matrix, and recruitment of mural cells (e.g., pericytes) to stabilize capillary development. Abnormal angiogenesis associated with tumors is characterized by vessel leakiness and hemorrhage, and is often associated with the lack of pericytes and/or accompanied by inability to bind VEGF-A associated matrix heparan.

Pericytes in the brain are derived from neural crest cells, and promote both neurogenesis and vasculogenesis, a process referred to herein as neurovasculogenesis. Pericytes have diverse support functions to regulate blood-brain barrier (BBB) integrity, angiogenesis, influence neuroinflammatory response, and have multipotent stem cell activity. Pericyte deficiency has been noted as an early hallmark in diabetes-associated microvascular diseases, such as retinopathy and nephropathy, and may contribute to abnormal angiogenesis, resulting in vessel leakiness and hemorrhage, increased metastases in mouse tumor models, cerebrovascular dysfunction in complex neurological disease such as Alzheimer's disease, and amyotrophic lateral sclerosis.

Provided herein, are methods of promoting vasculogenesis and/or normal angiogenesis in an individual in need thereof, comprising contacting a tissue comprising endothelial cells and pericytes or neural crest progenitor cells with a fetal support tissue product as claimed. In some embodiments, the vasculogenesis occurs as part of neurovasculogenesis. In some embodiments, neurovasculogenesis further comprises neurogenesis. Further provided herein, , are methods of treating an ischemic condition in an individual in need thereof, comprising contacting an ischemic tissue with a fetal support tissue product as claimed. Provided herein, are methods of treating a neuropathic condition in an individual in need thereof, comprising contacting an ischemic tissue with a fetal support tissue product as claimed.

Further provided herein, but not claimed are methods of inhibiting abnormal angiogenesis in an in an individual in need thereof, comprising contacting a tissue comprising endothelial cells with a fetal support tissue product. In examples not claimed, the tissue lacks pericytes. In examples not claimed, the method further comprises selecting the individual by detecting an absence of pericyte markers.

### Certain Definitions

Unless defined otherwise, all technical and scientific terms used herein have the same meaning as is commonly understood by one of skill in the art to which the claimed subject matter belongs.

As used herein, in some embodiments, ranges and amounts are expressed as "about" a particular value or range. About also includes the exact amount. Hence "about 5 µg" means "about 5 µg" and also "5 µg." Generally, the term "about" includes an amount that would be expected to be within experimental error.

As used herein, "fetal support tissue product" means any isolated product derived from tissue used to support the development of a fetus. Examples of fetal support tissue product includes, but are not limited to, (i) placental amniotic membrane (PAM), or substantially isolated PAM, (ii) umbilical cord amniotic membrane (UCAM) or substantially isolated UCAM, (iii) chorion or substantially isolated chorion, (iv) amnion-chorion or substantially isolated amnion-chorion, (v) placenta or substantially isolated placenta, (vi) umbilical cord or substantially isolated umbilical cord, or (vii) any combinations thereof. In some embodiments, the fetal support tissue is selected from the group consisting of placental amniotic membrane (PAM), umbilical cord amniotic membrane (UCAM), chorion, amnion-chorion, placenta, umbilical cord, and any combinations thereof. In some embodiments, the fetal support tissue comprises umbilical cord. Fetal support tissue product includes any form of the fetal support tissue, including cryopreserved, terminally-sterilized, lyophilized fetal support tissue or powders resulting from grinding fetal support tissue. In some embodiments, the fetal support tissue product is ground, pulverized, morselized, a graft, a sheet, a powder, a gel, a homogenate, an extract, or a terminally-sterilized product.

As used herein, "placenta" refers to the organ that connects a developing fetus to the maternal uterine wall to allow nutrient uptake, waste elimination, and gas exchange via the maternal blood supply. The placenta is composed of three layers. The innermost placental layer surrounding the fetus is called amnion. The allantois is the middle layer of the placenta (derived from the embryonic hindgut); blood vessels originating from the umbilicus traverse this membrane. The outermost layer of the placenta, the chorion, comes into contact with the endometrium. The chorion and allantois fuse to form the chorioallantoic membrane.

As used herein, "chorion" refers to the membrane formed by extraembryonic mesoderm and the two layers of trophoblast. The chorion consists of two layers: an outer formed by the trophoblast, and an inner formed by the somatic mesoderm; the amnion is in contact with the latter. The trophoblast is made up of an internal layer of cubical or prismatic cells, the cytotrophoblast or layer of Langhans, and an external layer of richly nucleated protoplasm devoid of cell boundaries, the syncytiotrophoblast. The avascular amnion is adherent to the inner layer of the chorion.

As used herein, "amnion-chorion" refers to a product comprising amnion and chorion. In some embodiments, the amnion and the chorion are not separated (i.e., the amnion is naturally adherent to the inner layer of the chorion). In some embodiments, the amnion is initially separated from the chorion and later combined with the chorion during processing.

As used herein, "umbilical cord" refers to the organ that connects a developing fetus to the placenta. The umbilical cord is composed of Wharton's jelly, a gelatinous substance made largely from mucopolysaccharides. It contains one vein, which carries oxygenated, nutrient-rich blood to the fetus, and two arteries that carry deoxygenated, nutrient-depleted blood away.

As used herein, "placental amniotic membrane" (PAM) refers to amniotic membrane derived from the placenta. In some embodiments, the PAM is substantially isolated.

As used herein, "umbilical cord amniotic membrane" (UCAM) means amniotic membrane derived from the umbilical cord. UCAM is a translucent membrane. The UCAM has multiple layers an epithelial layer, a basement membrane; a compact layer; a fibroblast layer; and a spongy layer. It lacks blood vessels or a direct blood supply. In some embodiments, the UCAM comprises Wharton's Jelly. In some embodiments, the UCAM comprises blood vessels and/or arteries. In some embodiments, the UCAM comprises Wharton's Jelly and blood vessels and/or arteries.

As used herein, "human tissue" means any tissue derived from a human body. In some embodiments, the human tissue is a fetal support tissue selected from the group consisting of placental amniotic membrane, umbilical cord, umbilical cord amniotic membrane, chorion, amnion-chorion, placenta, or any combination thereof.

As used herein, "minimal manipulation" means (1) for structural tissue, processing that does not alter the original relevant characteristics of the tissue relating to the tissue's utility for reconstruction, repair, or replacement; and (2) for cells or nonstructural tissues, processing that does not alter the relevant biological characteristics of cells or tissues.

As used herein, "graft" means a matrix of proteins (e.g., collagen and elastin) and glycans (e.g., dermatan, hyaluronan, and chondroitin) that is used to replace damaged, compromised, or missing tissue. In certain instances, the matrix is laid down and host cells gradually integrate into the matrix.

As used herein, "sheet" means any continuous expanse or surface. In some embodiments, a sheet of a fetal support tissue product is substantially flattened. In some embodiments, a sheet of a fetal support tissue product is flat. In some embodiments, a sheet of fetal support tissue product is tubular. In some embodiments, the sheet is any shape or size suitable for the wound to be treated. In some embodiments, the sheet is a square, circle, triangle, or rectangle.

The term "fresh fetal support tissue" refers to fetal support tissue that is less than 10 days old following birth, and which is in substantially the same form as it was following birth. In some embodiments, the fresh fetal support tissue comprises fetal support tissue cells. In some embodiments, the fetal support tissue cells comprise pericytes. In some embodiments, at least 50%, at least 60%, at least 70%, at least 80%, at least 90%, or at least 95% of the biological activity of the cell support tissue cells is maintained.

"Substantially isolated" or "isolated" when used in the context of a fetal support tissue product means that the fetal support tissue product is separated from most other non-fetal support tissue materials (e.g., other tissues, red blood cells, veins, arteries) derived from the original source organism.

As used herein, the phrase "wherein the biological and structural integrity of the isolated fetal support tissue product is substantially preserved" means that when compared to the biological activity and structural integrity of fresh fetal support tissue, the biological activity and structural integrity of the isolated fetal support tissue has only decreased by about 5%, about 10%, about 15%, about 20%, about 25%, about 30%, about 35%, about 40%, about 50%, or about 60%.

As used herein, "processing" means any activity performed on a fetal support tissue or a preparation comprising HC-HA/PTX3, other than recovery, donor screening, donor testing, storage, labeling, packaging, or distribution, such as testing for microorganisms, preparation, sterilization, steps to inactivate or remove adventitious agents, preservation for storage, and removal from storage.

As used herein, the terms "purified" and "isolated" mean a material (e.g., HC-HA/PTX3 complex) substantially or essentially free from components that normally accompany it in its native state. In some embodiments, "purified" or "isolated" mean a material (e.g., HC-HA/PTX3 complex) is about 50% or more free from components that normally accompany it in its native state, for example, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99% free from components that normally accompany it in its native state.

As used herein, "biological activity" means the activity of polypeptides and polysaccharides of the fetal support tissue product comprising HC-HA/PTX3. In some embodiments, the biological activity of polypeptides and polysaccharides found in the fetal tissue support product is anti-inflammatory, anti-scarring, anti-angiogenic, or anti-adhesion. In some embodiments, the biological activity refers to the in vivo activities of the HC-HA/PTX3 complex in the fetal tissue support product or physiological responses that result upon in vivo administration of the fetal support tissue product. In some embodiments, the biological activity of HC-HA/PTX3 complex in the fetal support tissue product is substantially preserved. In some embodiments, the activity of polypeptides and polysaccharides found in the fetal tissue support product is promoting wound healing. In some embodiments, the activity of polypeptides and polysaccharides found in the fetal support tissue product is preventing scarring. In some embodiments, the activity of polypeptides and polysaccharides found in the fetal support tissue product is reducing inflammation. Biological activity, thus, encompasses therapeutic effects and pharmaceutical activity of the HC-HA/PTX3 complex in the fetal support tissue product.

As used herein, "structural integrity" means the integrity of stroma and basement membrane that make up the fetal support tissue product. In some embodiments, the structural integrity of the fetal support tissue product results in suture pull out strength.

As used herein, a reconstituted HC-HA/PTX3 (rcHC-HA/PTX3) complex is an HC-HA/PTX3 complex that is formed by assembly of the component molecules of the complex in vitro. The process of assembling the rcHC-HA/PTX3 includes reconstitution with purified native proteins or molecules from biological source, recombinant proteins generated by recombinant methods, or synthesis of molecules by in vitro synthesis. In some instances, the purified native proteins used for assembly of the rcHC-HA/PTX3 are proteins in a complex with other proteins (i.e. a multimer, a multichain protein or other complex). In some instances, PTX3 is purified as a multimer (e.g. a homomultimer) from a cell and employed for assembly of the rcHC-HA/PTX3 complex.

As used herein, a purified native HC-HA/PTX3 (nHC-HA/PTX3) complex refers to an HC-HA/PTX3 complex that is purified from a biological source such as a cell, a tissue or a biological fluid. In some embodiments, the nHC-HA/PTX3 is purified from a fetal support tissue. In some embodiments the nHC-HA/PTX3 is purified from amniotic membrane. In some embodiments the nHC-HA/PTX3 is purified from umbilical cord. Such complexes are generally assembled in vivo in a subject or ex vivo in cells, tissues, or biological fluids from a subject, including a human or other animal.

As used herein, a PTX3/HA complex refers to an intermediate complex that is formed by contacting PTX3 with immobilized HA. In the methods provided herein, the PTX3/HA complex is the generated prior to the addition of HC1 to HA.

As used herein, "hyaluronan," "hyaluronic acid," or "hyaluronate" (HA) are used interchangeably to refer to a substantially non-sulfated linear glycosaminoglycan (GAG) with repeating disaccharide units of D-glucuronic acid and N-acetylglucosamine (D-glucuronosyl-N-acetylglucosamine).

As used herein, the term "tissue having unwanted changes" refers to tissue that is degenerated due to, for example, a degenerative disease (for example, arthritis, multiple sclerosis, Parkinson's disease, muscular dystrophy, and Huntington's disease) or aging; scar tissue; or damaged due to an insult, such as a burn, wound, laceration, injury, ulcer, surgery, or due to ischemia.

As used herein, the term "mesenchymal cell characteristic of the tissue" refers to specialized cells characteristic of the tissue, such as, for example, cardiomyocytes, osteoblasts (bone cells), chondrocytes (cartilage cells), myocytes (muscle cells), and adipocytes (fat cells).

As used herein, the term "high molecular weight" or "HMW," as in high molecular weight hyaluronan (HMW HA), is meant to refer to HA that has a weight average molecular weight that is greater than about 500 kilodaltons (kDa), such as, for example, between about 500 kDa and about 10,000 kDa, between about 800 kDa and about 8,500 kDa, between about 1100 kDa and about 5,000 kDa, or between about 1400 kDa and about 3,500 kDa. In some embodiments, the HMW HA has a weight average molecular weight of 3000 kDa or greater. In some embodiments, the HMW HA has a weight average molecular weight of 3000 kDa. In some embodiments, the HMW HA is Healon^{®} with a weight average molecular weight of about 3000 kDa. In some embodiments, HMW HA has a molecular weight of between about 500 kDa and about 10,000 kDa. In some embodiments, HMW HA has a molecular weight of between about 800 kDa and about 8,500 kDa. In some embodiments, HMW HA has a molecular weight of about 3,000 kDa.

As used herein, the term "low molecular weight" or "LMW," as in low molecular weight hyaluronan (LMW HA), is meant to refer to HA that has a weight average molecular weight that is less than 500 kDa, such as for example, less than about 400 kDa, less than about 300 kDa, less than about 200 kDa, less than about 100 kDa, less than about 50 kDa, less than about 40 kDa, less than about 30 kDa, less than about 20 kDa, about 200-300 kDa, about 1-300 kDa, about 15 to about 40 kDa, or about 8-10kDa.

As used herein, pentraxin 3, or PTX3, protein or polypeptide refers to any PTX3 protein, including a recombinantly produced protein, a synthetically produced protein, a native PTX3 protein, and a PTX3 protein extracted from cells or tissues. PTX3 include multimeric forms (e.g. homomultimer) of PTX3, including, , dimeric, trimeric, tetrameric, pentameric, hexameric, tetrameric, octameric, and other multimeric forms naturally or artificially produced.

As used herein, tumor necrosis factor stimulated gene-6 (TSG-6) refers to any TSG-6 protein or polypeptide, including , a recombinantly produced protein, a synthetically produced protein, a native TSG-6 protein, and a TSG-6 protein extracted from cells or tissues.

As used herein, inter-α-inhibitor (IαI) refers to the IαI protein comprised of light chain (i.e., bikunin) and one or both heavy chains of type HC1 or HC2 covalently connected by a chondroitin sulfate chain. In some embodiments, the source of IαI is from serum or from cells producing IαI e.g., hepatic cells or amniotic epithelial or stromal cells or umbilical epithelial or stromal cells under a constitutive mode stimulation by proinflammatory cytokines such as IL-1 or TNF-α.

As used herein, a "hyaluronan binding protein," "HA binding protein," or "HABP" refers to any protein that specifically binds to HA.

The terms "effective amount" or "therapeutically effective amount," as used herein, refer to a sufficient amount of an agent or a compound being administered which will relieve to some extent one or more of the symptoms of the disease or condition being treated. In some embodiments, the result is a reduction and/or alleviation of the signs, symptoms, or causes of a disease, or any other desired alteration of a biological system. For example, an "effective amount" for therapeutic uses is the amount of the composition including a compound as disclosed herein required to provide a clinically significant decrease in disease symptoms without undue adverse side effects. In some embodiments, an appropriate "effective amount" in any individual case is determined using techniques, such as a dose escalation study. The term "therapeutically effective amount" includes, for example, a prophylactically effective amount. An "effective amount" of a compound disclosed herein, is an amount effective to achieve a desired effect or therapeutic improvement without undue adverse side effects. It is understood that, in some cases, "an effective amount" or "a therapeutically effective amount" varies from subject to subject, due to variation in metabolism of the composition, age, weight, general condition of the subject, the condition being treated, the severity of the condition being treated, and the judgment of the prescribing physician. In some embodiments, an effective amount is an amount of a product or compound sufficient to promote vasculogenesis or normal angiogenesis in a tissue.

As used herein, the terms "subject," "individual" and "patient" are used interchangeably. None of the terms are to be interpreted as requiring the supervision of a medical professional (e.g., a doctor, nurse, physician's assistant, orderly, hospice worker). As used herein, the subject is any animal, including mammals (e.g., a human or non-human animal) and nonmammals. In one embodiment of the methods and compositions provided herein, the mammal is a human.

As used herein, the terms "treat," "treating" or "treatment," and other grammatical equivalents, include alleviating, abating or ameliorating one or more symptoms of a disease or condition, ameliorating, preventing or reducing the appearance, severity or frequency of one or more additional symptoms of a disease or condition, ameliorating or preventing the underlying metabolic causes of one or more symptoms of a disease or condition, inhibiting the disease or condition, such as, for example, arresting the development of the disease or condition, relieving the disease or condition, causing regression of the disease or condition, relieving a condition caused by the disease or condition, or inhibiting the symptoms of the disease or condition either prophylactically and/or therapeutically. In a non-limiting example, for prophylactic benefit, an rcHC-HA/PTX3 complex or composition disclosed herein is administered to an individual at risk of developing a particular disorder, predisposed to developing a particular disorder, or to an individual reporting one or more of the physiological symptoms of a disorder.

### Methods of Use

Provided herein, , are methods of promoting vasculogenesis in an individual in need thereof. Provided herein, are methods of promoting neurovasculogenesis in an individual in need thereof. In some embodiments, promoting vasculogenesis and/or neurovasculogenesis in an individual in need thereof comprises contacting a tissue with a fetal support tissue product as claimed. The tissue comprises endothelial cells and pericytes. In some embodiments, the tissue comprises neural crest progenitor cells. In some embodiments, the tissue comprises endothelial cells and the method comprises further recruiting pericytes to the tissue. In some embodiments, the tissue comprises endothelial cells and the method comprises further recruiting neural crest progenitor cells to the tissue. In some embodiments, the tissue is an ischemic tissue. In some embodiments, the methods described herein prevent necrosis of the tissue. In some embodiments, the fetal support tissue product recruits pericytes, neural crest progenitors, or a combination thereof to a site of administration. In some embodiments, the site of administration is a tissue. In some embodiments, the fetal support tissue product reprograms a progenitor cell into a cell that promotes vasculogenesis and/or neurovasculogenesis. In some embodiments, the progenitor cell is a neural crest progenitor cell. In some embodiments, the neural crest progenitor cell is reprogrammed into a pericyte.

Further provided herein, are methods of treating an ischemic condition in an individual in need thereof. In some embodiments, treating an ischemic condition in an individual comprises contacting an ischemic tissue with a fetal support tissue product described herein. The ischemic tissue comprises endothelial cells and pericytes. In some embodiments, the ischemic tissue comprises endothelial cells and the method comprises further recruiting pericytes to the ischemic tissue. In some embodiments, the methods described herein prevent necrosis of the ischemic tissue. In some embodiments, the ischemic condition comprises cardiac ischemia, ischemic colitis, mesenteric ischemia, brain ischemia, acute limb ischemia, cyanosis, and gangrene. Further provided herein, but not claimed are methods of treatment microvascular disease. In examples not claimed the microvascular disease is a diabetes-associated microvascular disease. In examples not claimed the diabetes-associated microvascular disease is retinopathy or nephropathy. In some embodiments, the ischemic condition is a neurotrophic or neuropathic condition. In some embodiments, the neuropathic condition diminishes the function of one nerve or more than one nerve. In some embodiments, the neuropathic condition is a hereditary neuropathy or an acquired neuropathy. In some embodiments, the acquired neuropathy is neuropathy caused by a trauma, an infection, a disease, a medication, a vascular disorder, a vitamin imbalance, or alcoholism. In some embodiments, the disease is diabetes.

In some instances, the tissue is an ocular tissue, a brain tissue, a cardiac tissue, a skin tissue, a joint, a spine, a soft tissue, a muscle tissue, a cartilage, a bone, a tendon, a ligament, a nerve, or an intervertebral disc. In some instances, the tissue is an ocular tissue. In some instances, the tissue is a cardiac tissue. In some instances, the tissue is a skin tissue. In some instances, the tissue having unwanted changes is a joint tissue. In some instances, the tissue is from a spine. In some instances, the tissue is an intervertebral disc. In some instances, the tissue is a soft tissue. In some instances, the tissue is a muscle tissue. In some instances, the tissue is a cartilage. In some instances, the tissue is a bone. In some instances, the tissue is a tendon. In some instances, the tissue is a ligament. In some instances, the tissue is a nerve.

In some instances, the tissue comprises degenerated tissue, a burn, a laceration, ischemic tissue, a wound, an injury, an ulcer, or a surgical incision. In some embodiments, the tissue comprises an ulcer, wound, perforation, burn, surgery, injury, or fistula. In some instances, the tissue comprises a degenerated tissue. In some instances, the tissue comprises a burn. In some instances, the tissue comprises a laceration. In some instances, the tissue comprises an ischemic tissue. In some instances, the tissue comprises a wound. In some instances, the tissue comprises an injury. In some instances, the injury is a myocardial infarction. In some instances, the tissue comprises an ulcer. In some embodiments, the ulcer is a diabetic ulcer. In some instances, the tissue comprises a surgical incision.

In some embodiments, the contacting occurs for a time sufficient vasculogenesis and/or neurovasculogenesis to occur. In some embodiments, the period of time at least 1 day, 2 days, 3 days, 4 days, 5 days, 6 days, 1 week, or 2 weeks.

In some embodiments, the contacting occurs for a time sufficient for the fetal support tissue product to reprogram a progenitor cell into a cell that promotes vasculogenesis and/or neurovasculogenesis. In some embodiments, the progenitor cell is a neural crest progenitor cell. In some embodiments, the neural crest progenitor cell is reprogrammed into a pericyte. In some embodiments, the period of time at least 1 day, 2 days, 3 days, 4 days, 5 days, 6 days, 1 week, or 2 weeks.

In some embodiments, the contacting occurs for a time sufficient to induce gene expression. In some embodiments, the contacting to induce gene expression comprises 1 day, 2 days, 3 days, 4 days, 5 days, 6 days, 1 week, or 2 weeks.

In some embodiments, the contacting occurs for a time sufficient to induce nuclear translocation of a transcription factor. In some embodiments, the contacting to induce nuclear translocation of a transcription factor comprises at least about 5 minutes, 10 minutes, 15 minutes, 20 minutes, 30 minutes, 1 hour, 4 hours, 8 hours, 12 hours, 16 hours, 1 day, 2 days, 3 days, 4 days, or more than 4 days. at least 1 day, 2 days, 3 days, 4 days, 5 days, 6 days, 1 week, or 2 weeks.

In some embodiments, recruiting a neural crest progenitor cell to the tissue comprises contacting the tissue with a fetal support tissue product described herein. In some embodiments, recruiting pericytes to the tissue comprises administering to the tissue a fetal support tissue product described herein. In some embodiments, the fetal support tissue product attracts pericytes, neural crest progenitor cells, or a combination thereof to a site of the administration. In some embodiments, the pericytes are cells expressing a pericyte phenotype. In some embodiments, the cells expressing a pericyte phenotype are limbal niche cells (LNCs).

In some embodiments, the ratio of endothelial cells to pericytes in the tissue is 1:1, 2:1, 3:1, 4:1, 5:1, 6:1, 7:1, 8:1, 9:1, or 10:1. In some embodiments, the tissue is contacted with pericytes to reach a ratio of endothelial cells to pericytes is 1:1, 2:1, 3:1, 4:1, 5:1, 6:1, 7:1, 8:1, 9:1, or 10:1. In some embodiments, pericytes are recruited to the tissue to reach a ratio of endothelial cells to pericytes of 1:1, 2:1, 3:1, 4:1, 5:1, 6:1, 7:1, 8:1, 9:1, or 10:1

In some embodiments, the fetal support tissue product comprises an extract of fetal support tissue, a fetal support tissue homogenate, a fetal support tissue powder, morselized fetal support tissue, pulverized fetal support tissue, ground fetal support tissue, a fetal support tissue graft, purified HC-HA/PTX3, reconstituted HC-HA/PTX3 or a combination thereof.

Provided herein, are methods of promoting vasculogenesis and/or neurovasculogenesis in an individual in need thereof, wherein contacting a tissue with a fetal support tissue product as claimed modulates gene expression. In some embodiments, the fetal support tissue product comprises native HC-HA/PTX3 complex, reconstituted HC-HA/PTX3 (rcHC-HA/PTX3) complex, or a combination thereof. In some embodiments, the HC-HA/PTX3 results in an increase in an expression of angiogenic genes, neurogenic genes, or a combination thereof. In some embodiments, the HC-HA/PTX3 results in an increase in an expression of angiogenic genes, neurogenic genes, or a combination thereof by at least about 0.5X, 1.0X, 1.5X, 2.0X, 3.0X, 4.0X, or more than 4.0X. In some embodiments, the angiogenic genes, neurogenic genes, or a combination thereof comprises *VEGF, PDGFα, PDGFβ, CD31, IGF-1, NGF, p75^{NTR}*, *Sox-2, Musashi-1, PDGFR*α*, PDGFRβ*, *VEGFRI,* or *VEGFR2.*

In some embodiments, a tissue is contacted with a fetal support tissue product comprising native HC-HA/PTX3 complex, reconstituted HC-HA/PTX3 (rcHC-HA/PTX3) complex, or a combination thereof for a sufficient amount of time to modulate gene expression. In some embodiments, the tissue is contacted with a fetal support tissue product comprising native HC-HA/PTX3 complex for at least about 5 minutes, 10 minutes, 15 minutes, 20 minutes, 30 minutes, 1 hour, 4 hours, 8 hours, 12 hours, 16 hours, 1 day, 2 days, 3 days, 4 days, or more than 4 days. at least 1 day, 2 days, 3 days, 4 days, 5 days, 6 days, 1 week, or 2 weeks.

In some embodiments, HC-HA/PTX3 modulates cellular function. In some embodiments, HC-HA/PTX3 promotes apoptosis, necrosis, or a combination thereof. In some embodiments, HC-HA/PTX3 promotes apoptosis, necrosis, or a combination thereof by at least about 0.5X, 1.0X, 1.5X, 2.0X, 3.0X, 4.0X, or more than 4.0X. In some embodiments, HC-HA/PTX3 inhibits cell proliferation. In some embodiments, HC-HA/PTX3 inhibits cell proliferation by at least about 0.5X, 1.0X, 1.5X, 2.0X, 3.0X, 4.0X, or more than 4.0X.

In some embodiments, HC-HA/PTX3 modulates cell signaling. In some embodiments, HC-HA/PTX3 modulates cell signaling by increasing gene expression, protein expression, protein activity, or combinations thereof. In some embodiments, HC-HA/PTX3 modulates cell signaling by decreasing gene expression, protein expression, protein activity, or combinations thereof. In some embodiments, HC-HA/PTX3 modulates SDF-1/CXCR signaling. In some embodiments, HC-HA/PTX3 modulates HIF1 signaling. In some embodiments, HIF1 comprises H1F1α. In some embodiments, HIF1 comprises HIF1β. In some embodiments, HC-HA/PTX3 modulates TGFβ signaling. In some embodiments, HC-HA/PTX3 modulates non-canonical TGFβ signaling. In some embodiments, HC-HA/PTX3 modulates CD44ICD signaling. In some embodiments, HC-HA/PTX3 modulates Hes signaling. In some embodiments, HC-HA/PTX3 modulates Pax6 signaling. In some embodiments, HC-HA/PTX3 modulates Notch signaling. In some embodiments, HC-HA/PTX3 modulates Notch signaling by modulating expression of Notch ligands, Notch receptors, or a combination thereof. In some embodiments, the Notch ligands comprises Notch 1, Notch 2, Notch 3, Notch 4, Jagged 1, Jagged 2, Jagged 3, DLL1, DLL2, DLL3, or DLL4. In some embodiments, the Notch ligands comprises Notch 2, Notch 3, Jagged 1 or DLL2.

In some embodiments, HC-HA/PTX3 modulates multiple signaling pathways. In some embodiments, HC-HA/PTX3 modulates SDF-1/CXCR, HIF1, TGFβ, CD44ICD, Hes, Pax6, Notch signaling, or combinations thereof.

Provided herein, in certain embodiments, are methods of promoting vasculogenesis and/or neurovasculogenesis in an individual in need thereof, wherein contacting a tissue with a fetal support tissue product results in cell reprograming. In some embodiments, HC-HA/PTX3 modulates cell reprograming. In some embodiments, HC-HA/PTX3 modulates cell aggregation, cell shape, or an expression of a cell-specific marker.

In some embodiments, HC-HA/PTX3 reprograms LNCs to a progenitor phenotype. In some embodiments, HC-HA/PTX3 reprograms LNCs to a vascular progenitor phenotype. In some embodiments, HC-HA/PTX3 modulates expression of FLK-1, CD34, CD31, α-SMA, PDGFRβ, NG2, Pax6, p75^{NTR}, Musashi-1, Sox2, Nestin, Msx1, FoxD3, FLK-1, PDGFRβ, CD31, or combinations thereof. In some embodiments, HC-HA/PTX3 modulates expression Pax6. In some embodiments, a time sufficient to reprogram LNCs is at least 1 hour, 2 hours, 3 hours, 4 hours, 5 hours, 6 hours, 7 hours, 8 hours, 9 hours, 10 hours, 11 hours, 12 hours, 24 hours, 36 hours, 2 days, 3 days, 4 days, 5 days, 6 days, 7 days, 2 weeks, 3 weeks, or 4 weeks.

In some embodiments the methods further comprise contacting a tissue with TGFβ1. In some embodiments, additional administration of TGFβ1is required to perform the methods described herein. In some embodiments, additional administration of TGFβ1is not required to perform the methods described herein. In some embodiments, the cell is contacted simultaneously with a fetal support tissue product comprising HC-HA/PTX3 and TGFβ1. In some embodiments, the tissue is contacted sequentially with the fetal support tissue product comprising HC-HA/PTX3 first and then the TGFβ1. In some embodiments, the tissue is contacted sequentially with the TGFβ1 first and then the fetal support tissue product comprising HC-HA/PTX3. In some embodiments, the TGFβ1 is administered in a therapeutically effective amount. In some embodiments, a therapeutically effective amount of TGFβ1 is an amount of TGFβ1 sufficient to enable the fetal support tissue product comprising HC-HA/PTX3 to perform the methods described herein.

### Fetal Support Tissue Products

In some embodiments, a fetal support tissue product is ground fetal support tissue, pulverized fetal support tissue, powdered fetal support tissue, micronized fetal support tissue, morselized fetal support tissue, a fetal support tissue graft, a fetal support tissue sheet, , a fetal support tissue homogenate, a fetal support tissue extract, or any combinations thereof. In some embodiments, the fetal support tissue product is terminally-sterilized. The fetal support tissue product is a purified native HC-HA/PTX3 complex, a reconstituted HC-HA/PTX3, or a combination thereof. In some embodiments, the fetal support tissue product is pulverized, powdered, or micronized fetal support tissue. In some embodiments, the fetal support tissue product is morselized fetal support tissue. In some embodiments, the fetal support tissue product is an extract of a fetal support tissue. In some embodiments, the fetal support tissue is a placental amniotic membrane, umbilical cord, umbilical cord amniotic membrane, chorion, amnion-chorion, placenta, amniotic stroma, amniotic jelly, or any combination thereof.

In some embodiments, the fetal support tissue product is an umbilical cord product, an amniotic membrane product, or umbilical cord amniotic membrane product. In some embodiments, the umbilical cord product comprises umbilical cord amniotic membrane and at least some Wharton's jelly. In some embodiments, the umbilical cord product lacks umbilical cord vein and arteries.

In some embodiments, the fetal support tissue product is purified native HC-HA/PTX3 complex (nHC-HA/PTX3) from a fetal support tissue. In some embodiments, the fetal support tissue product is a reconstituted HC-HA/PTX3 complex (rHC-HA/PTX3). In some embodiments, the fetal support tissue product consists essentially of nHC-HA/PTX3. In some embodiments, the fetal support tissue product consists essentially of rcHC-HA/PTX3. In some embodiments, the fetal support tissue product a combination of nHC-HA/PTX3 and rcHC-HA/PTX3. In some embodiments, the nHC-HA/PTX3 or the rcHC-HA/PTX3 further comprises a small leucine rich proteoglycan (SLRP). In some embodiments, the SLRP is a class I, class II or class II SLRP. In some embodiments, the SLRP is selected from among class I SLRPs, such as decorin and biglycan. In some embodiments, the SLRP is selected from among class II SLRPs, such as fibromodulin, lumican, PRELP (proline arginine rich end leucine-rich protein), keratocan, and osteoadherin. In some embodiments, the SLRP is selected from among class III SLRPs, such as epipycan and osteoglycin. In some embodiments, the SLRP is selected from among bikunin, decorin, biglycan, and osteoadherin. In some embodiments, the SLRP comprises a glycosaminoglycan. In some embodiments, the SLRP comprises keratan sulfate.

### Generation of fetal support tissue products

In some embodiments, the fetal support tissue product is derived from an umbilical cord (UC) tissue. In some embodiments, the fetal support tissue product is derived from an amniotic membrane (AM) tissue. In some embodiments, the fetal support tissue product is derived from an umbilical cord amniotic membrane tissue. In some embodiments, the fetal support tissue product comprises: isolated fetal support tissue that does not comprise a vein or an artery. In some embodiments, the fetal support tissue product comprises: isolated fetal support tissue that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *Treponema pallidum,* wherein the natural structural integrity of the fetal support tissue product is substantially preserved for at least 15 days after initial procurement. In some embodiments, the fetal support tissue product comprises umbilical cord amniotic membrane and Wharton's Jelly. In some embodiments, the biological activity of HC-HA/PTX3 complex in the fetal support tissue product is substantially preserved. In some embodiments, the biological activity of HC-HA/PTX3 complex in the fetal support tissue product is substantially preserved for at least 15 days. In some embodiments, the biological and structural integrity of the fetal support tissue product is substantially preserved for at least 20 days after initial procurement. In some embodiments, the biological and structural integrity of the fetal support tissue product is substantially preserved for at least 25 days after initial procurement. In some embodiments, the biological and structural integrity of the fetal support tissue product is substantially preserved for at least 30 days after initial procurement. In some embodiments, the biological and structural integrity of the fetal support tissue product is substantially preserved for at least 35 days after initial procurement. In some embodiments, the biological and structural integrity of the fetal support tissue product is substantially preserved for at least 40 days after initial procurement. In some embodiments, the biological and structural integrity of the fetal support tissue product is substantially preserved for at least 45 days after initial procurement. In some embodiments, the biological and structural integrity of the fetal support tissue product is substantially preserved for at least 50 days after initial procurement. In some embodiments, the biological and structural integrity of the fetal support tissue product is substantially preserved for at least 55 days after initial procurement. In some embodiments, the biological and structural integrity of the fetal support tissue product is substantially preserved for at least 60 days after initial procurement. In some embodiments, the biological and structural integrity of the fetal support tissue product is substantially preserved for at least 90 days after initial procurement. In some embodiments, the biological and structural integrity of the fetal support tissue product is substantially preserved for at least 180 days after initial procurement. In some embodiments, the biological and structural integrity of the fetal support tissue product is substantially preserved for at least 1 year after initial procurement. In some embodiments, the biological and structural integrity of the fetal support tissue product is substantially preserved for at least 2 years after initial procurement. In some embodiments, the biological and structural integrity of the fetal support tissue product is substantially preserved for at least 3 years after initial procurement. In some embodiments, the biological and structural integrity of the fetal support tissue product is substantially preserved for at least 4 years after initial procurement. In some embodiments, the biological and structural integrity of the fetal support tissue product is substantially preserved for at least 5 years after initial procurement. In some embodiments, the fetal support tissue is obtained from a human, a non-human primate, a cow or a pig.

In some embodiments, the fetal support tissue product is kept below 0°C until donor and specimen eligibility has been determined. In some embodiments, the fetal support tissue product is kept from between 0°C to -80°C until donor and specimen eligibility has been determined. In some embodiments, storing the fetal support tissue product at -80°C kills substantially all cells found in the fetal support tissue. In some embodiments, storing the fetal support tissue product at -80°C kills substantially all cells found in the fetal support tissue product while maintaining or increasing the biological activity of the fetal support tissue product (e.g., its anti-inflammatory, anti-scarring, anti-antigenic, and anti-adhesion properties) relative to fresh (i.e., non-frozen) fetal support tissue. In some embodiments, storing the fetal support tissue product at -80°C results in the loss of metabolic activity in substantially all cells found in the fetal support tissue. In some embodiments, the fetal support tissue is dried. In some embodiments, the fetal support tissue is not dehydrated.

***Processing* of *fetal support tissue*** (fetal support tissue is not specifically claimed but the processes described below may be used to obtain the fetal support tissue product as claimed)

In some embodiments, processing is done following Good Tissue Practices (GTP) to ensure that no contaminants are introduced into the fetal support tissue product.

In some embodiments, the fetal support tissue is tested for HIV-1, HIV-2, HTLV-1, hepatitis B and C, West Nile virus, cytomegalovirus, human transmissible spongiform encephalopathy (e.g., Creutzfeldt-Jakob disease) and *Treponema pallidum* using FDA licensed screening test. In some embodiments, any indication that the tissue is contaminated with HIV-1, HIV-2, HTLV-1, hepatitis B and C, West Nile virus, or cytomegalovirus results in the immediate quarantine and subsequent destruction of the tissue specimen. In some embodiments, the donor's medical records are examined for risk factors for and clinical evidence of hepatitis B, hepatitis C, or HIV infection.

In some embodiments, the fetal support tissue is frozen. In some embodiments, the fetal support tissue is not frozen. If the fetal support tissue is not frozen, it is processed as described below immediately.

In some embodiments, substantially all of the blood is removed from the fetal support tissue (e.g., from any arteries and veins found in the fetal support tissue, and blood that has infiltrated into the tissue). In some embodiments, substantially all of the blood is removed before the fetal support tissue is frozen. In some embodiments, blood is not removed from the fetal support tissue. In some embodiments, blood is not removed from the fetal support tissue before the fetal support tissue is frozen. In some embodiments, the blood is substantially removed after the fetal support tissue has been frozen.

In some embodiments, the fetal support tissue is washed with buffer with agitation to remove excess blood and tissue. In some embodiments, the fetal support tissue is soaked with buffer with agitation to remove excess blood and tissue.

In some embodiments, the fetal support tissue product is a fetal support tissue graft. In some embodiments, isolated fetal support tissue is used to generate a fetal support tissue graft. In some embodiments, the fetal support tissue is cut into multiple sections (e.g., using a scalpel). The size of the sections depends on the desired use of the fetal support tissue graft derived from the fetal support tissue. In some embodiments, the cut fetal support tissue is optionally washed again with buffer to further remove excess blood and tissue.

The umbilical cord comprises two arteries (the umbilical arteries) and one vein (the umbilical vein). In some embodiments, the vein and arteries are removed from the UC. In some embodiments, the vein and the arteries are not removed from the UC. In certain instances, the vein and arteries are surrounded (or suspended or buried) within the Wharton's Jelly. In some embodiments, the vein and arteries are removed concurrently with the removal of the Wharton's Jelly.

The desired thickness of the fetal support tissue product determines how the fetal support tissue product is processed. In some embodiments, the desired thickness of the fetal support tissue product determines how much of the Wharton's Jelly is removed. In some embodiments, the fetal support tissue product is contacted with a buffer to facilitate separation of the Wharton's Jelly and the UCAM. In some embodiments, the Wharton's jelly is removed using peeling, a rotoblator (i.e., a catheter attached to a drill with a diamond coated burr), a liposuction, a liquid under high pressure, a brush (e.g., a mechanized brush rotating under high speed), or a surgical dermatome. In some embodiments, Wharton's Jelly is not removed. In some embodiments, Wharton's Jelly and the umbilical vein and arteries are not removed. In some embodiments, Wharton's Jelly is not removed, and the umbilical vein and arteries are removed.

In some embodiments, the fetal support tissue product comprises isolated umbilical cord amniotic membrane (UCAM). In certain instances, the UCAM comprises proteins, glycans, protein-glycan complexes (e.g., a complex of hyaluronic acid and a heavy chain of IαI and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UC and into the surrounding tissue. In some embodiments, the UCAM is isolated by removing all of the Wharton's Jelly and umbilical vessels from the UC, leaving the UCAM. After substantially pure UCAM has been obtained, the UCAM is optionally washed with buffer to remove excess blood and tissue. In some embodiments, the UCAM comprises Wharton's Jelly. In some embodiments, the UCAM comprises Wharton's Jelly and the umbilical vein and arteries. In some embodiments, the UCAM comprises Wharton's Jelly and not the umbilical vein and arteries.

In some embodiments, the fetal support tissue product is in any suitable shape (e.g., a square, a circle, a triangle, a rectangle). In some embodiments, the fetal support tissue product is generated from a sheet of fetal support tissue. In some embodiments, the sheet is flat. In some embodiments, the sheet is tubular.

In some embodiments, the fetal support tissue product is cut into multiple sections (e.g., using a scalpel). In some embodiments, the fetal support tissue product is divided into sections that are about 1.0 cm x about 0.25 cm, 0.5 cm, 0.75 cm, 1.0 cm, 2.0 cm, 3.0 cm, 4.0 cm, 5.0 cm, or 6 cm. In some embodiments, the fetal support tissue product is divided into sections that are about 2 cm x about 2 cm, 3 cm, 4 cm, 5 cm, or 6 cm. In some embodiments, the fetal support tissue product is divided into sections that are about 3 cm x about 3 cm, 4 cm, 5 cm, or 6 cm. In some embodiments, the fetal support tissue product is divided into sections that are about 4 cm x about 4 cm, 5 cm, or 6 cm. In some embodiments, the fetal support tissue product is divided into sections that are about 5 cm x about 5 cm or 6 cm. In some embodiments, the fetal support tissue product is divided into sections that are about 6 cm x about 6 cm. In some embodiments, the fetal support tissue product is divided into sections that are about 8 cm x about 1 cm, 2 cm, 3 cm, 4 cm, 5 cm, 6 cm, 7 cm, or 8 cm. In some embodiments, the fetal support tissue product is divided into sections that are about 10 cm x about 10 cm. In some embodiments, the fetal support tissue product is divided into sections that are about 12 cm x about 10 cm. In some embodiments, the fetal support tissue product is divided into sections that are about 15 cm x about 10 cm. In some embodiments, the fetal support tissue product is divided into sections that are about 20 cm x about 10 cm. In some embodiments, the fetal support tissue product is divided into sections that are about 25 cm x about 10 cm. In some embodiments, the fetal support tissue product is divided into sections that are about 30 cm x about 10 cm.

In some embodiments, the fetal support tissue product is contacted with buffer under agitation to remove substantially all remaining red blood cells. In some embodiments, the fetal support tissue product is contacted with a buffer for 10 minutes, 15 minutes, 20 minutes, 25 minutes, 30 minutes, 40 minutes, 1 hour, 2 hours, 3 hours, 4 hours, 5 hours, 6 hours, 12 hours, 18 hours, 24 hours, or more than 24 hours. In some embodiments, the UC product is contacted with a buffer for 2 days, 3 days, 4 days, 5 days, 6 days, 7 days, 1 week, 2 weeks, 3 weeks, 4 weeks or more than 4 weeks.

In some embodiments, the fetal support tissue product comprises a pharmaceutically acceptable excipient, carrier, or combination thereof. In some embodiments, the fetal support tissue product is formulated as a non-solid dosage form. In some embodiments, the fetal support tissue product is formulated as a solid dosage form.

### Processing a fetal support tissue product

In some embodiments, isolated fetal support tissue is used to generate a morselized fetal support tissue product. As used herein, "morsel" refers to particles of tissue ranging in size from about 0.1 mm to about 1.0 cm in length, width, or thickness that have been obtained from a larger piece of tissue. A "morsel" as described herein, retains the characteristics of the tissue from which it was obtained and upon inspection is identifiable as said tissue. As used herein, the terms "morselized," "morselizing," and "morselization" refer to actions involving the "morsels" of the present application. In some embodiments, the morselized fetal support tissue product is further processed into a solution, suspension or emulsion by mixing the morselized fetal support tissue with a carrier. In some embodiments, the morselized fetal support tissue product is formulated into a solution, suspension, paste, ointment, oil emulsion, cream, lotion, gel, a patch, sticks, film, paint, or a combination thereof. In some embodiments, the morselized fetal support tissue product is contacted with a patch or wound dressing. In some embodiments, the morselized fetal support tissue product is formulated for parenteral injection, is administered as a sterile solution, suspension, or emulsion, or is formulated for inhalation.

In some embodiments, a mixture of amniotic membrane tissue and umbilical cord tissue in any ratio from 0.001:99.999 w/w % to 99.999:0.001 w/w % is morselized from either fresh or frozen tissue through the use of any morselizing tool known to one of skill in the art such as, for example, tissue grinder, sonicator, bread beater, freezer/mill, blender, mortar/pestle, Roto-stator, kitchen chopper, grater, ruler and scalpel to yield morsels ranging in size from about 0.1 mm to about 1.0 cm in length, width, or thickness. In some embodiments, the resulting morsels are homogenized to yield consistently sized morsels. In some embodiments, the resulting morsels are used wet, partially dehydrated or essentially dehydrated by any means known to one of skill in the art such as, for example, centrifuge or lyophilization. In some embodiments, the resulting fetal support tissue product is used immediately or stored for later use in any type of contained known to one of skill in the art such as, for example, pouch, jar, bottle, tube, ampule and prefilled syringe. In some embodiments, the morselized fetal support tissue product is sterilized by any method known to one of skill in the art such as, for example, γ radiation.

In some embodiments, isolated fetal support tissue is used to generate a pulverized fetal support tissue product. As used herein, "pulverized fetal support tissue product" means a fetal support tissue product comprising tissue that has been broken up (or, disassociated). In some embodiments, the pulverized fetal support tissue product is a dry powder. In some embodiments, the pulverized fetal support tissue product is further processed into a solution, suspension or emulsion by mixing the fetal support tissue powder with a carrier. In some embodiments, the pulverized fetal support tissue product is formulated into a solution, suspension, paste, ointment, oil emulsion, cream, lotion, gel, a patch, sticks, film, paint, or a combination thereof. In some embodiments, the pulverized fetal support tissue product is contacted with a patch or wound dressing. In some embodiments, the pulverized fetal support tissue product is formulated for parenteral injection, is administered as a sterile solution, suspension, or emulsion, or is formulated for inhalation.

In some embodiments, the isolated fetal support tissue is pulverized by any suitable method. In some embodiments, the isolated fetal support tissue is pulverized by use of a pulverizer (e.g., a Bessman Tissue Pulverizer, a Biospec biopulverizer, or a Covaris CryoPrep). In some embodiments, the isolated fetal support tissue is pulverized by use of a tissue grinder (e.g., a Potter-Elvehjem grinder or a Wheaton Overhead Stirrer). In some embodiments, the isolated fetal support tissue is pulverized by use of a sonicator. In some embodiments, the isolated fetal support tissue is pulverized by use of a bead beater. In some embodiments, the isolated fetal support tissue is pulverized by use of a freezer/mill (e.g., a SPEX^{®} SamplePrep Freezer/Mill or a Retsch Ball Mill). In some embodiments, the isolated fetal support tissue is pulverized by use of a pestle and mortar. In some embodiments, the isolated fetal support tissue is pulverized by manual use of a pestle and mortar.

In some embodiments, the fetal support tissue product is an extract from a fetal support tissue. In some embodiments, the fetal support tissue product is an HC-HA/PTX3 complex. In some embodiments, the HC-HA/PTX3 complex is an nHC-HA/PTX3, an rcHC-HA/PTX3, or the combination thereof. In some embodiments, the HC-HA/PTX3 complex is purified by any suitable method.

In some embodiments, the HC-HA/PTX3 complex is purified by centrifugation (e.g., ultracentrifugation, gradient centrifugation), chromatography (e.g., ion exchange, affinity, size exclusion, and hydroxyapatite chromatography), gel filtration, or differential solubility, ethanol precipitation or by any other available technique for the purification of proteins (See, e.g., Scopes, Protein Purification Principles and Practice 2nd Edition, Springer-Verlag, New York, 1987; Higgins, S. J. and Hames, B. D. (eds.), Protein Expression: A Practical Approach, Oxford Univ Press, 1999; and Deutscher, M. P., Simon, M. I., Abelson, J. N. (eds.), Guide to Protein Purification: Methods in Enzymology (Methods in Enzymology Series, Vol 182), Academic Press, 1997, ).

In some embodiments, an nHC-HA/PTX3 is isolated from an extract. In some embodiments, the extract is prepared from an amniotic membrane extract. In some embodiments, the extract is prepared from an umbilical cord extract. In some embodiments, the umbilical cord extract comprises umbilical cord stroma and/or Wharton's jelly. In some embodiments, the nHC-HA/PTX3 complex is contained in an extract that is prepared by ultracentrifugation. In some embodiments, the nHC-HA/PTX3 complex is contained in an extract that is prepared by ultracentrifugation using a CsCl,4-6M guanidine HCl gradient. In some embodiments, the extract is prepared by at least 2 rounds of ultracentrifugation. In some embodiments, the extract is prepared by more than 2 rounds of ultracentrifugation (i.e. nHC-HA/PTX3 2^{nd}). In some embodiments, the extract is prepared by at least 4 rounds of ultracentrifugation (i.e. nHC-HA/PTX3 4^{th}). In some embodiments, the nHC-HA/PTX3 complex comprises a small leucine-rich proteoglycan. In some embodiments, the nHC-HA/PTX3 complex comprises HC1, HA, PTX3 and/or a small leucine-rich proteoglycan.

### Cryopreservation

In some embodiments, the fetal support tissue product is frozen for cryopreservation. In some embodiments, cryopreserving the fetal support tissue product does not destroy the integrity of the fetal support tissue extracellular matrix. In some embodiments, the fetal support tissue product is exposed to a liquid gas (e.g., liquid nitrogen or liquid hydrogen). In some embodiments, the fetal support tissue product is exposed to liquid nitrogen. In some embodiments, the fetal support tissue product does not contact the liquid gas. In some embodiments, the fetal support tissue product is placed in a container and the container is contacted with liquid gas. In some embodiments, the fetal support tissue product is exposed to the liquid gas until the fetal support tissue product is frozen.

### Lyophilization

In some embodiments, the fetal support tissue product is lyophilized. In some embodiments, the fetal support tissue product is lyophilized before being morselized, pulverized, cryopreserved, sterilized, or purified. In some embodiments, the fetal support tissue product is lyophilized after being morselized, pulverized, cryopreserved, sterilized, or purified. In some embodiments, the fetal support tissue product is lyophilized following freezing. In some embodiments, the fetal support tissue product is lyophilized following freezing by any suitable method (e.g., exposure to a liquid gas, placement in a freezer). In some embodiments, the fetal support tissue product is frozen by exposure to a temperature below about 0°C. In some embodiments, the fetal support tissue product is frozen by exposure to a temperature below about -20°C. In some embodiments, the fetal support tissue product is frozen by exposure to a temperature below about -40°C. In some embodiments, the fetal support tissue product is frozen by exposure to a temperature below about -50°C. In some embodiments, the fetal support tissue product is frozen by exposure to a temperature below about -60°C. In some embodiments, the fetal support tissue product is frozen by exposure to a temperature below about -70°C. In some embodiments, the fetal support tissue product is frozen by exposure to a temperature below about -75°C. In some embodiments, the fetal support tissue product is frozen by exposure to a temperature below about -80°C. In some embodiments, the fetal support tissue product is frozen by exposure to a temperature below about -90°C. In some embodiments, the fetal support tissue product is frozen by exposure to a temperature below about -100°C. In some embodiments, the fetal support tissue product is frozen by exposure to a liquid gas. In some embodiments, the fetal support tissue product is placed in a vacuum chamber of a lyophilization device until all or substantially all fluid (e.g., water) has been removed. In some embodiments, a cryopreserved fetal support tissue product is lyophilized.

### Sterilization

In some embodiments, the fetal support tissue product is subject to terminal sterilization by any suitable (e.g., medically acceptable) method. In some embodiments, the fetal support tissue product is a lyophilized fetal support tissue product. In some embodiments, the fetal support tissue product is exposed to gamma radiation for a period of time sufficient to sterilize the fetal support tissue product. In some embodiments, the fetal support tissue product is exposed to gamma radiation at 25 kGy for a period of time sufficient to sterilize the fetal support tissue product. In some embodiments, the fetal support tissue product is exposed to an electron beam for a period of time sufficient to sterilize the fetal support tissue product. In some embodiments, the fetal support tissue product is exposed to X-ray radiation for a period of time sufficient to sterilize the fetal support tissue product. In some embodiments, the fetal support tissue product is exposed to UV radiation for a period of time sufficient to sterilize the fetal support tissue product.

### Rehydration

In some embodiments, the fetal support tissue product is partially or fully rehydrated. In some embodiments, the fetal support tissue product is rehydrated by contacting the fetal support tissue product with a buffer or with water. In some embodiments, the fetal support tissue product is contacted with an isotonic buffer. In some embodiments, the fetal support tissue is contacted with saline. In some embodiments, the fetal support tissue product is contacted with PBS. In some embodiments, the fetal support tissue product is contacted with Ringer's solution. In some embodiments, the Ringer's solution is Lactate Ringer's Saline. In some embodiments, the fetal support tissue product is contacted with Hartmann's solution. In some embodiments, the fetal support tissue product is contacted with a TRIS-buffered saline. In some embodiments, the fetal support tissue product is contacted with a HEPES-buffered saline; 50% DMEM + 50% Glycerol; 10%, 20%, 30%, 40%, 50%, 60%, 70%, 80%, 90% or 100% glycerol; and/or 10%, 20%, 30%, 40%, 50%, 60%, 70%, 80%, 90% or 100% propylene glycol.

In some embodiments, the fetal support tissue product is contacted with a buffer for 10 minutes, 15 minutes, 20 minutes, 25 minutes, 30 minutes, 40 minutes, 1 hour, 2 hours, 3 hours, 4 hours, 5 hours, 6 hours, 12 hours, 18 hours, 24 hours, or more than 24 hours. In some embodiments, the UC product is contacted with a buffer for 2 days, 3 days, 4 days, 5 days, 6 days, 7 days, 1 week, 2 weeks, 3 weeks, 4 weeks or more than 4 weeks.

In some embodiments, the fetal support tissue product is stored for later use. In some embodiments, storing the fetal support tissue product does not destroy the integrity of the fetal support tissue extracellular matrix. In some embodiments, the fetal support tissue product is lyophilized. In some embodiments, the fetal support tissue product is stored in any suitable storage medium.

In some embodiments, the fetal support tissue product is optionally contacted with a substrate (i.e., a supportive backing). In some embodiments, the fetal support tissue product is not contacted with a substrate. In some embodiments, the fetal support tissue product is orientated such that the fetal support tissue product is in contact with the substrate. In some embodiments, the fetal support tissue product is orientated such that the stroma is in contact with the substrate. In some embodiments the fetal support tissue product is orientated such that the epithelial side is in contact with the substrate.

In some embodiments, the fetal support tissue product is attached to the substrate. In some embodiments, the substrate is nitrocellulose paper (NC). In some embodiments, the substrate is nylon membrane (NM). In some embodiments, the substrate is polyethersulfone membrane (PES).

### HC-HA/PTX3 Compositions

In some embodiments, the fetal support tissue product is a native HC-HA/PTX3 (nHC-HA/PTX3) complex, a reconstituted HC-HA/PTX3 (rcHC-HA/PTX3) complex, or a combination thereof. In some embodiments, the fetal support tissue product comprises HC-HA/PTX3 and pharmaceutical excipient. In some embodiments, the fetal support tissue product consists essentially of an nHC-HA/PTX3 complex or a rcHC-HA/PTX3 complex. In some embodiments, the fetal support tissue product comprises a pharmaceutically acceptable diluent, excipient, vehicle, or carrier. In some embodiments, proper formulation is dependent upon the route of administration selected.

In some embodiments, the nHC-HA/PTX3 is isolated from an extract. In some embodiments, the extract is prepared from an amniotic membrane extract. In some embodiments, the extract is prepared from an umbilical cord extract. In some embodiments, the umbilical cord extract comprises umbilical cord stroma and/or Wharton's jelly. In some embodiments, the nHC-HA/PTX3 complex is contained in an extract that is prepared by ultracentrifugation. In some embodiments, the nHC-HA/PTX3 complex comprises a small leucine-rich proteoglycan. In some examples not claimed the nHC-HA/PTX3 complex comprises HC1, HA, PTX3 and/or a small leucine-rich proteoglycan (SLRP).

In some embodiments, ultracentrifugation is performed on a tissue extract. In some embodiments, ultracentrifugation is used to purify a nHC-HA/PTX3 soluble complex. In some embodiments, the nHC-HA soluble complex comprises a small leucine-rich proteoglycan. In examples not claimed the nHC-HA/PTX3 soluble complex comprises HC1, HA, PTX3 and/or a small leucine-rich proteoglycan.

In some embodiments, the nHC-HA/PTX3 complex is purified by immunoaffinity chromatography, affinity chromatography, or a combination thereof. In some embodiments, anti HC1 antibodies, anti-HC2 antibodies, or both are generated and affixed to a stationary support. In some embodiments, the HC-HA complex binds to the antibodies (e.g., via interaction of (a) an anti-HC1 antibody and HC1, (b) an anti-HC2 antibody and HC2, (c) an anti-PTX antibody and PTX3, (d) an anti-SLRP antibody and the SLRP, or (e) any combination thereof). In some embodiments, HABP is generated and affixed to a stationary support.

In some embodiments, the nHC-HA/PTX3 complex is purified from the insoluble fraction as described herein using one or more antibodies. In some embodiments, the nHC-HA/PTX3 complex is purified from the insoluble fraction as described herein using anti-SLRP antibodies.

In some embodiments, the nHC-HA/PTX3 complex is purified from the soluble fraction as described herein. In some embodiments, the nHC-HA/PTX3 complex is purified from the soluble fraction as described herein using anti-PTX3 antibodies.

In some embodiments, the nHC-HA/PTX3 complex comprises a small leucine rich proteoglycan (SLRP). In some embodiments, the nHC-HA/PTX3 complex comprises a class I, class II or class II SLRP. In some embodiments, the small leucine-rich proteoglycan is selected from among class I SLRPs, such as decorin and biglycan. In some embodiments, the small leucine-rich proteoglycan is selected from among class II SLRPs, such as fibromodulin, lumican, PRELP (proline arginine rich end leucine-rich protein), keratocan, and osteoadherin. In some embodiments, the small leucine-rich proteoglycan is selected from among class III SLRPs, such as epipycan and osteoglycin. In some embodiments, the small leucine-rich proteoglycan is selected from among bikunin, decorin, biglycan, and osteoadherin. In some embodiments, the small leucine-rich protein comprises a glycosaminoglycan. In some embodiments, the small leucine-rich proteoglycan comprises keratan sulfate.

In examples not claimed , a method for generating reconstituted HC-HA/PTX3 complexes comprises (a) contacting high molecular weight hyaluronan (HMW HA) with IαI and TSG-6 to HA to form an HC-HA complex pre-bound to TSG-6 and (b) contacting the HC-HA complex with pentraxin 3 (PTX3) under suitable conditions to form an rcHC-HA/PTX3 complex. Provided herein are rcHC-HA/PTX3 complexes produced by such method. In some examples not claimed HC1ofIαI forms a covalent linkage with HA In examples not claimed, the steps (a) and (b) of the method are performed sequentially in order. In examples not claimed, the method comprises contacting an HC-HA complex pre-bound to TSG-6 with PTX3. In examples not claimed, the purified, rcHC-HA/PTX3 complex is produced in vitro by a method comprising (a) contacting high molecular weight hyaluronan (HMW HA) with (i) pentraxin 3 (PTX3) protein, (ii) inter-α-inhibitor (IaI) protein comprising heavy chain 1 (HC1) and heavy chain 2 (HC2) and (iii) tumor necrosis factor α-stimulated gene 6 (TSG-6) to form an rcHC-HA/PTX3 complex comprising HMW HA, HC1, HC2, and PTX3; and (b) purifying the rcHC-HA/PTX3 complex from unwanted components. In examples not claimed, the purified nHC-HA/PTX3 does not comprise an inter-α-inhibitor (IaI) protein heavy chain 2 (HC2). In some examples not claimed the purified rcHC-HA/PTX3 comprises an inter-α-inhibitor (IaI) protein comprising heavy chain 2 (HC₂). In examples not claimed, the rcHC-HA/PTX3 comprises HA, HC1, HC2, and PTX3. In examples not claimed , the rcHC-HA/PTX3 comprises HA, HC1, HC2, PTX3, and TSG-6.

In examples not claimed , the method comprises first contacting high molecular weight hyaluronan (HMW HA) with pentraxin 3 (PTX3) under suitable conditions to form a PTX3/HA complex, then contacting the PTX3/HA complex with IαI and TSG-6.

In examples not claimed , the IαI protein and TSG-6 protein are contacted to the complex at a molar ratio of about 1:1, 2:1, 3:1, 4:1, 5:1, 6:1, 7:1, 8:1, 9:1, 10:1, 15:1, or 20:1 (IαI:TSG-6). In examples not claimed the ratio of IαI:TSG-6 ranges from about 1:1 to about 20:1, such as about 1:1 to about 10:1, such as about 1:1 to 5 about: 1, such as about 1:1 to about 3:1. In examples not claimed, the ratio of IαI:TSG-6 is 3:1 or higher. In examples not claimed, the ratio of IαI:TSG-6 is 3:1.

In examples not claimed , the steps (a) and (b) of the method are performed sequentially in order. In examples not claimed , the method comprises contacting a PTX3/HA complex with IαI and TSG-6.

In some embodiments, the pharmaceutical composition further comprises at least one pharmaceutically acceptable carrier. In some embodiments, the pharmaceutical composition further comprises an adjuvant, excipient, preservative, agent for delaying absorption, filler, binder, adsorbent, buffer, and/or solubilizing agent. Exemplary pharmaceutical compositions that are formulated to comprise an HC-HA/PTX3 complex provided herein include,
a gel, solution, suspension, emulsion, syrup, granule, powder, homogenate, ointment, tablet, capsule, pill, paste, cream, lotion, a patch, sticks, film, paint, an aerosol, or a combination thereof. In some embodiments, the fetal support tissue product comprising HC-HA/PTX3 is a graft or a sheet.

### Dosage Forms

Provided below are dosage forms of fetal support tissue product. I

The fetal support tissue product comprises an HC-HA/PTX3 complex. In some embodiments, the HC-HA/PTX3 complex is native complex purified from a fetal support tissue, or a reconstituted HC-HA/PTX3 complex or a combination thereof.

In some embodiments, a fetal support tissue product is administered as an aqueous suspension. In some embodiments, an aqueous suspension comprises water, Ringer's solution and/or isotonic sodium chloride solution. In some embodiments, the Ringer's solution is Lactate Ringer's Saline. In some embodiments, an aqueous suspension comprises a sweetening or flavoring agent, coloring matters or dyes and, if desired, emulsifying agents or suspending agents, together with diluents water, ethanol, propylene glycol, glycerin, or combinations thereof. In some embodiments, an aqueous suspension comprises a suspending agent. In some embodiments, an aqueous suspension comprises sodium carboxymethylcellulose, methylcellulose, hydroxypropylmethyl-cellulose, sodium alginate, polyvinyl-pyrrolidone, gum tragacanth and/or gum acacia. In some embodiments, an aqueous suspension comprises a dispersing or wetting agent. In some embodiments, an aqueous suspension comprises a naturallyoccurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. In some embodiments, an aqueous suspension comprises a preservative. In some embodiments, an aqueous suspension comprises ethyl, or n-propyl p-hydroxybenzoate. In some embodiments, an aqueous suspension comprises a sweetening agent. In some embodiments, an aqueous suspension comprises sucrose, saccharin or aspartame.

In some embodiments, a fetal support tissue product is administered as an oily suspension. In some embodiments, an oily suspension is formulated by suspending the active ingredient in a vegetable oil (e.g., arachis oil, olive oil, sesame oil or coconut oil), or in mineral oil (e.g., liquid paraffin). In some embodiments, an oily suspension comprises a thickening agent (e.g., beeswax, hard paraffin or cetyl alcohol). In some embodiments, an oily suspension comprises sweetening agents (e.g., those set forth above). In some embodiments, an oily suspension comprises an anti-oxidant (e.g., butylated hydroxyanisol or alpha-tocopherol).

In some embodiments, a fetal support tissue product is formulated for parenteral injection (e.g., via injection or infusion, including intraarterial, intracardiac, intradermal, intraduodenal, intramedullary, intramuscular, intraosseous, intraperitoneal, intrathecal, intravascular, intravenous, intravitreal, epidural, and/or subcutaneous). In some embodiments, the fetal support tissue product is administered as a sterile solution, suspension or emulsion. In some embodiments, the fetal support tissue product is formulated for inhalation.

In some embodiments, a formulation for injection is presented in unit dosage form, e.g., in ampoules or in multi-dose containers, with an added preservative.

In some embodiments, a fetal support tissue product comprising an HC-HA/PTX3 complex is formulated for topical administration. Topical formulations include,
ointments, creams, lotions, solutions, pastes, gels, films, sticks, liposomes, nanoparticles. In some embodiments, a topical formulation is administered by use of a patch, bandage or wound dressing.

In some embodiments, a fetal support tissue product comprising an HC-HA/PTX3 complex is formulated as composition is in the form of a solid, a cross-linked gel, or a liposome. In some embodiments, the fetal tissue support product comprising an HC-HA/PTX3 complex is formulated as an insoluble cross-linked hydrogel. In some embodiments, the fetal support tissue product is formulated as a gel.

In some embodiments, a topical formulation comprises a gelling (or thickening) agent. Suitable gelling agents include, celluloses, cellulose derivatives, cellulose ethers (e.g., carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (e.g., alginic acid), silicates, starch, tragacanth, carboxyvinyl polymers, carrageenan, paraffin, petrolatum, acacia (gum arabic), agar, aluminum magnesium silicate, sodium alginate, sodium stearate, bladderwrack, bentonite, carbomer, carrageenan, carbopol, xanthan, cellulose, microcrystalline cellulose (MCC), ceratonia, chondrus, dextrose, furcellaran, gelatin, ghatti gum, guar gum, hectorite, lactose, sucrose, maltodextrin, mannitol, sorbitol, honey, maize starch, wheat starch, rice starch, potato starch, gelatin, sterculia gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), or combinations thereof.

In some embodiments, a topical formulation disclosed herein comprises an emollient. Emollients include, castor oil esters, cocoa butter esters, safflower oil esters, cottonseed oil esters, corn oil esters, olive oil esters, cod liver oil esters, almond oil esters, avocado oil esters, palm oil esters, sesame oil esters, squalene esters, kikui oil esters, soybean oil esters, acetylated monoglycerides, ethoxylated glyceryl monostearate, hexyl laurate, isohexyl laurate, isohexyl palmitate, isopropyl palmitate, methyl palmitate, decyloleate, isodecyl oleate, hexadecyl stearate decyl stearate, isopropyl isostearate, methyl isostearate, diisopropyl adipate, diisohexyl adipate, dihexyldecyl adipate, diisopropyl sebacate, lauryl lactate, myristyl lactate, and cetyl lactate, oleyl myristate, oleyl stearate, and oleyl oleate, pelargonic acid, lauric acid, myristic acid, palmitic acid, stearic acid, isostearic acid, hydroxystearic acid, oleic acid, linoleic acid, ricinoleic acid, arachidic acid, behenic acid, erucic acid, lauryl alcohol, myristyl alcohol, cetyl alcohol, hexadecyl alcohol, stearyl alcohol, isostearyl alcohol, hydroxystearyl alcohol, oleyl alcohol, ricinoleyl alcohol, behenyl alcohol, erucyl alcohol, 2-octyl dodecanyl alcohol, lanolin and lanolin derivatives, beeswax, spermaceti, myristyl myristate, stearyl stearate, carnauba wax, candelilla wax, lecithin, and cholesterol.

In some embodiments, a fetal tissue support product comprising an HC-HA/PTX3 complex is formulated with one or more natural polymers. In some embodiments, a fetal tissue support product n comprising an HC-HA/PTX3 complex is formulated with a natural polymer that is fibronectin, collagen, laminin, keratin, fibrin, fibrinogen, hyaluronic acid, heparan sulfate, chondroitin sulfate. In some embodiments, a fetal tissue support product comprising an HC-HA/PTX3 complex is formulated with a polymer gel formulated from a natural polymer. In some embodiments, a fetal tissue support product comprising an HC-HA/PTX3 complex is formulated with a polymer gel formulated from a natural polymer, such as, , fibronectin, collagen, laminin, keratin, fibrin, fibrinogen, hyaluronic acid, heparan sulfate, chondroitin sulfate, and combinations thereof.

In some embodiments, a fetal tissue support product comprising an HC-HA/PTX3 complex is formulated for administration to an eye or a tissue related thereto. Formulations suitable for administration to an eye include, solutions, suspensions (e.g., an aqueous suspension), ointments, gels, creams, liposomes, niosomes, pharmacosomes, nanoparticles, or combinations thereof. In some embodiments, a fetal tissue support product comprising an HC-HA/PTX3 complex for topical administration to an eye is administered spraying, washing, or combinations thereof. In some embodiments, a fetal tissue support product comprising an HC-HA/PTX3 complex is administered to an eye via an injectable depot preparation.

As used herein, a "depot preparation" is a controlled-release formulation that is implanted in an eye or a tissue related thereto (e.g., the sclera) (for example subcutaneously, intramuscularly, intravitreally, or within the subconjunctiva). A depot preparation is formulated by forming microencapsulated matrices (also known as microencapsulated matrices) of a fetal tissue support product comprising an HC-HA/PTX3 complex in biodegradable polymers. In some embodiments, a depot preparation is formulated by entrapping a fetal tissue support product comprising an HC-HA/PTX3 complex in liposomes or microemulsions.

A formulation for administration to an eye has an ophthalmologically acceptable tonicity. In certain instances, lacrimal fluid has an isotonicity value equivalent to that of a 0.9% sodium chloride solution. In some embodiments, an isotonicity value from about 0.6% to about1.8% sodium chloride equivalency is suitable for topical administration to an eye. In some embodiments, a formulation for administration to an eye disclosed herein has an osmolarity from about 200 to about 600 mOsm/L. In some embodiments, a formulation for administration to an eye disclosed herein is hypotonic and thus requires the addition of any suitable to attain the proper tonicity range. Ophthalmically acceptable substances that modulate tonicity include,
sodium chloride, potassium chloride, sodium thiosulfate, sodium bisulfite and ammonium sulfate.

A formulation for administration to an eye has an ophthalmologically acceptable clarity. Examples of ophthalmologically-acceptable clarifying agents include,
polysorbate 20, polysorbate 80, or combinations thereof.

In some embodiments, a formulation for administration to an eye comprises an ophthalmologically acceptable viscosity enhancer. In some embodiments, a viscosity enhancer increases the time a formulation disclosed herein remains in an eye. In some embodiments, increasing the time a formulation disclosed herein remains in the eye allows for greater drug absorption and effect. Non-limiting examples of mucoadhesive polymers include carboxymethylcellulose, carbomer (acrylic acid polymer), poly(methylmethacrylate), polyacrylamide, polycarbophil, acrylic acid/butyl acrylate copolymer, sodium alginate and dextran.

In some embodiments, a formulation for administration to an eye is administered or delivered to the posterior segments of an eye (e.g., to the retina, choroid, vitreous and optic nerve). In some embodiments, a topical formulation for administration to an eye disclosed herein for delivery to the posterior of the eye comprises a solubilizing agent, for example, a glucan sulfate and/or a cyclodextrin. Glucan sulfates which are used in some embodiments include, ¹
dextran sulfate, cyclodextrin sulfate and β-1,3-glucan sulfate, both natural and derivatives thereof, or any compound which temporarily binds to and be retained at tissues which contain fibroblast growth factor (FGF), which improves the stability and/or solubility of a drug, and/or which improves penetration and ophthalmic absorption of a topical formulation for administration to an eye disclosed herein. Cyclodextrin derivatives which are used in some embodiments as a solubilizing agent include, α-cyclodextrin, β-cyclodextrin, γ-cyclodextrin, hydroxyethyl β -cyclodextrin, hydroxypropyl γ -cyclodextrin, hydroxypropyl β-cyclodextrin, sulfated α -cyclodextrin, sulfated β -cyclodextrin, sulfobutyl ether β -cyclodextrin.

### Dosages

The amount of pharmaceutical compositions administered is dependent in part on the individual being treated. In instances where pharmaceutical compositions are administered to a human subject, the daily dosage will normally be determined by the prescribing physician with the dosage generally varying according to the age, sex, diet, weight, general health and response of the individual, the severity of the individual's symptoms, the precise disease or condition being treated, the severity of the disease or condition being treated, time of administration, route of administration, the disposition of the composition, rate of excretion, drug combination, and the discretion of the prescribing physician.

In some embodiments, the dosage of the fetal support tissue product comprising an HC-HA/PTX3 complex is between about 0.001 to about 1000 mg/kg body weight/day. In some embodiments, the amount of the fetal support tissue product comprising an HC-HA/PTX3 complex is in the range of about 0.5 to about 50 mg/kg/day. In some embodiments, the amount of nHC-HA/PTX3 or rcHC-HA/PTX3 complex disclosed herein is about 0.001 to about 7 g/day. In some embodiments, the amount of the fetal support tissue product comprising an HC-HA/PTX3 complex is about 0.01 to about 7 g/day. In some embodiments, the amount of the fetal support tissue product comprising an HC-HA/PTX3 complex disclosed herein is about 0.02 to about 5 g/day. In some embodiments, the amount of the fetal support tissue product comprising an HC-HA/PTX3 complex is about 0.05 to about 2.5 g/day. In some embodiments, the amount of the fetal support tissue product comprising an HC-HA/PTX3 complex is about 0.1 to about 1 g/day.

In some embodiments, the fetal support tissue product comprising an HC-HA/PTX3 complex is administered, before, during or after the occurrence of unwanted changes in a tissue. In some embodiments, the fetal support tissue product comprising an HC-HA/PTX3 complex is administered with a combination therapy before, during or after the occurrence of a disease or condition. In some embodiments, the timing of administering the composition containing an nHC-HA/PTX3 or rcHC-HA/PTX3 disclosed herein varies. Thus, in some examples, the fetal support tissue product comprising an HC-HA/PTX3 complex is used as a prophylactic and is administered continuously to subjects with a propensity to develop unwanted changes in a tissue in order to prevent the occurrence of unwanted changes in the tissue. In some embodiments, the fetal support tissue product comprising an HC-HA/PTX3 complex is administered to a subject during or as soon as possible after the onset of the unwanted changes. In some embodiments, the administration of the fetal support tissue product comprising an HC-HA/PTX3 complex is initiated within the first 48 hours of the onset of the unwanted changes, preferably within the first 48 hours of the onset of the symptoms, more preferably within the first 6 hours of the onset of the symptoms, and most preferably within 3 hours of the onset of the symptoms. In some embodiments, the initial administration is via any route practical, such as, for example, an intravenous injection, a bolus injection, infusion over 5 minutes to about 5 hours, a pill, a capsule, transdermal patch, buccal delivery, or combination thereof. The fetal support tissue product comprising an HC-HA/PTX3 complex is preferably administered as soon as is practicable after the onset of unwanted changes is detected or suspected, and for a length of time necessary for the treatment, such as, for example, from about 1 month to about 3 months. In some embodiments, the length of treatment varies for each subject, and the length is determined using the known criteria. In some embodiments, the fetal support tissue product comprising an HC-HA/PTX3 complex or a formulation containing a complex is administered for at least 2 weeks, preferably about 1 month to about 5 years, and more preferably from about 1 month to about 3 years.

In some embodiments, the fetal support tissue product comprising an HC-HA/PTX3 complex is administered in a single dose, once daily. In some embodiments, the fetal support tissue product comprising an HC-HA/PTX3 complex is administered in multiple doses, more than once per day. In some embodiments, the fetal support tissue product comprising an HC-HA/PTX3 complex is administered twice daily. In some embodiments, the fetal support tissue product comprising an HC-HA/PTX3 complex is administered three times per day. In some embodiments, an nHC-HA/PTX3 or rcHC-HA/PTX3 complex is administered four times per day. In some embodiments, the fetal support tissue product comprising an HC-HA/PTX3 complex is administered more than four times per day.

In the case wherein the individual's condition does not improve, upon the doctor's discretion the fetal support tissue product comprising an HC-HA/PTX3 complex is administered chronically, that is, for an extended period of time, including throughout the duration of the individual's life in order to ameliorate or otherwise control or limit the symptoms of the individual's disease or condition.

In some embodiments, the fetal support tissue product comprising an HC-HA/PTX3 complex is packaged as articles of manufacture containing packaging material, a pharmaceutical composition which is effective for prophylaxis and/or treating a disease or condition, and a label that indicates that the pharmaceutical composition is to be used for reprogramming a fibroblastic cell in a tissue having unwanted changes due to a disease or condition. In some embodiments, the pharmaceutical compositions are packaged in unit dosage forms contain an amount of the pharmaceutical composition for a single dose or multiple doses. In some embodiments, the packaged compositions contain a lyophilized powder of the pharmaceutical compositions, which is reconstituted (e.g., with water or saline) prior to administration.

### Medical Device and Biomaterials Compositions

In some embodiments, the fetal support tissue product comprising an HC-HA/PTX3 complex is assembled directly on a surface of or formulated as a coating for an implantable medical device. In some embodiments, an nHC-HA/PTX3 or rcHC-HA/PTX3 complex is assembled directly on a surface of an implantable medical device or a portion thereof.

Exemplary implantable medical devices include, an artificial joint, orthopedic device, bone implant, contact lenses, suture, surgical staple, surgical clip, catheter, angioplasty balloon, sensor, surgical instrument, electrode, needle, syringe, wound drain, shunt, urethral insert, metal or plastic implant, heart valve, artificial organ, lap band, annuloplasty ring, guide wire, K-wire or Denham pin, stent, stent graft, vascular graft, pacemaker, pellets, wafers, medical tubing, infusion sleeve, implantable defibrillator, neurostimulator, glucose sensor, cerebrospinal fluid shunt, implantable drug pump, spinal cage, artificial disc, ocular implant, cochlear implant, breast implant, replacement device for nucleus pulposus, ear tube, intraocular lens, drug delivery system, microparticle, nanoparticle, and microcapsule.

In some embodiments, a fetal tissue support product comprising an HC-HA/PTX3 complex is assembled directly on a scaffold, a microparticle, a microcapsule or microcarrier employed for the delivery of a biomaterial, such as a stem cell or an insulin producing cell. In some embodiments, a fetal tissue support product comprising an HC-HA/PTX3 complex is attached to the microcapsule or assembled directly on a microcapsule.
The invention is defined in the claims. Any of the following examples that do not fall within the scope of the claims are provided for illustrative or comparative purposes only.

### EXAMPLES

### Example 1: A co-culture of Limbal Niche Cells (LNC) with Human Umbilical Vein Endothelial Cells (HUVEC) on HC-HA/PTX3 prevented apoptosis in HUVEC

Previously it was demonstrated that soluble HC-HA/PTX3 can suppress HUVEC viability independently by blocking CD44 Ab for 24 h, inhibit cell proliferation and reduce cell death in HUVEC. It has also been reported that collagenase-isolated clusters containing mesenchymal vimentin+ cells from cornea limbus heterogeneously express ESC and Nestin. Such LNCs can be further expanded on coated Matrigel^{™} (MG) more than 12 passages in MESCM. P4 LNCs maintain the phenotype expressing vascular pericyte markers (pericyte-EC) markers (e.g. FLK-1, CD34, CD31, α-SMA, PDGFRβ and NG2) with MSC tri-lineage differentiation. When HC-HA/PTX3 was added to limbal epithelial cells (LEPCs) in the presence of LNC, cell proliferation was reduced with quiescence markers of nuclear Bmi-1. It remains unclear whether the addition of LNCs, which possess pericyte phenotype may prevent HUVEC from cell death in the presence of HC-HA/PTX3.

### Materials and methods

**Cells:** GFP HUVEC (P3) were purchased from Neuromics (Cat#GF01). These cells were isolated from normal human umbilical vein and transfected with GFP-lentiviral particle at passage 1. Puromycin resistant GFP HUVEC were maintained on fibronectin coated solution in Endo-growth medium containing 5% FBS and growth supplement until passage 3. Cells were split 1:3 every three days when ~70%-90% confluence is reached. Cells of passage 3-8 were used for all experiments.

LNC (P2-P5) was expanded in MESCM containing 4 ng/ml bFGF and 10 ng/ml LIF on 6-well plastic coated with 5% Matrigel^{™}.

**Co-Culture:** Total 2x10⁴/per 96 well of GFP-HUVEC, LNC or GFP-HUVECs/LNCs (1:1)¹ were resuspended in EGM medium with 10 ng/ml of VEGF on coated fibronectin coating mix (Athena, 0407). The Endothelial Basal Medium-2 (EBM-2) contained 2% FBS, basic fibroblast growth factor (bFGF), EGF, insulin-like growth factor-1 (IGF-1), vascular endothelial growth factor (VEGF), hydrocortisone, ascorbic acid, heparin, gentamicin, and amphotericin-B (Lonza). 2-2.5ug/per 96 well (25ug/ml} of soluble or immobilized HC-HA/PTX3 was added during or prior the seeding and cultured at 37°C for 48 h. For vascular tube formation, cells were seeded at the density of 10⁵ cells per cm² on the surface of Matrigel^{™}, which was prepared by adding 50 µl of 100% Matrigel^{™} into 24 well plates for 30 minutes (min) before use, and cultured in EGM2 to elicit vascular tube-like network as reported. P4/3D cells or HUVEC alone were also seeded at the same density as the controls. Experiments were performed in triplicate.

**Cell Death Assay (GFP-certified Apoptosis/Necrosis Detection Kit):** A cell death assay kit was used for live cell imaging and to determine the suitable termination time. A positive control (apoptotic inducer and necrosis agent) was added at least 4 hours (h) before cell death assay and cell cultivation was terminated followed by manufacture suggested protocol. A pilot study was tested on the image visualization of positive and negative control in LNC or GFP HUVEC on a fibronectin 96 coated plate for 24 h prior to the actual experiment (Table 1; **FIG. 1**). An apoptosis inducer (Staurosporine) was used with a final concentration at 2µM (1:500). The negative control was treated with DMSO. Five represented field of images were documented. GFP-HUVEC or non-GLP LNC were counted and compared for positive apoptosis Annexin V shown in Cyanine 3 (yellow) and positive for necrosis (red-7-AAD). The percentage of positive of yellow or red in total GFP-HUVEC, LNCs or total cells was calculated and compared.

**Table 1. Experimental layout of pilot study.**

| | 1 | 2 | 3 | 4 | 5 |
|---|---|---|---|---|---|
| Co-Culture 1.5-2x10^4/ per 96 well | GFP-HUVEC (+ ctrl) | GFP-HUVEC (+Neg) | GFP-HUVE C | GFP-HUVE C | GFP-HUVEC +LNC |
| Treatment (25ug/ml ) | Fibronectin Apoptosis inducer (Staurosporine) | | PL | Immobilized HCHA | |
| Medium | **MESCM** on immobilized 96 wells | | | | |
| Readout | Cell counts positive of yellow or red in GFP HUVEC and/or LNCs. | | | | |

### Results and Conclusion

On plastic (PL) at 24h, apoptosis and necrosis were absent in HUVEC alone or co-cultured with LNC. When treated with HC-HA/PTX3 for 24h, HUVEC alone showed a significantly higher percentage (>7.1%, white arrows; **FIGS. 2A-2B**) necrosis than HUVEC co-cultured with LNC, suggesting that HUVEC co-cultured with LNC can prevent HUVEC from necrosis.

On PL, cell apoptosis was promoted in GFP-HUVEC alone on HC-HA/PTX3 but not in PL or HA (**FIG. 3A**, n=3). HC-HA/PTX3 promoted cell apoptosis in GFP-HUVEC but not in pericytes or LNC. However, simultaneously or sequentially added HC-HA/PTX3 at 6h post seeding promoted cell apoptosis in GFP-HUVEC when co-cultured with Pericytes or LNC alone (**FIG. 3B**, n=3). On Matrigel^{™} at 24h, GFP-HUVEC alone promoted classical tube formation (**FIG. 4**). When HC-HA/PTX3 was simultaneously added to GFP-HUVEC, the HUVEC failed to form tube formation (**FIG. 4**). When HC-HA/PTX3 was simultaneously added to LNC co-cultured with GFP-HUVEC, GFP-HUVEC tube formation was observed as early as 4h and GFP-HUVEC quickly formed aggregates and wrapped around by LNC at 24h. This result suggested that the reunion of LNC to GFP-HUVEC is critical to prevent GFP-HUVEC cell death that is otherwise induced by HC-HA/PTX3 (**FIG. 4**).

In summary, GFP-HUVEC formed classical vascular tube formation on Matrigel^{™}. HC-HA/PTX3 encouraged anti-angiogenesis effect by facilitating the apoptosis and necrosis in GFP-HUVEC alone. Reunion of GFP-HUVEC to LNC prevented GFP-HUVEC from apoptosis and necrosis induced by the HC-HA/PTX3.

### Example 2: A co-culture of LNC with HUVEC on HC-HA/PTX3 inhibited cell proliferation and promoted tube formation in HUVEC

In vitro vascular tube formation is the most direct functional evidence indicating the ability of endothelial cell to form vascular tubes and lumens. It remains unclear whether the addition of LNC can promote cell proliferation and angiogenesis in the presence of HC-HA/PTX3.

### Materials and methods

**Cells:** GFP HUVEC (P3) were purchased from Neuromics (Cat#GF01). These cells were isolated from normal human umbilical vein and transfected with GFP-lentiviral particle at passage 1. Puromycin resistant GFP HUVEC were maintained on fibronectin coated solution in Endo-growth medium containing 5% FBS and growth supplement until passage 3. Cells were split 1:3 every three days when ~70%-90% confluence is reached. Cells of passage 3-8 were used for all experiments.

LNC (P2-P5) was expanded in MESCM containing 4 ng/ml bFGF and 10 ng/ml LIF on 6-well plastic coated with 5% Matrigel^{™}.

### Results and Conclusion

Addition of soluble HA in HUVEC or pericytes (LNC) alone **(****FIGS. 2A-2B****)** promoted cell proliferation as suggested by Edu nuclear staining. In contrast, addition of soluble HC-HA/PTX3 inhibited cell proliferation of both cell types (**FIGS. 3A-3B**). When HUVEC seeded together with pericytes or LNCs simultaneously with treatment, HC-HA/PTX3 promoted cell death and inhibited proliferation in contrast to HA treatment at 24h (**FIG. 5**). The reunioned GFP-HUVEC and LNC and grew into sprout-like LNC at low dosage of HC-HA/PTX3 (25ug/ml) but inhibited the growth into sprout at high dosage (100ug/ml). (**FIG. 6**) The reunion GFP-HUVEC and LNC aggregates promoted angiogenesis sprouting on Matrigel^{™}.

### Example 3: Immobilized HC-HA/PTX3 promoted signaling that can be correlated with cell aggregation in P10 LNC

It remains unclear whether HC-HA/PTX3 uniquely promotes early signaling (CXCR4/SDF-1, HIF or other) before cell aggregation in P10 LNC.

### Materials and methods

**Cell culture:** For time course study, 1x10⁵/ml of P10 LNC was seeded on three substrates: 3D Matrigel^{™}, HA, or HC-HA/PTX3 in MESCM at 5, 15, 30, 60 mins, 24 h and 48 h (Table 2).

**Table 2. Experimental setup.**

| Experimental Group | | Length of Treatment (min) |
|---|---|---|
| | 1. P10 LNC HC-HA/PTX3 | 0 |
| | 2. P10 LNC HC-HA/PTX3 | 15 |
| | 3. P10 LNC HC-HA/PTX3 | 30 |
| | 4. P10 LNC HC-HA/PTX3 | 60 |
| | 5. P10 LNC HC-HA/PTX3 | 120 |
| | 6. P10 LNC HC-HA/PTX3 | 240 |
| | 7. P10 LNC HC-HA/PTX3 | 24h |
| | 8. P10 LNC HC-HA/PTX3 | 48h |
| | 9. P10 LNC 3D MG | 0 |
| | 10. P10 LNC 3D MG | 15 |
| | 11. P10 LNC 3D MG | 30 |
| | 12. P10 LNC 3D MG | 60 |
| | 13. P10 LNC 3D MG | 120 |
| | 14. P10 LNC 3D MG | 240 |
| | 15. P10 LNC 3D MG | 24h |
| | 16. P10 LNC 3D MG | 48h |
| | 17. P10 LNC HA | 0 |
| | 18. P10 LNC HA | 15 |
| | 19. P10 LNC HA | 30 |
| | 20. P10 LNC HA | 60 |
| | 21. P10 LNC HA | 120 |
| | 22. P10 LNC HA | 240 |
| | 23. P10 LNC HA | 24h |
| | 24. P10 LNC HA | 48h |

**qPCR:** Comparisons were made of mRNA expression of HIF1α, HIF1β, HIF2α, SDF-1, CXCR4, Hes1 at 5, 15, 30, 60, 120, 240 min or 24 and 48 h.

**Immunostaining:** LNC were then subjected to cytospin to determine nuclear translocation of HIF1α, HIF1β, HIF2α, SDF-1, CXCR4, and Hes1 at 5, 15, 30 and 60 mins.

### Results and Discussion

Time course of phase contrast of 10 LNC HC-HA/PTX3 were observed promoting sphere formation as early as 60 min and 120 min in 3D Matrigel^{™} (**FIG. 7**). The time course mRNA expression of CXCR4 (**FIG. 8A**) and SDF-1 (**FIG. 8B**) revealed HC-HA/PTX3 promoted the upregulation of transcript levels of CXCR4 as early as 15 min and reached at peak at 60 min. The upregulation of transcript levels of SDF-1 was promoted later after 240 min.

Immunofluorescence staining confirmed cytoplasmic/membrane CXCR4/SDF-1 were present in control (**FIGS. 9A-9D**). HC-HA/PTX3 promotes CXCR4 translocated to nucleus as early as 15 min and was prominently expressed in most cells at 30 min (**FIG. 9A**). At 60 min, CXCR4 was no longer expressed in nucleus (**FIG. 9A**). In contrast, HA and 3D MG promoted membrane translocation of CXCR4 (**FIG. 9B**). These data suggested HC-HA/PTX3 uniquely promotes transient nuclear CXCR4 prior to the sphere formation in P10 LNC at 60 min. (3D hanging drops derived aggregates have been shown to promote expression of CXCR4 in human MSC so as to promote the adhesion of HUVEC.)

It is unclear the role of the nuclear translocation of CXCR4. CXCR4 nuclear localization can promote nuclear HIF1α, and nuclear HIF1α promotes CXCR4 transcription, which promotes nuclear CXCR4 expression as a feed-forward loop in carcinomas metastasis. CXCR4 has also reported to translocate to nucleus upon the binding of SDF-1 or non-muscle myosin heavy chain IIA protein in renal carcinoma cells. Nuclear translation of CXCR4 has also been associated with HIF1α in rat neural crest stem cells during hypoxia to inhibit proliferation. HIF1α binds directly as the upstream to the hypoxia response element on the CXCR4 promoter and thereby up-regulates CXCR4 expression in endothelial cells and various carcinoma cells.

The time course mRNA expression pattern of the HIF signaling revealed that HC-HA/PTX3 uniquely upregulated expression of HIF1β (**FIG. 10A**) at 15 min while it did not cause any significant differences in HIF1α (**FIG. 10B**) or HIF2α (**FIG. 10C**). Immunofluorescence (IF) staining confirmed that HC-HA/PTX3 promotes nuclear staining of HIF1α (**FIG. 12A**) and HIF1β (**FIG. 11A**) at 5 min and sustained nuclear staining of HIF1α till 15 min and 30 min (significantly reduced in cell aggregates at 60 min). However, HA promoted both cytoplasmic and nuclear HIF1α (**FIG. 12B**) and HIF1β (**FIG. 11B**). IF staining showed nuclear phosphorylated PHD2 (Phospho-PHD2 (p-PHD2) serine 125) in the control. HC-HA/PTX3 uniquely inhibited nuclear p-PHD2 at 5 min and 15 min, during which time nuclear HIF1α was most notable (**FIG. 13A**). In contrast, HA and 3D MG maintained nuclear Phospho-PHD2 throughout 60 min (**FIG. 13B**). These data shows there was no significant differences between HC-HA/PTX3, HA, or 3D MG in the protein expression of the non-phosphorylated form of PHD2 (**FIGS. 13D-13F**) and PP2A/B55α (**FIGS. 15A-15C**). The IF data demonstrated HC-HC/PTX3 promoted high activities of nuclear PP2A C subunit at 5 and 30 min (**FIG. 14A**). In contrast, HA (**FIG. 14B**) or 3D MG (**FIG**. **14C**) showed significantly less expression of nuclear PP2A C subunit throughout the entire 60 min.

Interestingly, addition of AMD3100 upregulated two peaks of HIF-1α and HIF-1β transcripts, suggesting that with or without SDF1-CXCR4 signaling acts differently in transcript regulation of HIF-1α and HIF-1β (**FIGS. 10D** and **10E**)

IF staining showed that there were no differences in HIF2α (**FIG. 15**) and Aryl hydrocarbon receptor (AHR) (**FIG. 17**) during the entire time course study. The above data suggested HC-HA/PTX3 uniquely promotes transient nuclear expression of HIF1α and HIF1β (as early as 5 min), which was correlated with transient downregulation of phosphor-PHD2, which is known to promote the proteasomal degradation of HIF-1α. In contrast, HA promotes the continue expression of cytoplasmic and nuclear HIF1α and HIF1β and the maintenance of p-PHD2 (serine 125) in nucleus.

HIF1α is a master regulator of cellular processes including regulation of oxygen concentrations, aerobic glycolysis, cell migration, and inflammation. Interestingly, HIF-1α has been reported to be associated in mammalian tissue regeneration. HIF complex binds to DNA at specific promoter or enhancer sites [e.g. hypoxia response elements (HREs)], resulting in transcriptional regulation of more than 100 gene products. These include molecules of interest in regenerative processes such as angiogenesis, which is induced by vascular endothelial growth factor (VEGF), VEGF receptor-1 (VEGFR-1), platelet-derived growth factor (PDGF), and erythropoietin (EPO). Other processes involved in regeneration include tissue remodeling, which is induced by urokinase-type plasminogen activator receptor (uPAR), matrix metalloproteinase 2 (MMP2), MMP9, and tissue inhibitors of metalloproteinase (TIMPs), and glycolytic metabolism induced by lactate dehydrogenase (LDH), which converts pyruvate into lactate and pyruvate dehydrogenase kinase (PDK), which blocks the entry of pyruvate into the tricarboxylic acid (TCA) cycle. Inhibition HIF1a delay the spontaneous regeneration ear closer in adult MRL mouse suggesting HIF1a play central node for regeneration.

HIF1β is required for the ligand-binding subunit to translocate from the cytosol to the nucleus after ligand binding, enhances HIF target gene activation. HIF-1 binds to HRE sequence promoters in BMP4 and CXCR4.

PHD-2 is mainly in the cytoplasm, shuttles between the cytoplasm and the nucleus and can be in the nucleus in cancer cells. PHD2 mediated hydroxylation of HIF-1α predominantly occurs in the nucleus.

PHD2 is phosphorylated on serine 125 by m-TOR mediated P70S6-kinase (p70S6K) to increases its ability to degrade HIF1α, but dephosphorylated by PP2A/B55α. It remained unclear whether HC-HA/PTX3 promotes PP2A to dephosphorylate the PHD2 in the nucleus. Protein phosphatase 2A holoenzyme is a heterotrimeric protein composed of a structural subunit A, a catalytic subunit C, and a regulatory subunit B. Phosphorylation of PP2A at Tyr307 by Src occurs in response to EGF or insulin and results in a substantial reduction of PP2A activity. Reversible methylation on the carboxyl group of Leu309 of PP2A has been observed. Methylation alters the confirmation of PP2A, as well as its localization and association with B regulatory subunits.

HC-HA/PTX3 also uniquely induced unclear CXCR4 at 15 min (Example 3). Nuclear HIF1α accumulation requires nuclear translocation CXCR4 and nuclear HIF1α promoted CXCR4 transcription in a feed-forward loop to promote carcinoma metastasis. It has been reported the initiation HIF1α is required to upregulate SDF-1/CXCR signaling to promote positive feedback between glial-neuronal interaction in mouse central post-stroke pain suggesting strong relationship of feedback loop between HIF and CXCR4. It remains unclear about the relationship between nuclear CXCR4, HIF1 α, and p-PHD2.

mRNA expression of MMP2 and MMP9 has been shown to be downregulated in P4 LNC on 3D Matrigel^{™} but not by HC-HA/PTX3. It is unclear the protein level. It has also been shown that PTX3 and TSG-6 downregulate the activation of MMP1 and MMP-3 in mRNA and protein of conjunctivochalasis fibroblast. HC-HA/PTX3 inhibits MT1-MMP in HCF with/out TGF-β1. (P-272, unpublished protein data) It was possible the regenerative process may involve rapid turnover of MMPs by increase of TIMPs. Since MMPs and TIMPs are involved during tissue regeneration, it is tempting to speculate that HC-HA/PTX3 may be involved increase of TIMPs for quickly turnover MMPs.

Time course revealed HC-HA/PTX3 promoted mRNA expression of Hes1 as early as 15 min and at peak by thousand-fold at 120 min in P10 LNC when compared to the transcript level on HA or 3D Matrigel^{™} (**<0.05, n=3) **(****FIG. 18A****).** Expression levels of Notch3 **(****FIG. 18B****)** and Jag1 **(****FIG. 18C****)** were significantly upregulated when compare to 3D Matrigel^{™}.

Immunofluorescence staining confirmed the HC-HA/PTX3 promotes nucleus Hes1 as early as 5 min **(****FIG. 19A****)** where HA promotes nucleus Hes1 at as early as 15 min **(****FIG. 19B****).** Expression of Notch1 was verified and absent in the nucleus within 60 min suggesting the activation of Hes1 may be notch independent **(****FIG. 20A****).**

Hes1 has been known to regulate the undifferentiated status/maintenance of neural stem cell progenitors to promote proper neuronal differentiation and cell-cell interactive lateral inhibition. Expression of Hes1 often in an oscillatory manner every 2 hours as demonstrated in fibroblast and neural progenitors. Without Hes gene, progenitor cells prematurely differentiate into certain types of neurons only and are depleted before they have proliferated sufficiently for other neuronal and glial cell types. These data showed transient nuclear translocation of Hes1 within 5 min when treated by HC-HA/PTX3. The sustained expression of Hes1 enhanced repression the pro-neural gene and maintained the low proliferative or quiescence mode of cells. Notch-Hes1 mediation is responsible for activation of HIF1α signaling for phosphorylation STAT3 at Tyr 416. It remains unclear the mechanistic event responsible for nuclear translation of protein Hes1 but expressed from post-transcriptional event.

Expression of Hes1 has been demonstrated to be mediated through Notch dependent and -independent pathways to promote angiogenesis and neurogenesis. Oscillation of Hes1 has demonstrated notch independence and mediation through BMP and LIF signaling in ES cells, FGF2-JNK axis in ES derived neural progenitors, NGF-NF-KB with sustained expression of Hes1 to maintain the dendriotogenesis, VEGF-FLK-1-ERK for retinal progenitor proliferation and retinal ganglion cell fate specification and acetylation of Pax3 binding the promoter of Hes1 to enhance neural SC maintenance.

### Example 4: HC-HA/PTX3 promoted increased expression of angiogenic and neurogenic genes

It remained unclear whether the phenotypic changes in LNC by HC-HA/PTX3 were different from HA or 3D Matrigel^{™}. The early changes of mRNA levels were screened for angiogenic genes VEGF, CD31, VEGFRB and IGF-1 and neurogenic genes NGF and p75^{NTR}.

### Materials

**Cell culture:** 1x10⁵/ml of P10 LNC was seeded on three substrates: 3D Matrigel^{™}, HA, or HC-HA/PTX3 in MESCM for 48 h. For time course study on HC-HA/PTX3, P10 LNC will be treated HC-HA/PTX3 for 5, 15, 30, 60 mins, 24 h and 48 h.

**qPCR:** Comparison mRNA expression VEGF, PDGFα, CD31 and IGF-1 for angiogenesis and NGF and p75^{NTR} for neurogenesis at 5, 15, 30, 60, 120, 240 min 24 or 48 h on 3D MG, immobilized HA and immobilized HC-HA/PTX3.

**Immunostaining:** LNC were then subjected to cytospin to determine nuclear translocation of HIF1α, HIF1β, HIF2α, SDF-1, CXCR4, and Hes1 at 5, 15, 30 and 60 mins.

### Results and Conclusions

Time course on mRNA expression showed that HC-HA/PTX3 significantly upregulated transcript levels of VEGF **(****FIG. 21A****)** and PDGFα **(****FIG. 21B****)** in P10 LNC in a cyclic pattern as early as 15 min and peaked at 240 min when compare to 3D Matrigel^{™} or HA. HC-HA/PTX3 is also significantly upregulated the transcript levels of CD31 **(****FIG. 21C****)** and IGF-1 **(****FIG. 21D****)** at 240 min after the cell aggregation. Interestingly, HC-HA/PTX3 also significantly upregulated the transcript levels of NGF **(****FIG. 21E****)** and p75^{NTR} **(****FIG. 21F****)** within 24 h when compare to HA or 3D MG. The above data collectively suggests the HC-HA/PTX3 uniquely upregulated the angiogenic and neurogenic genes different from basement membrane on 3D Matrigel^{™} and HA within 24 h.

### Example 5: Early signaling in promoting angiogenesis

Previously it was found cell-cell aggregation between LNC and SC is mediated by CXCR4/SDF-1 axis, in which CXCR4 is strongly expressed by limbal stromal NCs and SDF-1 is expressed by SC. Inhibition of CXCR4 by AMD3100 or a blocking antibody to CXCR4 at the time of seeding disrupted their reunion and yielded separate aggregates with a reduced size, while resultant epithelial spheres exhibited more corneal differentiation and a notable loss of holoclones. It remained unclear whether HC-HA/PTX3 uniquely promoted early signaling (CXCR4/SDF-1, HIF or other) before cell aggregation in P10 LNC.

**Cell culture:** For time course study, 1x10⁵/ml of P4 LNC were seeded on substrates HA or HC-HA/PTX3 in MESCM at 5, 15, 30, 60 min, 24 h and 48 h (Table 3).

**Table 3**

| Experimental Group | | Length of Treatment (min) |
|---|---|---|
| | 1. P4 LNC HC-HA/PTX3 | 0 |
| | 2. P4 LNC HC-HA/PTX3 | 15 |
| | 3. P4 LNC HC-HA/PTX3 | 30 |
| | 4. P4 LNC HC-HA/PTX3 | 60 |
| | 5. P4 LNC HC-HA/PTX3 | 120 |
| | 6. P4 LNC HC-HA/PTX3 | 240 |
| | 7. P4 LNC HC-HA/PTX3 | 24h |
| | 8. P4 LNC HC-HA/PTX3 | 48h |
| | 9. P4 LNC HA | 0 |
| | 10. P4 LNC HA | 15 |
| | 11. P4 LNC HA | 30 |
| | 12. P4 LNC HA | 60 |
| | 13. P4 LNC HA | 120 |
| | 14. P4 LNC HA | 240 |
| | 15. P4 LNC HA | 24h |
| | 16. P4 LNC HA | 48h |

**qPCR:** Comparison mRNA expression of HIF1α, HIF1β, HIF2α, SDF-1, CXCR4, Hes1 for at 5, 15, 30, 60, 120, 240 min or 24 and 48 h.

**Immunostaining:** LNC then were subjected to cytospin to determine nuclear translocation of HIF1α, HIF1β, HIF2α, SDF-1, CXCR4, and Hes1 at 5, 15, 30 and 60 min.

### Results and Conclusions

**CXCR4:** Immunofluorescence staining confirmed cytoplasmic/membrane CXCR4 were present in the control. HC-HA/PTX3 promoted CXCR4 translocated to the nucleus 5, 30 min, transient nuclear translocation **(****FIG 9A****).** In contrast, HA does not **(****FIG. 9B****).** The above data suggested HC-HA/PTX3 uniquely promoted transient nuclear CXCR4 prior to the sphere formation and neurogenesis in P4 LNC. It has been shown that CXCR4 nuclear localization promoted nuclear HIF-1α and nuclear HIF-1α promoted CXCR4 transcription as a feed-forward loop in carcinomas metastasis. CXCR4 has also been reported translocate to nucleus upon the binding of SDF-1 or non-muscle myosin heavy chain IIA protein in renal carcinoma cells. Nuclear translation of CXCR4 has also been associated with HIF-1α in rat neural crest stem cells during hypoxia to inhibit proliferation. HIF1α binds directly as the upstream to the hypoxia response element on the CXCR4 promoter and thereby up-regulates CXCR4 expression in endothelial cells and various carcinoma cells.

**HIF:** Immunofluorescence staining suggested that HC-HA/PTX3 promoted extended HIF1α nuclear translocation (5-60 min) **(****FIG. 12A****).** Similarly, HA did the same **(****FIG. 12B****),** suggesting that this promotion was caused by HA molecules. The results suggested that HC-HA/PTX3 and HA played a role in angiogenesis. HC-HA/PTX3 promoted transit HIF1β nuclear translocation (15 min) **(****FIG. 11A****).** In contrast, HA promoted extended HIF1β nuclear translocation (5-30 min) **(****FIG. 11B****).** It is unclear what caused this discrepancy. No changes for HIF2α were observed in LNC treated with either HC-HA/PTX3 or HA, suggesting that HIF2α is not involved in actions mentioned above. HC-HA/PTX3 also promoted nuclear translocation of AHR (15 min) **(****FIG. 17****),** suggesting that AHR may play a role in angiogenesis.

HIF1β is required for the ligand-binding subunit to translocate from the cytosol to the nucleus after ligand binding, enhances HIF target gene activation. HIF-1 binds to HRE sequence promoters in BMP4 and CXCR4. HIP-1α is a master regulator of cellular processes including regulation of oxygen concentrations, aerobic glycolysis, cell migration, and inflammation. Interestingly, HIF-1α has reported associate in mammalian tissue regeneration. HIF complex binds to DNA at specific promoter or enhancer sites [hypoxia response elements (HREs)], resulting in transcriptional regulation of more than 100 gene products). These include molecules of interest in regenerative processes such as angiogenesis, which is induced by vascular endothelial growth factor (VEGF), VEGF receptor-1 (VEGFR-1), platelet-derived growth factor

(PDGF), and erythropoietin (EPO). Other processes involved in regeneration include tissue remodeling, which is induced by urokinase-type plasminogen activator receptor (uPAR), matrix metalloproteinase 2 (MMP2), MMP9, and tissue inhibitors of metalloproteinase (TIMPs), and glycolytic metabolism induced by lactate dehydrogenase (LDH), which converts pyruvate into lactate and pyruvate dehydrogenase kinase (PDK), which blocks the entry of pyruvate into the tricarboxylic acid (TCA) cycle. Inhibition HIF1α delay the spontaneous regeneration ear closer in adult MRL mouse suggesting HIF1α play central node for regeneration.

**Hes:** Hes1 has been known to regulate the undifferentiated status/maintenance of neural stem cell progenitors to promote proper neuronal differentiation and cell-cell interactive lateral inhibition. Expression of Hes1 often in oscillatory manner of every 2 hours demonstrated in fibroblast and neural progenitors. Without Hes gene, progenitor cells prematurely differentiate into certain types of neurons only and are depleted before they have proliferated sufficiently for other neuronal and glial cell types. These data showed that HC-HA/PTX3 promoted transient nuclear translocation of Hes1 in 15 min when treated by HC-HA/PTX3 **(****FIG. 19A****).** Similarly, HA promotes Hes1 nuclear translocation 5-15 min **(****FIG. 19B****).** The results suggest that Hes1 nuclear translocation is caused by HA.

**NICD and SDF1:** No changed were observed in LNC treated with HC-HA/PTX3 **(****FIG. 22D****)** or HA.

### Example 6: HC-HA/PTX3 purified from human amniotic membrane reverts late passaged limbal niche cells to nuclear Pax6+ neural crest progenitors by promoting cell aggregation via CXCR4/SDF-1 signaling

HC-HA/PTX3 was purified from water-soluble AM extract as a unique matrix consisting of high molecular weight hyaluronic acid (HA) covalently linked with heavy chain 1 (HC1) from inter-α-trypsin inhibitor ("-" is used to denote the covalent linkage) and further complexed with pentraxin 3 (PTX3) ("/" is used to denote the non-covalent linkage). HC-HA/PTX3 has been shown to exert an anti-inflammatory action that extends from innate immune responses by facilitating apoptosis of stimulated neutrophils and polarizing M2 macrophages to adaptive immune responses by suppressing activation of Th1 and Th17 lymphocytes to downregulate alloreactive immune responses. In addition, HC-HA/PTX3 also suppresses the TGF-β1 promoter activity in human corneal fibroblasts. Herein, it was discovered that HC-HA/PTX3 differs from 3D MG in reverting late passaged LNC to regain the nuclear Pax6+ NC progenitor status by promoting early cell aggregation through CXCR4/SDF-1 signaling but not BMP signaling.

### Results

### Progressive Loss of Nuclear Pax6+ NC Phenotype by Serial Passage of LNC

The serial passage of *LNC* to P10 results in the loss of the NC progenitor status that is characterized by nuclear Pax6 staining, expression of ESC markers and NC progenitor markers such as Sox2, p75NTR, Musashi-1, Nestin, Msx1, and FoxD3, and neuroglial differentiation. Because there are regional difference of expression of nuclear Pax6, LNC were serially passaged on coated MG in MESCM to P10 and characterized their phenotype by transcript expression and immunoassaying to establish the baseline. The results confirmed that the transcript expression level of Pax6, Sox2, p75NTR, Musashi-1, and Nestin by P10 LNC was indeed significantly reduced when compared to that of P2 LNC (FIG. 35A, ## p < 0.01, n=3). Immunofluorescence staining further confirmed the loss of nuclear staining of Pax6 in P10 LNC and notable reduction of staining to such NC markers as p75NTR and Musashi-1 when compared to P4 LNC **(****FIG. 35B****).**

### Immobilized HC-HA/PTX3 promotes cell aggregation and reverts P10 LNC to nuclear Pax6+ neural crest progenitors

P4 LNC expanded on coated MG in MESCM form cell aggregation when reseeded on 3D MG or immobilized HC-HA/PTX3, of which the latter also helps regain expression of ESC markers. It was wondered whether P10 LNC could behave the same to regain the nuclear Pax6+ NC progenitor status by reseeding on immobilized HC-HA/PTX3. P10 LNC expanded on coated MG in MESCM was therefore reseeded on coated MG, 3D MG or immobilized HC-HA/PTX3 in MESCM for 48 h. Phase contrast microscopy showed that P10 LNC formed cell aggregation in 3D MG and immobilized HC-HA/PTX3 at 24 h and 48 h **(****FIG. 24A****).** Quantitative RT-PCR showed that transcript levels of Pax6, p75^{NTR}, Musashi-1, Nestin, Msx-1, and FoxD3 were significantly upregulated in P10 LNC on immobilized HC-HA/PTX3 when compare to on coated MG **(****FIG. 24B**, ** p < 0.01, n=3) or 3D MG **(****FIG. 24B**, ^{##} p < 0.01, n=3). The immunofluorescence staining confirmed the reappearance of nuclear Pax6 with other neural crest markers, Sox2, p75^{NTR} and Musashi-1 but no difference in Nestin **(****FIG. 24E****).** The differentiation potential into neurons, oligodendrocytes, and astrocytes by P10 LNC after being re-seeded on 3D MG or immobilized HC-HA/PTX3 was examined. Phase contrast microscopy showed that P10 LNC exhibited a reduced size and adopted expanded differentiation potential into neurons, astrocytes and oligodendrocytes in when re-seeded on immobilized HC-HA/PTX3 when compared to their counterpart re-seeded in 3D MG **(****FIG. 24D****).** Collectively, these results suggested that immobilized HC-HA/PTX3, but not 3D MG, uniquely reverted P10 LNC to nuclear Pax6+ NC progenitors with higher neuroglial differentiation potential.

### Soluble HC-HA/PTX3 promotes early cell aggregation and reverts to Pax6+ NC progenitors

It was then tested whether soluble HC-HA/PTX3 added directly into MESCM in P10 LNC seed on coated MG might also achieve the same outcome. Phase contrast microscopy showed that cell aggregation was also promoted by soluble HC-HA/PTX3 as early as 60 min (marked by a white arrow) but aggregated cells spread to single spindle cells on coated MG by 24 h while cell aggregation became more prominent in 3D MG **(****FIG. 25A****)** similar to what is shown in Figure 2. Quantitative RT-PCR revealed significant upregulation of p75^{NTR}, NGF and Musashi-1 transcripts by soluble HC-HA/PTX3 at 24 and 48 h when compared to 3D MG **(****FIGS. 25B-25D****,** ^{##} p<0.01, n=3). Immunofluorescence staining also confirmed nuclear staining of Pax6 and Sox2 and cytoplasmic staining of p75^{NTR} achieved by soluble HC-HA/PTX3 when compared to cells cultured on 3D MG at 48 h **(****FIG. 25A****).** Such a staining pattern resembled what was noted on immobilized HC-HA/PTX3 **(****FIG. 24E****).**

### Cell aggregation promoted by soluble HC-HA/PTX3 is mediated by CXCR4/SDF-1 signaling and leads to nuclear Pax6+ NC progenitors

Previously it had been reported the reunion between P4 LNC and LEPC in 3D MG is mediated by CXCR4/SDF-1 signaling with the receptor CXCR4 strongly expressed by LNC and SDF-1 ligand expressed by LEPC and such reunion is pivotal to maintain self-renewal of LEPC. Therefore, it was wondered whether cell aggregation promoted by soluble HC-HA/PTX3 might also be mediated by CXCR4/SDF-1 signaling in P10 LNC. To test this hypothesis, CXCR4/SDF-1 signaling was perturbed by addition of AMD3100, which is a small-molecule CXCR4 inhibitor. Phase contrast microscopy confirmed that cell aggregation was indeed promoted by soluble HC-HA/PTX3 at 60 min in P10 LNC, similar to what was noted above, and that such aggregation was completely aborted by AMD3100 **(****FIG. 26A****).** The time course study of the transcript expression by qRT-PCR showed that CXCR4 transcript was marked upregulated by four-fold as early as 15 min and reached a high peak by nearly 500-fold at 60 min when soluble HC-HA/PTX3 was added to P10 LNC on coated MG in comparison to their counterpart in 3D MG **(****FIG. 26B****,** ** p < 0.01 and ** p < 0.01, n=3). Addition of AMD310 significantly downregulated such upregulation of CXCR4 transcript at 24 h and completely aborted at 48 h **(****FIG. 26B****).** In contrast, the SDF-1 transcript was not upregulated during the first 60 min in all cultures but was significantly upregulated by 40-fold at 24 h by 3D MG and 10-fold by soluble HC-HA/PTX3, of which the latter was also completely abolished by AMD3100 **(****FIG. 26B**, ## p<0.01, n=3). Immunofluorescence staining of CXCR4 showed membrane/cytoplasmic staining throughout the 60 min period in 3D MG. In contrast, CXCR4 staining was membrane/cytoplasmic at 0 and 5 min but nuclear at 15 and 30 min and reverted to predominant membranous in cell aggregation at 60 min in soluble HC-HA/PTX3 **(****FIG. 26D****).** The latter staining pattern was reverted to that of 3D MG when AMD3100 was added **(****FIG. 26D****).** In contrast, the immunofluorescence of SDF-1 was strongly membranous/cytoplasmic throughout 60 min in cells seeded in 3D MG or soluble HC-HA/PTX3 and became negative after addition of AMD3100 **(****FIG. 26D****).** Blockade of CXCR4/SDF-1 signaling by AMD3100 not only prevented cell aggregation promoted by soluble HC-HA/PTX3 but also led to significant downregulation of Pax6, p75NTR, NGF, Musashi-1, Msx-1 and FoxD3 transcripts **(****FIG. 26E****,** ** p<0.01, n=3). Furthermore, nuclear Pax6 staining promoted by soluble HC-HA/PTX3 was aborted by AMD3100 in P10 LNC **(****FIG. 26D****).** To confirm the abovementioned finding, quantitative comparison of subcellular cytoplasmic and nuclear fractions of CXCR4 and Pax6 were conducted. Consistent to what was observed, Western blot analysis showed both CXCR4 and Pax6 protein decrease in cytoplasmic fractions with increase of nuclear translocation at 15 and 30 min in soluble HC-HA/PTX3. **(****FIG. 26F****)** Blockade of CXCR4/SDF-1 signaling by AMD3100, both CXCR4 and Pax6 remain in cytoplasmic fraction at all time point. **(****FIG. 26F****)** These data collectively indicated that cell aggregation promoted by soluble HC-HA/PTX3 was mediated by CXCR4/SDF-1 signaling, which was causatively linked to the regain of the nuclear Pax6+ NC progenitor phenotype in P10 LNC.

### CXCR4/SDF-1 is required for activation of BMP signaling by HC-HA/PTX3

It has been reported that immobilized HC-HA/PTX3, but not 3D MG, upregulates BMP signaling in P4 LNC, which is responsible for the maintenance of limbal SC quiescence. Thus, it was questioned whether BMP signaling might also be promoted by soluble HC-HA/PTX3 in P10 LNC and if so whether it might be affected by CXCR4/SDF1 signaling activated by HC-HA/PTX3. qRT-PCR showed that transcript expression of BMP ligands and BMP receptors by P10 LNC was significantly downregulated when compared to P4 LNC expanded on coated MG **(****FIG. 27A****,** ** p<0.01, n=3) Immunofluorescence staining confirmed that nuclear localization of pSmad1/5/8 was weakly expressed in P4 LNC and nil in P10 LNC **(****FIG. 27B****).** In contrast, qRT-PCR revealed that the expression levels of BMP2, BMP4, and BMP6 transcripts were significantly upregulated by soluble HC-HA/PTX3 when compared to 3D MG. **(****FIGS. 27C-27E****,** ## p < 0.01, n=3) Interestingly, the upregulation of BMP4 and BMP6 was as early as 15 min and cyclic to a higher level toward 48 h while that of BMP2 was only noted after 24 h **(****FIGS. 27C-****27E).** Addition of AMD3100 aborted the transcript levels of BMP2, BMP4, and BMP6 throughout 48 h **(****FIGS. 27C-27E****,** ** p<0.01, n=3). Immunofluorescence staining further confirmed strong nuclear staining of pSmad1/5 indicating that canonical BMP signaling was promoted by soluble HC-HA/PTX3 in P10 LNC but absent nuclear staining after being treated with AMD3100 **(****FIG. 27F****).** Western blot confirmed soluble HC-HA/PTX3 promotes nuclear pSmad1/5 as early as 5, 15, 30 min; Blockade of CXCR4/SDF-1 signaling by AMD3100, nuclear pSmad1/5 was not promoted. **(****FIG. 27G****)** These findings strongly suggested that CXCR4/SDF-1 signaling promoted by HC-HA/PTX3 was also causally linked to activation of canonical BMP signaling in P10 LNC.

*Suppression of BMP signaling does not affect nuclear Pax6 staining and cell aggregation mediated by CXCR4*/*SDF-1 signaling promoted by HC-HA*/*PTX3*

BMP signaling promoted by soluble HC-HA/PTX3 was perturbed to determine whether BMP signaling was required for cell aggregation mediated by CXCR4/SDF-1 signaling. To do so, P10 LNC was pre-treated with or without SDN-193189, a small molecule BMP inhibitor (data not shown) or short interfering RNAs (siRNA) to BMP receptors, i.e., BMPR1A, BMPR1B, BMPR2, and Activin A receptor, type I (ACVR1) seeded on coated MG before adding soluble HC-HA/PTX3 in MESCM for another 48 h. Quantitative RT-PCR and immunofluorescence staining confirmed the efficiency of siRNAs to BMP receptors in reducing the transcript expressions of BMP receptors **(****FIG. 36A****,** ** p<0.01, n=3) and preventing nuclear staining of pSmad1/5/8 **(****FIG. 36B****).** However, phase contrast microscopy revealed that cell aggregation of P10 LNC by soluble HC-HA/PTX3 was not affected by either LDN-193189 or siRNAs to BMP receptors when compared to the control pre-treated with scrambled RNA (scRNA) **(****FIG. 36C****).** Quantitative RT-PCR further revealed that there was no significant difference in the expression level of CXCR4 and SDF-1 throughout 48 h when P10 LNC were pre-treated siRNAs to BMP receptors **(****FIGS. 36D-36E****,** P>0.1 n=3). Furthermore, immunofluorescence staining also showed that the transient nuclear translocation of CXCR4 and nuclear Pax6 staining were not affected **(****FIG. 36F****).** Collectively, these data indicated that cell aggregation, nuclear Pax6 staining, and activation of CXCR4/SDF-1 signaling by HC-HA/PTX3 were not affected when canonical BMP signaling was inhibited.

### Discussion

Early passaged P4 LNC regain the expression of ESC markers lost during serial passage in coated MG when reseeded on immobilized HC-HA/PTX3. Herein, it was shown that late passaged P10 LNC also regained the nuclear Pax6+ NC multipotent NC progenitor phenotype lost during serial passage when reseeded on immobilized HC-HA/PTX3 **(****FIGS. 24A-24E****).** Although both immobilized HC-HA/PTX3 and 3D MG promoted cell aggregation **(****FIGS. 24A-****24E),** such phenotypic reversal was unique to HC-HA/PTX3 because cell aggregation occurred as early as 60 min when soluble HC-HA/PTX3 was added in MESCM even when P10 LNC were still cultured on coated MG, but not in their counterparts without HC-HA/PTX3 or reseeded on 3D Matrigel **(****FIGS. 25A-25E****).** The notion that cell aggregation induced by HC-HA/PTX3 was different from that by 3D MG was further supported by activation of CXCR4/SDF-1 signaling by the former but not the latter. This was illustrated by notable upregulation of CXCR4 transcript and nuclear translocation of CXCR4 prior to cell aggregation facilitated by HC-HA/PTX3 **(****FIGS. 26A-26F****).** Suppression of CXCR4 by AMD3100 not only abolished upregulation of CXCR4 transcript and nuclear translocation of CXCR4 but also eliminated membranous and cytoplasmic staining of SDF-1 to interrupt CXCR4/SDF-1 signaling. Because it also abolished cell aggregation at 60 min, we concluded that early cell aggregation facilitated by HC-HA/PTX3 was mediated by CXCR4/SDF-1 signaling and pivotal to the phenotypic reversal to nuclear Pax6+ NC progenitor status as illustrated by the finding after addition of AMD3100 **(****FIGS. 26A-****26F).** Because phenotypic reversal occurred only by HC-HA/PTX3 but not Matrigel, of which both caused cell aggregation, it was speculated that cell aggregation triggered by homotypic CXCR4/SDF-1 signaling is unique. Future studies are needed to see if such a mechanism can be expanded to understand mesenchymal cell aggregation/condensation that is linked to promote organogenesis in tooth, bone, hair, skin and muscle.

CXCR4 is highly expressed in LNC subjacent to limbal basal epithelial stem/progenitors, but its expression also declined with serial passage on coated Matrigel (data not shown). Herein, it was noted nuclear translocation of CXCR4 soon after addition of HC-HA/PTX3 **(****FIGS. 26A-26F****).** Furthermore, addition of AMD3100 prevented such transient nuclear translocation of CXCR4 and abolished cell aggregation and ensuing phenotypic reversal **(****FIGS. 26A-26F****).** Therefore, it is tempting to speculate that HC-HA/PTX3 activates CXCR4/SDF-1 signaling by nuclear translocation of CXCR4. As yet nuclear location of CXCR4 has been regarded as a strong indicator for high malignancy in several cancer cells and associated with HIF1α as a feed- forward loop to promote tumor growth and cancer metastasis. Because nuclear translocation of CXCR4 in LNC occurred in normal cells and much faster, i.e., 15 and 30 min after addition of HC-HA/PTX3, than what has been noted by sustained SDF-1 stimulation in cancer cells, future studies are needed to determine whether nuclear translocation of CXCR4 in LNC is promoted by HC-HA/PTX3 through a similar mechanism.

Immobilized HC-HA/PTX3, but not 3D MG, has been shown to activate BMP signaling in P4 LNC, which is required to maintain limbal epithelial SC quiescence. Herein, it was learned that BMP signaling evidenced by nuclear translocation of pSmad1/5/8 and upregulation of BMP ligands and receptors was also lost during serial passage **(****FIGS. 27A-27B****)** along with the loss of nuclear Pax6 staining **(****FIGS. 35A-35B****).** However, both immobilized (not shown) and soluble HC-HA/PTX3 uniquely activated BMP signaling in P10 LNC as evidenced by nuclear staining of pSmad1/5/8 at 30 min and upregulation of BMP4 and BMP6 transcript in a cyclic wave pattern before cell aggregation **(****FIGS. 27A-27G****).** BMP signaling is involved during the early stage of somatic cell reprogramming, which is also highlighted by cell aggregation and mesenchymal epithelial transition from adult skin fibroblasts to Induced Pluripotent Stem cells (iPSCs). These data revealed that disruption of CXCR4/SDF-1 signaling by AMD3100 abolished the aforementioned BMP signaling promoted by HC-HA/PTX3 **(****FIGS. 27A-27G****).** In contrast, disruption of BMP signaling by siRNAs to BMP receptors neither affected cell aggregation mediated by CXCR4/SDF-1 signaling based on CXCR4 transcript expression and nuclear CXCR4 staining nor abolished nuclear Pax6 staining **(****FIGS. 36A-36F****).** Collectively, these results suggest that HC-HA/PTX3 promotes early cell aggregation by activating CXCR4/SDF-1 signaling, which is also required to activate BMP signaling in P10 LNC and that CXCR4/SDF-1 signaling is, but BMP signaling is not pivotal in the phenotypic reversal of P10 LNC.

HC-HA/PTX3 purified from human AM exerts a broad anti-inflammatory and anti-scarring actions and supports LNC to ensure limbal epithelial SC quiescence. These actions collectively explain the molecular mechanism explaining why cryopreserved amniotic membrane may promote regenerative healing. Herein, for the first time, evidence has been provided to suggest that HC-HA/PTX3 may also facilitate the reversal of aged LNC to regain their Pax6+ NC progenitor status, a finding that helps explain why transplantation of AM augments the success of *in vivo* and *ex vivo* expansion of limbal SCs to treat corneal blindness caused by limbal SC deficiency. Because Pax6+ NC progenitors have wide differentiation potential into neurovascular cells, HC-HA/PTX3 might also support SC in many other neurovascular niches of the body.

### Material and Methods

### Cell Isolation and Expansion

Human corneolimbal rim and central cornea button stored at 4°C in Optisol (Chiron Vision, Irvine, CA) for less than 7 days were obtained from donors (Florida Lions Eye Bank, Miami, FL). Tissue were rinsed three times with PBS pH 7.4 containing 50 µg/ml gentamicin and 1.25 µg/ml amphotericin B, the excess sclera, conjunctiva, iris, corneal endothelium and trabecular meshwork were removed up to the Schwalbe's line for the corneoscleral rim before being cut into superior, nasal, inferior, and temporal quadrants at 1 mm within and beyond the anatomic limbus. An intact epithelial sheet, including basal epithelial cells, was obtained by subjecting each limbal quadrant to digestion with 10 mg/ml dispase in modified embryonic stem cell medium (MESCM), which was made of Dulbecco's Modified Eagle's Medium (DMEM)/F-12 nutrient mixture (F-12) (1:1) supplemented with 10% knockout serum, 10 ng/ml LIF, 4 ng/ml bFGF, 5 mg/ml insulin, 5 mg/ml transferrin, 5 ng/ml sodium selenite supplement (ITS), 50 µg/ml gentamicin and 1.25 µg/ml amphotericin B in plastic dishes containing at 4°C for 16 h under humidified 5% CO2 incubator. Remaining stroma were subjected to 2 mg/mL collagenase A at 37°C for 16 h to generate floating clusters.

For expansion, single cells derived from limbal clusters or CSC after digestion with 0.25% trypsin and 1mM EDTA (T/E) were seeded at 1x10⁴/cm² in the 6-well plate pre-coated with 5% Matrigel^{™} in MESCM and cultured in humidified 5% CO2 with media change every 3-4 days for total 6-7 days. For cells culture in three-dimensional (3D) Matrigel, Matrigel was prepared by adding 50% Matrigel diluted in MESCM per chamber of an 8-well chamber slide following incubation at 37°C for 60 min. LNC cells were seeded in 3D Matrigel and cultured for 24 h or 48 h in MESCM.

Upon 80% confluence, P10 LNC cultured on coated MG were pre-treated with 0.1% DMSO with or without 20 µg/mL AMD3100 or 100 nM LDN-193189 for 30 min before being trypsinized and seeded at 2x10⁵/mL on coated MG in MESCM containing 20 µg/mL of AMD3100 or 100 nM LDN-193189 with 20 µg/mL soluble HC-HA/PTX3 for another 48 h. For the siRNA knockdown, 80% confluent P10 LNC on 6-well coated MG were subjected to transfection by mixing 200 µL of serum-free, antibiotic-free MESCM with 4 µL of HiPerFect siRNA transfection reagent (Final dilution, 1:300) and 6 µL of 20 µM of scRNA or siRNAs for BMPR1A, BMPR1B, BMPR2, and ACVR1 at the final concentration of 100 nM, drop-wise, followed by culturing in 1 mL of fresh MESCM at 37°C for 24 h before soluble HC-HA/PTX3 was added at a final concentration of 20 µg/mL in MESCM.

### Purification, characterization and immobilization of HC-HA/PTX3

HC-HA/PTX3 was purified from cryopreserved human placentas provided by Bio-Tissue, Inc. (Miami, FL), with modification. In brief, AM retrieved from placenta was cryopulverilzed by FreezeMill (FreezerMill 6870, SPEX^{®} SamplePrep, Metuchen, NJ), extracted by PBS (pH 7.4) at 4 °C for 1 h, and the centrifuged at 48,000 x g at 4 °C for 30 min to generate the supernatant which was designated as AM extract. This extract was then fractionated by ultracentrifugation in a CsCl gradient at an initial density of 1.35 g/ml in 4 M GnHCl at 125,000 g at 15 °C for 48 h (Optima^{™} L-80 X, SW41 rotor, Beckman Coulter, Indianapolis, IN). A total of 12 fractions (1 mL/fraction) were collected from each ultracentrifuge tube. The weight of each fraction was measured to calculate the density, while HA content and protein content in each fraction were measured by the enzyme-linked immunosorbent HA Quantitative Test Kit (Corgenix, Broomfield CO) and the BCA Protein Assay Kit (Life Technologies, Grand Island, NY), respectively. The fractions of 2 - 12 which contained most of HC-HA/PTX3 were pooled and were further subjected to three consecutive runs of ultracentrifugation at 125,000 g in CsCl/4 M guanidine HCl at a density of 1.40 g/mL for the 2^{nd} run and 1.42 g/mL for 3^{rd} and 4^{th} run, each run at 15 °C for 48 h. The fractions 3-9 after the 4^{th} run were pooled and dialyzed against distilled water at 4 °C for 48 h with a total of 5 times of water change, lyophilized, stored at -80 °C, and designated as HC-HA/PTX3. Before use, HC-HA/PTX3 was qualified by verifying its biochemical composition containing high molecular weight HA based on agarose gel electrophoresis and validate the presence of HC1 (ab70048, Abcam, Cambridge, MA) and PTX3 (ALX-804-464-C100, Enzo Life Sciences, Farmingdale, NY) in purified HC-HA/PTX3 by Western blot with or without HAase digestion (1 U/µg HA) in the presence of protease inhibitors (Sigma-Aldrich, St. Louis, MO). Because the negligible amount of protein therein, the amount of HC-HA/PTX3 used in the experiment was expressed based on the HA amount.

100 µL of 20 µg/mL HC-HA/PTX3 was immobilized on Covalink-NH 96 wells (Pierce) by first sterilizing the Covalink-NH 96 wells in 70% alcohol for 30 min and then the wells were washed with distilled water two times. HC-HA/PTX3 with the crosslinking reagents of Sulfo-NHS at 9.2 mg/mL (Pierce) and 1-ethyl-3(3-dimethylaminopropyl) carbodiimide (Pierce) at 6.15 mg/mL were added to each well and incubated at 4 °C overnight. After that, the un-crosslinked HC-HA/PTX3 and crosslinking reagents were removed and the wells were washed twice with 2 M NaCl/50 mM MgSO₄/PBS, followed by two washes of PBS.

### Neuroglial Differentiation

A total of 1x10^{4/}ml of P10 LNC were seeded on 50 µg /ml poly-L-ornithine and 20 µg/ml laminin-coated or Collagen Type IV coated cover glass in 48-well plate in NSCM supplement with 0.5% N2 and 1% B27 for 2 days. For neuronal differentiation, medium was then replaced to neuronal induction base medium containing DMEM/F12 (1:3) with 0.5% N2 and 1% B27 in additional to 10 ng/ml FGF2 and 20 ng/ml of BDNF (medium A) for 3 days and replaced with base medium in addition to 6.7 ng/ml FGF2 and 30 ng/ml of BDNF for another 3 days. Cell then replaced to base medium in addition to 2.5 ng/ml FGF2, 30 ng/ml BDNF, and 200 mM ascorbic acid for another 8 days. For oligodendrocyte differentiation, medium then replaced with base medium containing DMEM/F12 (1:1) with 1% N2 in addition to 10 ng/ml FGF2, 10 ng/ml PDGF, and 10 µM forskolin for 4 days and then medium was replaced by the base medium in addition to 10 ng/ml FGF2, 30 ng/ml 3,3,5-triiodothyronine, and 200 µM ascorbic acid for another 7 days. For astrocyte differentiation (Thermo Scientific, Santa Clara, CA), medium was replaced by DMEM containing 1% FBS, 1% N2, and 2mM GlutaMax for 10 days. Induction media were changed every 3-4 days.

### Subcellular fractionation and western blotting

Nuclear and cytoplasmic fractions were prepared using the NE-PER^{®} Nuclear and Cytoplasmic Extraction Reagents Kit (Pierce, Rockford, IL, USA) as per manufacturer's instruction. Briefly, the treated P10 LNC were washed once on cold PBS and centrifuged at 500 g for 5 min.

The cell pellet was suspended in 100 µL of cytoplasmic extraction reagent I containing protease inhibitor by vortexing. The suspension was incubated on ice for 10 min followed by the addition of 6 µL of a second cytoplasmic extraction reagent II, vortexed for 5s, incubated on ice for 1 min and centrifuged for 5 min at 16 000 g. The supernatant fraction (cytoplasmic extract) was transferred to a pre-chilled tube. The insoluble pellet fraction, which contains crude nuclei, was resuspended in 50 µL of nuclear extraction reagent by vortexing during 15s three times and incubated on ice for 10 min each, then centrifuged for 10 min at 16 000 g. The resulting supernatant, constituting the nuclear extract, was used for the subsequent experiments. Protein concentration was quantitated using the BCA protein assay kit (Pierce). Equal amounts of protein were loaded in each lane and separated on 4-15% gradient acrylamide gels under denaturing and reducing conditions for Western blotting. The protein extracts were transferred to the nitrocellulose membrane, which was then blocked with 5% (w/v) fat-free milk in TBST. [50 mM Tris-HCl, pH 7.5, 150 mM NaCl, 0.05% (v/v) Tween-20], followed by sequential incubation with the specific primary antibody against either Pax6, CXCR4, phospho-Smad1/5/8 and its respective horseradish peroxidase (HRP)-conjugated secondary antibody using β-actin and Histone H3 for their respective cytoplasmic or nucleus fraction of loading control. Immunoreactive proteins were detected with Western Lighting Chemiluminescence (PerkinElmer, Waltham, MA) and images captured by GE ImageQuant LAS 4000 (GE Healthcare Biosciences, Pittsburgh, PA).

### Quantitative Real-Time PCR

Total RNAs were extracted from expanded LNC by RNeasy Mini Kit (Qiagen, Valencia, CA) according to manufacturer's guideline and 1-2 ug of RNA extract was reverse transcribed to cDNA with reverse-transcribed using Applied Biosystem^{™} High Capacity Reverse Transcription Kit (Thermo Fisher, Santa Clara, CA) using primers. The resultant cDNAs were amplified by specific TaqMan gene expression assay mix and universal PCR master mix in QuantStudio^{™} 5 Real Time PCR System (Thermo Fisher, Santa Clara, CA) with real-time RT-PCR profile consisting of 10 min of initial activation at 95°C, followed by 40 cycles of 15 sec denaturation at 95°C, and 1 min annealing and extension at 60°C. The threshold was set at 10 times the standard deviation above the mean baseline emission value for the first 15 cycles. Threshold cycle number (Ct) was calculated with QuantStudio Design and Analysis v.1.4.3 (Thermo Fisher, Santa Clara, CA). The relative gene expression data were analyzed by the comparative CT method (ΔΔCT). All assays were performed in triplicate. The results were normalized by glyceraldehyde 3-phosphate dehydrogenase (GAPDH) as an internal control. All assays were performed in triplicate.

### Immunofluorescence Staining

Single cells of LNC or CSC at different passages were harvested with 0.05% trypsin and 1mM EDTA at 37°C for 10 min and prepared for cytospin using Cytofuge (StatSpin Inc., Norwood, MA) at 1000 g for 8 min. Cells were fixed with 4% formaldehyde, pH 7.0, for 15 min at room temperature, permeabilized with 0.2% Triton X-100 in PBS for 15 min and blocked with 2% bovine serum albumin (BSA) for 1 h before incubated with primary antibodies for 16 h at 4°C. After 3 washes with PBS, the corresponding Alexa Fluor-conjugated secondary IgG (all 1: 100 dilution) were incubated for 60 min and 3 washing with PBS. After 3 washes with PBS, the second primary antibodies were incubated for 60 min and followed with the corresponding Alex Fluor-conjugated secondary IgG. The nucleus was counterstained with Hoechst 33342 before being analyzed with Zeiss LSM 700 confocal microscope (Carl Zeiss, Thornwood, NY). Corresponding mouse and rabbit sera were used as negative controls for primary monoclonal and polyclonal antibodies, respectively.

### Statistical analysis

All summary data were reported as mean ± SD. Significance was calculated for each group and compared with two-tailed Student's t-test by Microsoft Excel (Microsoft, Redmond, WA). Test results were reported as p values, where p < 0.05 were considered statistically significant.

### Example 7: Pax6 controls neural crest potential of limbal niche cells to support self-renewal of limbal epithelial stem cells

On the ocular surface, corneal epithelial stem cells (SCs) reside in limbus bordered between cornea and conjunctiva. From the limbal stroma subjacent to limbal epithelial SC, a subpopulation of limbal niche cells (LNC) that express SC markers such as Oct4, Sox2, Nanog, Rex1, Nestin, N-cadherin, and SSEA4 and exhibit differentiation potential into vascular endothelial cells, pericytes, osteoblasts, chondrocytes, and adipocytes. From the entire human limbal stroma, others have also isolated progenitors that can differentiate into neurons and retinal sensory cilia. It has been reported that limbal niche cells (LNC) in the stroma support limbal epithelial stem (progenitor) cells better by promoting holoclones and preventing corneal epithelial differentiation than that in central corneal stromal cells. Interestingly, a subpopulation of corneal stromal cells (CSC) can also be isolated to exhibit sphere formation and differentiation potential into adipocytes, neurons, and chrondrocytes besides keratocan-expressing keratocytes. The aforementioned limbal and corneal stromal progenitors expressed developmental neural crest genes, such as ATP binding cassette (ABCG2), Nestin, Musashi-1, Sox2, Six2/3, and Sox9. These results indicated both limbal and corneal stroma may contain multi-potent progenitors. It is plausible that these stromal progenitors are derived from migrating per-ocular mesenchyme of the cranial neural crest during development.

Paired box homeotic gene 6 (Pax6) is an evolutionally conserved transcription factor essential for proper development of eye, central nerve system, craniofacial skeletal, olfactory epithelium, and pancreas. In the eye, the primarily function of Pax6 is mediated the commitment of head ectoderm of optic vesicle into the lens ectoderm and promote the formation of lens vesicle. Homozygous Pax6-deficient mouse embryo exhibits lack of eyes and nose and dies soon at birth. Expression of Pax6 is dosage dependent as a mutation or missing allele leads to aniridia in humans and the small eye (sey, Pax6^{+/-}) in mouse animal model. Patients with aniridia-related keratopathy (ARK) observed as typical ocular surface disease with limbal stem cells deficiency (LSCD). However, the pathophysiology of underlying mechanism that leads to LSCD remains to be elucidated. Post-natal expression of Pax6 is restricted to corneal and limbal epithelial cells. Studies reported inadequate levels of Pax6 in corneal epidermis leads to abnormal differentiation in human and mouse. Interestingly, Pax6^{+/-} in heterozygous adult mice has profound severe defect in cornea stroma and endothelium but less of impact in epithelial cells with delay wound healing. Because transient expression of Pax6 is noted in the corneal stroma during development and in aforementioned limbal and corneal stromal progenitors, it was hypothesized that expression of Pax6 in the limbal stroma might have a unique developmental role in maintaining corneal epithelial homeostasis. Herein, the expression and nuclear localization of Pax6 was found to differentiate LNC from CSC and causally correlated with the neural crest progenitor status regarding marker expression, neurosphere formation, and neuroglial differentiation. Furthermore, such a phenotype is crucial to endow LNC with the capability of supporting self-renewal of limbal epithelial SCs by suppressing corneal epithelial differentiation and maintaining holoclone formation.

### Results

### Unique Nuclear Expression of 46 kDa Pax6 in Limbal Niche Cells

To determine whether there was any difference between LNC and CSC in the expression of Pax6 immediately after isolation, LNC and CSC were isolated from epithelium-containing limbal stroma and epithelially denuded corneal stroma from the same donor using collagenase digestion. Double immunostaining of Pax6 and pan-cytokeratin (PCK) showed positive nuclear staining of Pax6 in PCK (+) epithelial cells as expected but also in freshly isolated PCK (-) LNC **(****FIG. 29A****,** arrows). In contrast, weak cytoplasmic staining of Pax6 was noted in PCK (-) CSC **(****FIG. 29A****).** LNC and CSC were then expanded on coated Matrigel^{™} in a modified serum-free ESC medium (MESCM) and compared to CSC expanded on plastic in DMEM/10%FBS or in neural stem cell expansion medium (NSCM). Phase images showed that cells in these cultures at the same passage 4 (P4) all exhibited similar spindle cells **(****FIG. 29B****).** Compared to P4 CSC cultured on coated Matrigel^{™} in MESCM, P4 LNC had significant higher transcript expression of Pax6 as well as other neural crest markers such as p75^{NTR}, Musashi-1, Sox2, Nestin, Msx1, and FoxD3 **(****FIG. 29C****,** ## p<0.05). Compared to P4 CSC expanded on coated Matrigel^{™} in MESCM, expression of Pax6, Musashi-1, Sox2 and Msx1 was higher in P4 CSC cultured in NSCM **(****FIG. 29C****,** * p<0.1, ** p<0.05), but the expression of p75^{NTR}, Nestin, Msx1, and FoxD3 transcripts was downregulated when P4 CSC were cultured in DMEM/10%FBS **(****FIG. 29C****,** * p<0.1, ** p< 0.05).

Immunofluorescence staining confirmed the universal expression of vimentin by these mesenchymal cells. However, nuclear staining of Pax6 was noted in P4 LNC while cytoplasmic staining of Pax6 was predominantly noted in P4 CSC when both cultured on coated Matrigel^{™} in MESCM **(****FIG. 29D****).** In addition, P4 LNC expressed nuclear expression of p75^{NTR}, Musashi-1, Sox2, and Sox10 and strong cytoplasmic expression of Nestin. In contrast, the CSC counterpart expressed weak or absent with the nuclear staining of Pax6, p75^{NTR}, Musashi-1, and Sox2 and exhibited weak cytoplasmic staining of Nestin **(****FIG. 29D****).** After confirming the specificity of the antibody to recognize 46 kDa Pax6 protein in the positive control of ARPE-19 cell lysate as previously reported, it was then demonstrated by western blot analysis that 46 kDa Pax6 protein was prominently expressed by P4 LNC more so than P4 CSC **(****FIG. 29E****).** These results collectively suggested that 46 kDa Pax6 contributed to the nuclear Pax6 staining of P4 LNC and correlated with high expressions of other neural crest markers.

### Nuclear Pax6 in LNC was Lost After Serial Passage

It has previously been reported that P4 LNC exhibit vascular angiogenic potential to differentiate into vascular endothelial cells or pericytes capable of stabilizing vascular tube formation and more potent potential than human bone marrow-derived mesenchymal stem cells to differentiate into osteoblasts, chondrocytes, and adipocytes. To know whether serial passages might affect the aforementioned nuclear localization of Pax6 and expression of the aforementioned neural crest markers in LNC, LNC was isolated from four different limbal quadrants (labeled as A - D) and CSC from the central cornea (labeled as E) of the same donor tissue **(****FIG. 30A****)** and serially expanded on coated Matrigel^{™} in MESCM. Both LNC and CSC exhibited similar spindle cells at P4 and gradual cell enlargement at P10 **(****FIG. 30B****).** LNC from Region A (i.e., the superior limbus) reached P13 with 20.2 cumulative cell doublings, LNC from Regions B - D reached P8 - P9 with an average of 10.9 ± 1.9 cumulative cell doublings, while CSC reached P8 with 9.6 cell doublings **(****FIG. 30C****).** LNC expanded after P2 did not express transcripts of such epithelial markers as cytokeratin 12 (CK12) and cytokeratin 15 (CK15). Transcript expression of pericyte markers such as α-SMA, PDGFRβ, and mesenchymal stem cell markers such as CD105 was higher at P4 **(****FIG. 30D****).** It was further noted the continuous expression of FLK-1 (VEGFR2), CD31, and CD73 by serial passage **(****FIG. 30D****,** ** p<0.01, n=3). Compared to the expression level at P4, serial passages reduced expression of Pax6, p75^{NTR}, Musashi-1, Sox2, Nestin, FoxD3 and Msx1 in LNC isolated from Region A **(****FIG. 30E****,** ** p<0.01, n=3) and Region B.

Immunofluorescence staining further showed that nuclear staining of Pax6 by P4 LNC and became nearly nil staining by P10 LNC; nuclear staining of p75^{NTR} and Sox2 at P4 was lost in P10 LNC **(****FIG. 30F****).** Cytoplasmic and nuclear staining of Musashi-1 and Nestin at P4 was reduced at P10 when cell enlargement was noted **(****FIG. 30F****).** Western Blot analysis confirmed that 46 kDa Pax6 protein was prominently expressed by P4 LNC and nearly nil in P10 LNC **(****FIG. 29E****).** The percentage of nuclear Pax6 (+) LNC in Region A showed a progressive decline by serial passages **(****FIG. 30G****).** These data collectively indicated that serial passage of LNC on coated Matrigel^{™} in MESCM resulted in the progressive loss of nuclear Pax6, which was accompanied by decreased expression of neural crest markers and increased expression of angiogenesis and MSC markers.

### Neural Potential in LNC Declined by Serial Passage

*In vitro* neurosphere growth assay is gold standard for neural stem cells. Because serial passage of LNC led to reduced expression and loss of nuclear Pax6 staining and other neural crest markers, it was wondered whether such a change was correlated with the loss of the neural progenitor status defined by neurosphere formation and neuroglial differentiation potential. LNC from 4 regions and CSC were serially passaged and seeded at the same density of 5x10³/cm² in poly-HEMA coated 12-well in the neurosphere medium containing 1.6% of methylcellulose for 7 days. Spheres emerged with an increasing size **(****FIG. 31A****,** representative P4 and P10 LNC from Region A). Live and dead assay showed these spheres from P4 LNC on day 6 were alive as shown by positive calcein-AM staining and negative ethidium homodimer staining **(****FIG. 31B****).** The counting of spheres with a size of greater than 50 µm in diameter at day 6 showed that CSC yielded a very low sphere-forming efficiency, i.e., 0.3 ± 0.1%, between P2 to P8 (Fig. 3C). In contrast, P2 LNC from all 4 regions had a significant higher efficiency of 2.9 ± 0.5% (^{##}p=0.0006, n=3) with Region A being significantly higher than other 3 regions **(****FIG. 31C****,** ** p=0.003, n=3). For all limbal regions, the sphere-forming efficiency declined after serial passage and reached 0.8 ± 0.4% by P10 **(****FIG. 31C****).** P4 LNC neurospheres expressed a significantly higher transcript level of p75^{NTR} and Musashi-1 than P4 CSC neurospheres **(****FIG. 31D****,** ** p=0.001, n=3). P4 CSC neurospheres expressed significantly lower levels of p75^{NTR} and Musashi-1 (Fig. 3D, ^{#} p=0.001, n=3) but higher levels of Nestin and Msx1 **(****FIG. 31D****,** ^{#} p=0.001, n=3) than P4 CSC cultured on coated Matrigel^{™} as the control. Immunofluorescence staining confirmed the positive nuclear Pax6 staining and cytoplasmic and nuclear staining of Musashi-1 in P4 LNC neurospheres but weak cytoplasmic staining of Pax6 and negative expression of Musashi-1 in P4 CSC neurospheres, and no difference in the staining pattern of Nestin **(****FIG. 31E****).** P4 LNC cultured on coated Matrigel^{™} could be differentiated into neurons with expression of neurofilament M (NFM, red) and β-III tubulin (green), oligodendrocytes with expression of O4, and astrocytes with expression of glial fibrillary acidic protein (GFAP) **(****FIG. 31F****).** As a comparison, P10 LNC could not differentiate into astrocytes although they were still able to adopt differentiation into neurons and oligodendrocytes with larger cells **(****FIG. 31F****).** These results collectively supported the notion that serial passage of LNC resulted in the loss of the neural crest progenitor status as evidenced by reduced neurosphere formation and neuroglial differentiation potential.

### Forced Expression of Pax6 Restored Neural Crest Progenitor Status in P10 LNC

Forced expression of Pax6 was carried out in P10 LNC, which exhibited a gradual loss of transcript expression of Oct4, Sox2, Nanog, and Rex14 and the loss of nuclear Pax6 staining as well as expression of neural crest markers. The optimal transfection efficiency of the adenoviral plasmid construct with CMV promoter and enhanced green fluorescent protein (GFP) with or without Pax6, i.e., Ad-GFP-Pax6 (experimental) and Ad-GFP (control) **(****FIG. 32A****)** was confirmed to be at the multiplicity of infection (MOI) of 100 **(****FIG. 32B****,** * p<0.1 and ** p<0.05, n=3). P10 LNC transfected by GFP-Pax6 upregulated transcript expression of ESC markers (Oct4, Sox2, Nanog) and neural crest markers (p75^{NTR}, Musashi-1, and FoxD3) when compared to cells transfected by GFP **(****FIG. 32C****,** ** p<0.05, n=3). Western blot analysis showed overexpression in P10 LNC enhanced the intensity of 46 kDa Pax6 band **(****FIG. 32D****).** Following the overexpression of 46 kDa Pax6, there was upregulation of Oct4 (39 kDa), p75^{NTR} (30 kDa), and Musashi-1 (39 kDa) proteins **(****FIG. 32D****).** Immunofluorescence staining confirmed nuclear Pax6 staining in P10 LNC transfected by GFP-Pax6 but not GFP **(****FIG. 32E****).** Nuclear Pax6 staining was co-localized with enhanced nuclear staining of Oct4 and Sox2 **(****FIG. 32E****).** In addition, forced expression of Pax6 also resulted in enhanced nuclear and cytoplasmic expression of p75^{NTR} and nuclear expression of Musashi-1 **(****FIG. 32E****).**

Neurosphere formation **(****FIG. 33A****)** and neurosphere-forming efficiency **(****FIG. 33B****,** *p=0.001, n=3) were also significantly promoted in P10 LNC transfected by GFP-Pax6 when compared to cells transfected by GFP. Furthermore, cell morphology was reduced in size in P10 LNC transfected by GFP-Pax6 during their respective differentiation into neuronal, astrocytes and oligodendrocytes **(****FIG. 33C****).** The loss of differentiation potential into astrocyte by P10 LNC **(****FIG. 31F****)** was restored after transfection with GFP-Pax6, which also promoted the potential to differentiate into neurons with strong expression of NFM and oligodendrocytes with expression of O₄ **(****FIG. 33C****).** These data collectively indicated a strong causal relationship between the nuclear localization of Pax6 and the restoration of the neural crest progenitor status.

### P10 LNC with Forced Expression of Pax6 Supported Self-Renewal of LEPC

Reunion of single LEPC with single P4 LNC or P4 LNC aggregates in 3D Matrigel^{™} prevented corneal fate decision/differentiation of limbal epithelial progenitor cells (LEPC). Furthermore, corneal fate decision is prevented more by reunion between LEPC and P4 LNC than that between LEPC and P4 CSC4. The same experiment was repeated and noted that reunion between LEPC and P4 LNC generated similar cell aggregates **(****FIG. 34A****)** but with higher expression of ΔNp63α and reduced expression of CK12 when compared to that between LEPC and P4 CSC **(****FIGS. 34B-34C****).** Under the same condition, reunion of LEPC with P10 LNC did not alter the transcript expression but promoted expression of CK12 protein when compared to that with P4 LNC **(****FIGS. 34B-34C****),** suggesting that loss of nuclear Pax6 staining in P10 LNC was associated with the outcome favorable of driving LEPC toward more corneal fate decision. In contrast, compared to that with P10 LNC, reunion with P10 LNC with forced expression of Pax6 significantly higher transcript expression of Bmi1 but downregulated CK12 transcript and protein **(****FIGS. 34B-34C****),** suggesting that gain of Pax6 expression in LNC was linked to suppression of corneal fate decision in LEPC.

In an *in vitro* colony forming assay on mitomycin-treated 3T3 fibroblast feeder layers, reunion between LEPC and P4 LNC on 3D Matrigel^{™} yielded greater clonal growth of holoclone **(****FIG. 34D****).** Herein, it was noted that the colony-forming efficiency (CFE) of holoclone was significantly promoted when reunion of LEPC was made with P4 LNC when compared to LEPC alone or with P4 CSC **(****FIG. 34E****,** *p = 0.02) when the same number of PCK+ cells were seeded. Compared to reunion between LEPC and P4 LNC, the CFE of holoclone was significantly reduced in reunion between LEPC and P10 LNC-GFP **(****FIG. 34E****,** *p = 0.02), suggesting that late passage LNC, which lost nuclear Pax6 staining, did not support clonal growth of LEPC as potent as P4 LNC, which maintained nuclear Pax6 staining. In contrast, the holoclone CFE was significantly promoted in reunion between LEPC and P10 LNC with forced expression of Pax6 when compared to that between LEPC and P10 LNC GFP **(****FIG. 34E****,** ** p = 0.0001), suggesting that nuclear Pax6 staining endowed P10 LNC with a capacity of supporting clonal growth by LEPC. Further characterization of the resultant holoclone by immunofluorescence staining revealed nuclear p63α+ holoclone in LEPC no matter if they were reunioned with P4 CSC, P4 LNC, or P10 LNC with or without forced expression of Pax6 **(****FIG. 34F****).** However, nuclear Pax6+ LEPCs were noted in holoclone formed following reunion with P4 CSC, both nuclear Pax6+ and Pax6- LEPCs were noted in holoclone formed following reunion with P4 LNC and P10 LNC GFP, while nuclear Pax6- LEPCs were noted in holoclone formed following reunion with P10 LNC GFP-Pax6 **(****FIG. 34F****).** CK12+ basal and suprabasal LEPCs were noted in holoclone generated following reunion with P4 CSC and P10 LNC GFP, CK12+ basal LEPCs were noted in holoclone generated following reunion with P4 LNC, while CK12- basal LEPCs were noted in holoclone generated following reunion with P10 LNC GFP-Pax6 **(****FIG. 34F****).** Collectively, these findings strongly suggested that overexpression of Pax6 in P10 LNC prevented corneal fate decision and promoted holoclone formation by LEPC in 3D Matrigel^{™}.

### Discussion

During eye morphogenesis, Pax6-expressing cranial neural crest cells are involved in the formation of lens placodes, retina, and anterior segment. During eye development, nuclear Pax6+ staining is observed in corneal stroma, ciliary body, endothelial and trabecular meshwork. Herein, it was found nuclear Pax6+ staining in freshly isolated **(****FIG. 29A****)** and early passaged (P4) of LNC **(****FIG. 30F****),** but not in their corneal counterpart, i.e., P4 CSC, which exhibited weak cytoplasmic Pax6 staining **(****FIG. 29D****).** Western blot analysis confirmed that it was 46 kDa Pax6 responsible for the nuclear Pax6 staining of P4 LNC **(****FIG. 29E****).** Because such a phenotype was correlated with higher expression of ESC markers such as Oct4, Sox2 and many other neural crest markers such as p75^{NTR}, Musashi-1, Sox2, Nestin, Msx1 and FoxD3 **(****FIG. 30E****),** neurosphere formation **(****FIGS. 31A-31E****)** and differentiation potential into neuroglial lineages **(****FIG. 31F****),** nuclear staining with 46 kDa Pax6 in LNC may be used as a hallmark to signify the neural crest progenitor status. The role of Pax6 in neuronal differentiation has also been reported by others. The strong nuclear Pax6+ staining has also been noted in radial glia cells of the ventricular (germinal) zone housing neural stem/progenitor cells. Pax6-haploinsufficiency leads to reduced production of neural stem/progenitors in adult hippocampus rat. Non-viral plasmid transfection of Pax6 and Sox2 in adult human fibroblast direct reprogram cells to a neural precursor cell-like state. The Pax6 -Brg1/BAF complex is essential and sufficient to convert glia into neuron in the adult mouse olfactory bulb. Hence, a gradual loss of nuclear Pax6 staining in LNC during serial passage might contribute to the gradual loss of the expression of neural crest markers and reduction of neurosphere formation and neuroglial differentiation potential **(****FIGS. 31A-31F****).** Interestingly, such gradual loss of neural crest potential during serial passage was correlated with an increase expression of angiogenesis and MSC markers, suggesting that LNC have the plasticity of undergoing both neuronal and vascular differentiation potentials, a notion that has also been noted in adult mammalian neural crest derived carotid body. Future studies are needed to see if LNC can be ascribed an important role in partaking in regenerative wound healing, which requires restoration of both neural and vascular tissue components.

The critical role of Pax6 in governing the neural crest progenitor status was further substantiated by forced expression of 46 kDa Pax6 in late passaged LNC. Gain of function by forced expression with adenoviral vector GFP-Pax6 resulted in the reappearance of nuclear 46 kDa Pax6 staining in P10 LNC and re-expression of neural crest markers **(****FIGS. 32C-32E****)** and increased neurosphere formation and neuroglial differentiation potential **(****FIGS. 33A-33C****).** Expression of ESC markers such as Oct4, Sox2, Nanog and Rex1, which are noted in freshly isolated LNC, is also gradually lost during serial passage. Herein, it was noted that forced expression of Pax6 in P10 LNC helped regain expression of Oct4 and Sox2 and neural crest markers **(****FIGS. 32C-32E****).** Chromatin immunoprecipitation chip sequencing study reveals that Pax6 targets to several gene promotors in neural progenitor cells. Pax6 binds directly to pluripotent genes, Oct4 and Nanog to repress their expression and to promote neural neuroectoderm genes in human ES cells, and cooperates with Sox2 to ensure the unidirectional lineage commitment toward neuronal differentiation in radial glial cells. Therefore, it is plausible that nuclear localization of Pax6 might help to reinforce the nuclear Oct4, Sox2, and Nanog to ensure the neural crest progenitor status in LNC.

For the post-natal corneal and limbal epithelia, Pax6 together with p63 specifies limbal epithelial SCs from the surface ectoderm and with Wnt7A controls corneal fate decision by promoting CK12 expression by limbal and corneal epithelial cells. To demonstrate the important role of Pax6 in LNC to modulate self-renewal of limbal epithelial SCs, an *in vitro* colony forming assay was utilized, which is frequently used to measure the self-renewal property of a single SC. For epithelial stem (progenitor) cells, the standard of proof relies on categorizing resultant clones based on morphology and phenotypic characterization as holoclone, meroclone, and paraclone. Only holoclones are capable of performing extensive proliferation and self-renewal, whilst meroclones have a limited proliferative capacity and cannot self-renew and paraclones are incapable of further proliferation. Previously, the aforementioned practice was followed, adopted the same criteria, and reported that the reunion of P4 LNC with limbal epithelial progenitor cells (LEPC) supports self-renewal of the latter in 3D Matrigel^{™} by demonstrating the greater yield of holoclones with nil expression of corneal epithelial differentiation marker, cytokeratin 12, when compare to LEPC alone. Herein, by taking advantage of the success in establishing the *in vitro* reunion assay between LNC and LEPC, which contain limbal epithelial SCs34, P10 LNC, which lost nuclear Pax6 staining **(****FIG. 30F****),** were shown to yield fewer holoclones than P4 LNC **(****FIG. 34E****).** In contrast, reunion between LEPC and P10 LNC with forced expression of Pax6 yielded significantly more holoclones than LEPC alone or reunion between P10 LNC GFP and LEPC **(****FIG. 34E****).** The reunion between LEPC and P4 LNC prevented corneal fate decision as evidenced by suppression of CK12 expression and promoted holoclone formation in LEPC when compared to LEPC alone or LEPC with P4 CSC **(****FIGS. 34B-****34C).** Although transcript expression of epithelial progenitor markers such as Bmi-1 and ΔNp63α and corneal fate maker such as CK12 did not change in LEPC when reunion with P4 LNC or P10 LNC, forced expression of 46 kDa Pax6 in P10 LNC upregulated Bmi-1 transcript and downregulated CK12 transcript and protein **(****FIGS. 34B-34C****),** indicating that Pax6 plays an important role in LNC in preventing LEPC from taking corneal fate decision. This finding was accompanied by an increase of CFE of holoclone **(****FIG. 34E****),** in which the basal epithelial monolayer uniquely exhibited small uniform nuclear p63 α + staining, Pax6- nuclear staining, and negative CK12 **(****FIG. 34F****).**

Based on the studies, Pax6 plays an important role in LNC to support self-renewal of limbal epithelial SCs. The finding that LNC from the superior limbus, i.e., Region A **(****FIG. 30A****),** maintained the longest passage number with the highest nuclear Pax6+ staining and exhibited greatest neurosphere formation also supports the general belief that superior limbus contains the most prominent limbal palisade of Vogt, which specifies the limbal SC niche.

### Materials and Methods

### Cell Isolation and Expansion

Human corneolimbal rim and central cornea button stored at 4°C in Optisol (Chiron Vision, Irvine, CA) for less than 7 days were obtained from different donors (Florida Lions Eye Bank, Miami, FL). After rinsing three times with PBS pH 7.4 containing 50 µg/ml gentamicin and 1.25 µg/ml amphotericin B, the excess sclera, conjunctiva, iris, corneal endothelium and trabecular meshwork were removed up to the Schwalbe's line for the corneoscleral rim before being cut into superior, nasal, inferior, and temporal quadrants **(****FIG. 30A****,** denoted as region A to D) at 1 mm within and beyond the anatomic limbus. An intact epithelial sheet including basal epithelial cells was obtained by subjecting each limbal quadrant to digestion with 10 mg/ml dispase in modified embryonic stem cell medium (MESCM), which was made of Dulbecco's Modified Eagle's Medium (DMEM)/F-12 nutrient mixture (F-12) (1:1) supplemented with 10% knockout serum, 10 ng/ml LIF, 4 ng/ml bFGF, 5 mg/ml insulin, 5 mg/ml transferrin, 5 ng/ml sodium selenite supplement (ITS), 50 µg/ml gentamicin and 1.25 µg/ml amphotericin B in plastic dishes containing at 4°C for 16 h under humidified 5% CO₂ incubator. LNC were isolated by digestion with 2 mg/ml collagenase A at 37°C for 16 h to generate floating clusters. CSC were isolated in the same manner except that the overlying epithelium from the central cornea **(****FIG. 30A****,** denoted as region E) was digested with 10 mg/ml of dispase II at 37°C for 2 h in MESCM to remove epithelial sheets first.

For expansion, single cells derived from limbal clusters or CSC after digestion with 0.25% trypsin and 1mM EDTA (T/E) were seeded at 1x10⁴/cm² in the 6-well plate pre-coated with 5% Matrigel^{™} in MESCM and cultured in humidified 5% CO₂ with media change every 3-4 days for total 6-7 days. In some instance, CSC were expanded in Neural Stem Cells Serum-Free Expansion Medium (NSCM) consist of DMEM/F-12 (1:1) supplemented, 2% Neural Supplement (consist of B-27 and N-2), 20 ng/ml human FGF-basic recombinant, 20 ng/ml human EGF recombinant. CSC were also expanded on plastic in DMEM with 10% FBS, 50 µg/ml gentamicin and 1.25 µg/ml amphotericin B. When cells reach at 80-90% confluence and were serially expanded at the seeding density of 5x10³ per cm² for up to 13 passages. The extent of total expansion was measured by the number of cell doubling (NCD) calculate from formulate: NCD= log10(y/x)/log10², where "y" is the final density of the cells and "x" is the initial seeding density of the cells.

### In vitro reunion assay

An *in vitro* reunion assay was performed. In brief, P4 LNC, P4 CSC, and P10 LNC transfected with Ad-GFP or Ad-GFP-Pax6 that were expanded on coated Matrigel^{™} were seeded in 3D Matrigel^{™} at the density of 5 × 10⁴ cells/cm² to generate aggregates in MESCM for 24 h. Single LEPC obtained from dispase-isolated limbal epithelial sheet were seeded at the density of 5 × 10⁴ cells/cm² in 3D Matrigel^{™} with or without the aforementioned LNC or CSC aggregates for 6 days. The resultant spheres were harvested by digesting Matrigel^{™} with 10 mg/ml dispase II at 37°C for 2 h, of which some were rendered into single cells by T/E before being prepared for cytospin.

### In vitro colony forming assay

An *in vitro* epithelial colony forming assay was performed on mitomycin-treated 3T3 fibroblast feeder layers in supplemental hormonal epithelial medium (SHEM), which was made of an equal volume of HEPES-buffered DMEM and Ham's F-12 containing bicarbonate, 0.5% dimethyl sulfoxide, 2 ng/ml mouse-derived epidermal growth factor, 5 mg/ml insulin, 5 mg/ml transferrin, 5 ng/ml sodium selenite, 0.5 mg/ml hydrocortisone, 30 ng/ml cholera toxin A subunit, 5% fetal bovine serum (FBS), 50 mg/ml gentamicin, and 1.25 mg/ml amphotericin B. In brief, a total 2,000 single cells obtained from LEPC with or without reunion with P4 LNC, P4 CSC, and P10 LNC transfected with GFP or GFP-Pax6 were seeded on MMC-treated 3T3 fibroblast feeder layers for 10 days. The resultant clonal growth was fixed in 4% paraformaldehyde and assessed by 1% rhodamine B staining solution for marking clones for the measurement of colony-forming efficiency by calculating the percentage of the clone number divided by the total number of PCK+ cells seeded that was determined by double immunostaining of PCK/Vim. Clone morphology was subdivided into holoclone, meroclone, and paraclone based on the criteria established for skin keratinocytes49.

### Forced expression of GFP-Pax6

The forced expression experiment was performed in P10 LNC on coated Matrigel^{™} in MESCM by adding Ad-GFP-Pax6, which is pre-packaged human adenovirus Type-5 vector (dE1/E3) expressing human enhanced GFP-Pax6 construct gene (NCBI reference sequence of Pax6 is BC011953) under the control of the cytomegalovirus (CMV) promoter or Ad-GFP, which is the empty vector with GFP promoter (both purchased from Vector Biolabs, Malvern, PA), at the MOI of 0, 4, 20, 100, 500 and 2500 for 1 to 5 days.

### Neurosphere formation

Single cells of both LNC or CSC expanded at different passages were plated at cell density of 5000/cm² on anti-adhesive poly-HEMA in 12 well-plate for 6 days in neural stem cell medium (NSCM) consisting of 20 ng/ml EGF, 20 ng/ml FGF2, 2% NSCM supplement, and 1.6% methylcellulose. Sphere formation was monitored by phase microscope and spheres with the size of greater than 50 µm in diameter were counted throughout the entire 12-well on day 6 by Zeiss Axio-Observer Z1 Motorized Inverted Microscope (Carl Zeiss, Thornwood, NY). The neurosphere-forming efficiency was calculated by subdividing the total number of spheres by the total number of seeded cells x 100%.

### Neuroglial differentiation

1x10⁴/ml of P4 or P10 LNC were seeded on 50 µg /ml poly-L-ornithine and 20 µg/ml laminin-coated or Collagen Type IV coated cover glass in 48-well plate in NSCM supplement with 0.5% N2 and 1% B27 for 2 days. For neuronal differentiation, medium was then replaced to neuronal induction base medium containing DMEM/F12 (1:3) with 0.5% N2 and 1% B27 in additional to 10 ng/ml FGF2 and 20 ng/ml of BDNF (medium A) for 3 days and replaced with base medium in addition to 6.7 ng/ml FGF2 and 30 ng/ml of BDNF for another 3 days. Cell then replaced to base medium in addition to 2.5 ng/ml FGF2, 30 ng/ml BDNF, and 200 mM ascorbic acid for another 8 days. For oligodendrocyte differentiation, medium then replaced with base medium containing DMEM/F12 (1:1) with 1% N2 in addition to 10 ng/ml FGF2, 10 ng/ml PDGF, and 10 µM forskolin for 4 days and then medium was replaced by the base medium in addition to 10 ng/ml FGF2, 30 ng/ml 3,3,5-triiodothyronine, and 200 µM ascorbic acid for another 7 days. For astrocyte differentiation (Thermo Scientific, Santa Clara, CA), medium was replaced by DMEM containing 1% FBS, 1% N2, and 2mM GlutaMax for 10 days. Induction media were changed every 3-4 days.

### RNA extraction, reverse transcription, and quantitative real-time PCR

Total RNAs were extracted from expanded LNC, CSC, or neurospheres on day 6 by RNeasy Mini Kit (Qiagen, Valencia, CA) according to manufacturer's guideline and 1-2 µg of RNA extract was reverse transcribed to cDNA with reverse-transcribed using High Capacity Reverse Transcription Kit (Applied Biosystems, Foster City, CA) using primers. The resultant cDNAs were amplified by specific TaqMan gene expression assay mix and universal PCR master mix in 7300 Real Time PCR System (Applied Biosystems, Foster City, CA) with real-time RT-PCR profile consisting of 10 min of initial activation at 95°C, followed by 40 cycles of 15 sec denaturation at 95°C, and 1 min annealing and extension at 60°C. The relative gene expression data were analyzed by the comparative CT method (ΔΔCT). All assays were performed in triplicate. The results were normalized by glyceraldehyde 3-phosphate dehydrogenase (GAPDH) as an internal control.

### Immunofluorescence staining

Single cells of LNC or CSC at different passages and their neurospheres with or without knockdown by forced expression of Pax6 were harvested with 0.05% trypsin and 1mM EDTA at 37° C for 10 min and prepared for cytospin using Cytofuge (StatSpin Inc., Norwood, MA) at 1000 rpm for 8 min. Cells were fixed with 4% formaldehyde, pH 7.0, for 15 min at room temperature, permeabilized with 0.2% Triton X-100 in PBS for 15 min and blocked with 2% bovine serum albumin (BSA) for 1 h before incubated with primary antibodies for 16 h at 4°C. After 3 washes with PBS, the corresponding Alexa Fluor-conjugated secondary IgG (all 1: 100 dilution) were incubated for 60 min and 3 washing with PBS. The method to calculate the % nuclear Pax6 positive cells was based on counting of nuclear Pax6 positive cells using AxioVision software (Carl Zeiss, Thornwood, NY) of immunofluorescence staining images with Pax6 staining and Hoechst 33342 counter nuclear staining taken by confocal microscopy. Corresponding mouse and rabbit sera were used as negative controls for the primary monoclonal and polyclonal antibodies, respectively. Neurospheres were also incubated in NSCM containing 4 µM of EthD-1 and 2 µM of Calcein AM at 37°C for 30 min for fluorescence detected at 494/517 nm for viable and 528/617 nm for dead cells, respectively under the confocal microscope.

### Western blot

Cell lysates were extracted from P10 LNC transfected with Ad-Pax6 GFP or Ad-GFP on day 4 by cold lysis buffer containing radioimmunoprecipitation assay buffer, protease inhibitor cocktail (100x) and 1mM phenylmethylsulfonyl fluoride.(Sigma-Aldrich, St. Louis, MO) Total protein of the cell lysate was measured and normalized by the BCA assay (Pierce, Rockford, IL) and 5 µg of protein lysate was loaded on a 4-15% (w/v) gradient sodium dodecyl sulfate-polyacrylamide gel and transferred to nitrocellulose membrane using mini Trans-blot electrophoretic transfer apparatus (Bio-Rad, Hercules, CA). Each membrane was blocked with 5% (W/V) fat-free milk in 50 mM Tris-HCl, pH 7.5, containing 150 mM NaCl, and 0.05% Tween-20 for 1 h before incubation with specific primary antibodies in 5% (W/V) fat-free milk overnight at 4°C follow by their respective horseradish peroxidase-conjugated secondary antibodies using antibody against Histone 3 and β-actin as the loading control. The immunoreactive bands were detected by Western Lightning Chemiluminescence (PerkinElmer, Waltham, MA) using an ImageQuant LAS 4000 digital imaging system (GE Healthcare Piscataway, NJ).

### Statistical analysis

All summary data were reported as mean ± SD. Significance was calculated for each group and compared with two-tailed Student's t-test and ANOVA by Microsoft Excel (Microsoft, Redmond, WA). Test results were reported as p values, where p < 0.05 were considered statistically significant.

### Example 8: Nuclear translocation of CXCR4 in cells

Endogenous CXCR4 found in cytoplasmic and nucleus of young fetal blood and bone marrow mesenchymal stem cells (MSC) was compared to plasma membrane expressing CXCR4 in adult MSC. Internalization of CXCR4 has been noted to interact with other proteins, such as ferritin, heat shock cognate protein (Hsc73), plectin, and Myosin IIA after SDF-1 treatment. Interestingly, the internalization of endogenous CXCR4 has reported specifically regulated by Rac1 via extracellular domain 2 (ECL2) that control conformational heterogeneity of CXCR4. Inhibition of Rac1 by inhibitors NSC23766 or EHT1864 leads the reduced cell surface CXCR4. Different CXCR4 antibodies against this domain can differentiate conformation changes thus affecting coreceptor efficiency on the cell surface. These data use an antibody against CXCR4 (Clone 44716.111), which is known to specifically recognize this ECL2 domain and was found translocated to nucleus at 15 min. A previous observation showed that transient activation of Rac1 at 5 and 15 min but reduced at 30 min by soluble HC-HA/PTX3, in contrast to a gradual decline of Rac1 GTPase activities by HA **(****FIGS. 37A-37C****).** This may suggest that internalization of CXCR4 to nucleus is correlated to the reduction of RAC1 at 30 min

### Example 9: Determination of whether reversal of Pax P10 LNC neural crest progenitors promoted by HC-HA/PTX3 LNC can maintain self-renewal of limbal epithelial progenitor/stem cells on 3D Matrigel (MG)

Previously it has been shown that in an *in vitro* reunion assay between limbal epithelial progenitor cells (LEPC) and P4 LNC maintains the self-renewal status and prevent corneal SC epithelial from differentiation in 3D Matrigel and promoted their clonogenic potential on mitomycin C-arrested 3T3 fibroblast feeder layers. Both immobilized and soluble HC-HA/PTX3 have been demonstrated to reverse P10 LNC with neural crest phenotype at 48 h and CXCR4 mediated signaling is necessary to promote Pax6 P10 LNC. In this example, it was asked whether the reversed Pax6 P10 LNC can support self-renewal of LEPC on 3D MG.

### Experimental Design

The epithelial progenitor status of the sphere growth was determined by a clonal assay on 3T3 fibroblast feeder layers in supplemental hormonal epithelial medium, which was made of an equal volume of HEPES-buffered DMEM and Ham's F-12 containing bicarbonate, 0.5% dimethyl sulfoxide, 2 ng/ml mouse-derived epidermal growth factor, 5 mg/ml insulin, 5 mg/ml transferrin, 5 ng/ml sodium selenite, 0.5 mg/ml hydrocortisone, 30 ng/ml cholera toxin A subunit, 5% fetal bovine serum (FBS), 50 mg/ml gentamicin, and 1.25 mg/ml amphotericin B. The feeder layer was prepared by treating 80% sub confluent 3T3 fibroblasts with 4 mg/ml mitomycinC (MMC) at 37C for 2 hours in DMEM containing 10% newborn calf serum before seeding at the density of 2 x 104 cells per square centimeter.

P10 and P4 LNC were pre-treated with or without immobilized HC-HA/PTX3 or soluble HC-HA/PTX3 for 48h. 5x10⁴ /cm² treated LNC were reunion with 5x10⁴ /cm² LEPC on 3D MG, sphere growth was harvested on day 6 by 10 mg/ml of dispase 37C for 2h. Harvested spheres were subjected for qPCR and colony forming assay.

For colony forming assay, 500 LEPC or reunion 1,000 single cells sphere growth were seeded on MMC-treated 3T3 fibroblast feeder layers for another 8-10 days. Resultant clonal growth was assessed by 1% rhodamine B staining, which allowed measurement of the colony-forming efficiency by calculating the percentage of the clone number divided by the total number of PCK cells initial seeding with double immunostaining with PCK and Vimentin. Clone morphology was subdivided into holoclone, meroclone, and paraclone based on the criteria established for skin keratinocytes.

### Results

Findings in cross-sectioned human corneoscleral rims demonstrated that strong membrane Notch 1 and Notch 2 receptors were predominantly expressed in corneal and conjunctiva epithelia but absent in limbal basal epithelium. The data further suggested antibody against NICD staining was predominantly found in nuclei of suprabasal corneal and conjunctival epithelium but weakly expressed in nuclei of limbal suprabasal epithelium and absent in the limbal basal epithelium further suggest that NICD-Notch signaling was inhibited in limbal basal epithelium **(****FIG. 38A****).** Furthermore, it was further found Notch3, Jagged1, and Hes1 were strongly expressed in limbal basal epithelium and its subjacent mesenchymal cells. **(****FIGS. 38A-****38B).** Consistently, collagenase isolated limbal cluster revealed weak nuclear NICD expressed PCK+ cells. Interestingly, PCK-negative population (non-epithelial) contained mixture of nuclear NICD(+) cells (non-circled arrows) and NICD(-) cells **(****FIG. 38B****,** circled arrows). These data collectively suggests that Notch3/Jagged1 may play differential role from Notch1 in limbal epithelium and mesenchymal cells.

Recently, it has been reported that P10 LNC on HC-HA/PTX3 promotes cell aggregation and nuclear Pax6 with neuro crest phenotypes and neural crest potential. These preliminary data demonstrated that (5) P4 LNC on immobilized HC-HA/PTX3, but not 3D Matrigel, upregulated transcript expression of Notch2/3, Notch ligand, Jagged1, Dll, and Hes1 signaling **(****FIG. 39A****).** It is unclear the unique upregulation of Notch3 promotes by HC-HA/PTX3 promotes LNC into lineage negative neuroepithelium that has not yet committed to epithelium (p63-).

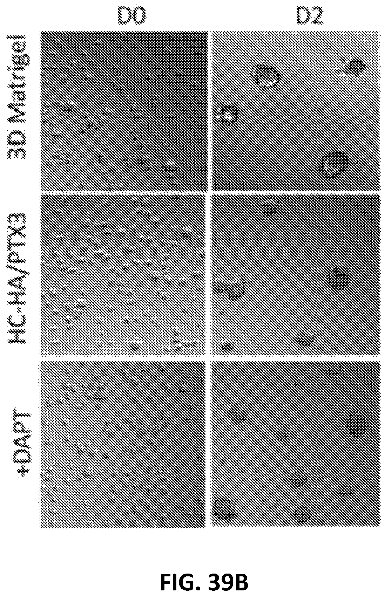

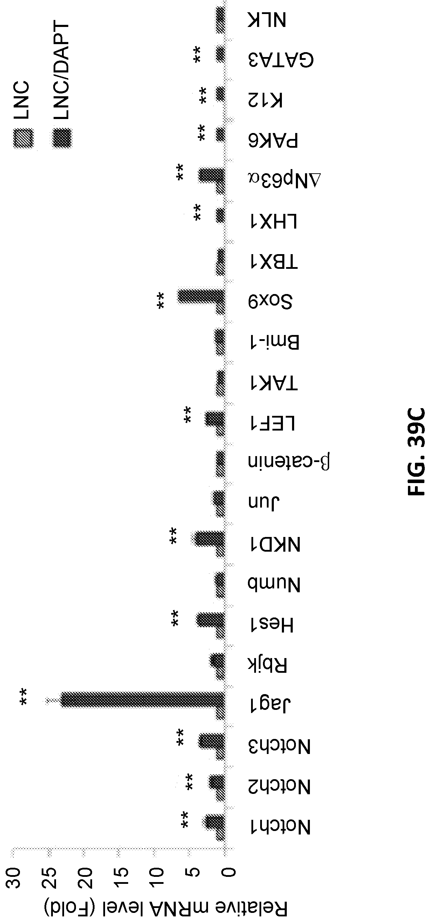

Blocking notch signaling by DAPT did not prevent cell aggregation **(****FIG. 39B****)** but further promoted Notch1/2/3/Jagged 1/Hes1 signaling with MET epithelial phenotype (p63a, Pax6, Sox9) **(****FIG. 39C****),** suggesting inhibition of α-secretase that blocks the canonical notch signaling in LNC on HC-HA/PTX3 actually promotes the aforementioned gene expression. If such upregulation is correlates with notch signaling, i.e., nuclear Hes1, notch signaling can be promoted by non-canonical Notch signaling **(****FIGS. 39A-39E****).** It was demonstrated in Western blot that HCF on HC-HA/PTX3/4P or 4G promotes E-cadherin **(****FIG. 39E****),** suggesting that HC-HA/PTX3 may be also promote MET in LNC. If so, it is plausible that such MET is mediated by Jagg1-notch3 signaling, which may not be suppressed by canonical notch signaling. It remains unclear whether the Notch 3/Jagged 1 is required to maintain the abovementioned signaling.

When two cell types, LEPC and LNC, were compared on immobilized HC-HA/PTX3, LEPC alone expressed Notch1, DLL1, Jag2, LFNG and MFNG whereas LNC alone expressed Notch2, Notch3 and Jagged 1 **(****FIG. 40A****).** This data is consistent to the notch ligands and receptors expression in **FIG. 39A****.** When LEPC was co-cultured with LNC on immobilized HC-HA/PTX3, the transcript of Notch2/3 were further promoted suggesting co-culture of LEPC+LNC were reinforced expression of Notch2/3.

Previous it was shown that co-cultured of LNC+LEPC on HC-HA/PTX3 promotes BMP and PCP signaling and quiescence markers, Bmi-1 of LEPC. The mechanism of how BMP and PCP signaling were activated remains unclear. These preliminary data demonstrated when blocking Notch signaling by DAPT in LEPC+LNC significantly downregulated the quiescence epithelium markers **(****FIG. 41A****)** and led to absence of nuclear psmad/1/5/8 and c-Jun. Because DAPT also inhibit other Notch receptors, Notch3 specific inhibitors is required to verify such finding warrant that BMP and c-Jun requires Notch3/Jagged1 specific signaling for SC quiescence.

### Example 10: In vivo expression of Notch signaling in human cornea, limbus, and conjunctiva

In the cornea, Notch signaling has been reported in regulating the maintenance of the corneal transient amplified corneal epithelium (TAC) in fate decision, differentiation and wound healing. Notch 1-/-mouse leads cornea epithelial into hyperproliferative skin-like epidermis. Overexpressed in cornea epithelium-specific K14 NICD transgenic mice promoted corneal epithelial wound healing. Although Notch1/2 receptors have been reported to predominantly expressed at human corneal suprabasal epithelium and absent at limbal basal epithelium, other groups have reported the opposite finding that membrane Notch1 at limbal basal and subjacent suprabasal epithelium. Notch ligands, Delta I, Jagged 1 and Jagged 2 have characterized expressed throughout the entire corneal epithelium. HEY and HES proteins cooperate with each other in suppressing bHLH activator-driven neuronal differentiation and in maintaining the neural stem cell fate. The objective of this example is to confirm and identify whether they are more than one Notch signaling occur between corneal epithelium and subjacent stroma in cornea, limbus and conjunctiva.

### Experimental Design

Human corneoscleral rims for less than 5 days were obtained from the Florida Lions Eye Bank and handled according to the declaration of Helsinki. Briefly, after the rims were rinsed three times PBS with 50 µg/ml gentamicin and 1.25 lg/ml amphotericin B; the iris, trabecular meshwork, and endothelium were removed.

### Results

The results in cross-sectioned human corneoscleral rims demonstrated that strong membrane Notch 1 and Notch 2 receptors are predominantly expressed in corneal and conjunctiva epithelia but absent in limbal basal epithelium. These data further suggests antibody against NICD staining is predominantly found in nuclei of suprabasal corneal and conjunctival epithelium but weakly expressed in nuclei of limbal suprabasal epithelium and absent in the limbal basal epithelium further suggest that NICD-Notch signaling is inhibited in limbal basal epithelium **(****FIG. 38A****).** Furthermore, it was found Notch3, Jagged1, and Hes1 were strongly expressed in limbal basal epithelium and its subjacent mesenchymal cells **(****FIGS. 38A-38B****).**

### Example 11: Notch3/Jagged1/Hes1 expression in basal epithelium and PCK-/Vimentin+/Pax6+ LNC from freshly isolated limbal clusters

Previously it had been demonstrated collagenase A isolated clusters contain limbal epithelial with its subjacent mesenchymal niche. Those niche cells uniquely express neural crest progenitor defined PCK-/Vim+/Pax6+ mesenchymal expressed Sox2, p75^{NTR}, Musashi-1 and Msx1. It was questioned whether expression Notch3/Jagged1/Hes1 are indeed highly expressed in limbus basal epithelial with subjacent stroma when compared to cornea corneal stromal and epithelial cells.

### Experimental Design

Human tissue was handled according to the Declaration of Helsinki. In this study, human corneoscleral rim from donors aged 61years were provided by the Florida Lions Eye Bank. Immediately after the central corneal button had been used for corneal transplantation, they were transferred in Optisol-GS (Bausch & Lomb; www.bausch.com) and transported at 4°C to the laboratory. The rim was then rinsed three times with PBSx1 pH7.4 containing 50 mg/mL gentamicin and 1.25 mg/mL amphotericin B. All materials used for cell culturing. After removal of excessive sclera, conjunctiva, iris, and corneal endothelium, the tissue was cut into 12 one-clock-hour segments, from which a limbal segment was obtained by incisions made at 1mm within and beyond the anatomic limbus. An intact epithelial sheet including basal epithelial cells could be obtained by subjecting each limbal segment to digestion with MESCM. Alternatively, central cornea contains intact epithelial sheet consisted of predominant suprabasal epithelial cells was obtained by dispase digestion at 37°C for 2 h and the remaining stroma was then digested with 1mg/mL collagenase A in MESCM at 37°C for 16 h from the stroma. In parallel, each limbal segment, without any further trimming off any stromal tissue, was directly digested with 1mg/mL collagenase A in SHEM at 37C for 16 h under humidified 5% CO2 to generate a cell aggregate termed "cluster."

### Results

Results are illustrated in **FIG. 38B****.** PCK-negative population (non-epithelial) contained mixture of nuclear NICD(+) cells (white arrows) and NICD(-) cells **(****FIG. 38B****,** circled arrows).

### Example 12: HC-HA/PTX3, but not basement membrane 3D Matrigel, uniquely activated Notch3 in LNC

Consistently, collagenase isolated limbal cluster revealed weak nuclear NICD expressed PCK+ cells. The preliminary data as seen in Example 11 collectively suggested that Notch3/Jagged1 may play differential role from Notch1 in limbal epithelium and mesenchymal cells.

P4 LNC on HC-HA/PTX3, but not on plastic or 3D Matrigel, uniquely promotes Notch signaling by upregulation of notch ligands notch2, notch3, DLL2 and receptors Jagged 1 and DLL2. In contrast, 3D Matrigel uniquely promotes Beta-1,3-N-acetylglucosaminyltransferance manic fringe (MFNG) **(****FIG. 42****).** Addition of LEPC to LNC on HC-HA/PTX3, Notch2 and Notch3 were unique expressed in LNC where the upregulation of notch1, DLL1, Jagged 1, Jagged 2, Lunatic fringe (LFNG) and MFNG are LEPC dependent. Nuclear Bmi-1 in LEPC is expressed in limbus but not cornea or conjunctiva. It remains unclear whether the collagenase isolated cluster express in similar fashion.

### Experimental Design

1x10⁵/ml of P10 LNC were seeded on three substrates, coated Matrigel, HA or HC-HA/PTX3 in MESCM 48 h. For time course study on soluble HC-HA/PTX3, P10 LNC were treated HC-HA/PTX3 for 5, 15, 30, 60 min, 24 h and 48 h.

### Results

Time course revealed HC-HA/PTX3 promoted mRNA expression of Notch3/Jag1 and Hes1 as early as 15 minutes and at peak by thousand-fold at 120 min in P10 LNC when compared to the transcript level on 3D Matrigel **(****FIGS. 18A-18C**,**<0.05, n=3) Immunofluorescence staining confirmed the HC-HA/PTX3 promotes nuclear Hes-1 as early as 5 min but weakly expressed in 3D Matrigel. Expression of Notch1 and notch3 absent in the nucleus within 60 min when antibodies were used that recognized nuclear NICD domain, suggesting the nuclear Hes-1 may be notch mediated through non-canonical Notch signaling.

### Discussion

Activation of Notch signaling has been reported necessary to convert cranial neural crest derived mesenchyme to perivascular cells. Constitutive activation of notch pathway through expression of NICD, in mouse embryonic fibroblast cell line or cranial neural crest mesenchyme were sufficient to promote cells into perivascular cell fate. Activation of ligand binds to Notch triggers shedding of its extracellular domain by a metalloprotease.

Expression of Hes1 has been demonstrated to be mediated through Notch dependent and -independent pathways to promote angiogenesis and neurogenesis. Oscillation of Hes1 has been demonstrated notch independent and mediated through BMP and LIF signaling in ES cells, FGF2-JNK axis in ES derived neural progenitors, NGF-NF-KB with sustained expression of Hes1 to maintain the dendriotogensis, VEGF-FLK-1-ERK for retinal progenitor proliferation and retinal ganglion cell fate specification and acetylation of Pax3 binding the promoter of Hes1 to enhance neural SC maintenance.

Hes1 has been known to regulate the undifferentiated status/maintenance of neural stem cell progenitors to promote proper neuronal differentiation and cell-cell interactive lateral inhibition. Expression of Hes1 often in an oscillatory manner of every 2 hours has been demonstrated in fibroblast and neural progenitors. Without Hes gene, progenitor cells prematurely differentiate into certain types of neurons only and are depleted before they have proliferated sufficiently for other neuronal and glial cell types. These data showed that transient nuclear translocation of Hes1 within 5 minutes when treated by HC-HA/PTX3. The sustained expression of Hes1 enhances repression the pro-neuronal gene and maintain the low proliferative or quiescence mode of cells. Notch-Hes1 mediated is responsible for activation of HIF1a signaling for phosphorylation STAT3 at Tyr 416. It remains unclear mechanism event responsible for nuclear translation of protein Hes1 but expressed from post-transcriptional event.

### Example 13: HC-HA/PTX3, but not HA and 3D MG, reverted P10 LNC to Pax6 (nuclear positive) neural crest progenitors with angiogenic phenotype

The native limbal niche cells isolated from the limbus has been shown to possess with neural crest and angiogenic potentials. Recently, it has been reported that serially passage of LNC at P10 results in the loss of neural crest progenitor status, which was characterized by downregulation of neural crest progenitor markers such as p75^{NTR}, Musashi-1, Sox2, Nestin, Msx1, and FoxD3, and neuroglial differentiation. Similarly, cells also lose the angiogenic progenitor status characterized by downregulation of FLK-1, PDGFRβ and CD31. It has been demonstrated that the reversal of aged P10 LNC with neural crest potential can be achieved by seeding in soluble HC-HA/PTX3, but not in 3D basement membrane Matrigel. Because HC-HA/PTX3 complex purified from AM consists of HMW HA (>3000 kDa) covalently linked with HC1 and tightly bound PTX3, it was speculated whether HC-HA/PTX3, but not HA, can uniquely reverse the aged LNC to their native neural crest progenitor, p75^{NTR}, Musashi-1, Sox2, Nestin, Msx1, and FoxD3 and vascular progenitor phenotype, FLK-1, PDGFRβ and CD31.

### Experimental Design

Single cells derived from limbal clusters after digestion with 0.25% trypsin and 1mM EDTA (T/E) were seeded at 1x10⁴/cm² in the 6-well plate pre-coated with 5% Matrigel^{™} in MESCM and cultured in humidified 5% CO2 with media change every 3-4 days for total 6-7 days.

Cells treated by HC-HA/PTX3, HA or coated MG were lysed and harvested for RT-PCR. The comparison the mRNA expression of CXCR4, SDF-1 on soluble HC-HA/PTX3 (25 µg/ml) for 15, 30, 60, 120, 240 min, 24h and 48h for neural crest (p75^{NTR}, NGF, Sox2, Musashi-1) and angiogenic markers (PDGFRβ, VEGFR, and CD31).

For cytospin, P10 LNC were harvested at 48h and subjected for IF for p75^{NTR}, Sox2, PDGFRβ, CD31.

Supernatant at 0h, 1h, 2h,4h, 24h, 48h after treating with HC-HA/PTX3 were collected for measurement of VEGF, PDGFRβ and NGF measured in samples of culture medium using a specific ELISA (Quantikine Human VEGF Immunoassay; R&D Systems, Minneapolis, MN). This assay recognized VEGF165, as well as VEGF121. An enzyme immunoassay multi-well reader to read at an emission of 450 nm was used to quantify the results. The inter-assay coefficient of variation was 8.5%, and the sensitivity of the assay was 5 pg/ml.

### Results

Phase contrast microscopy showed that cell aggregation was promoted by soluble HC-HA/PTX3 as early as 60 min but not in HA or coated MG **(****FIG. 44****).** Quantitative RT-PCR revealed significant upregulation of neural crest progenitor markers, p75^{NTR}, NGF, Sox2 and Musashi-1 transcripts and angiogenic progenitor markers receptors PDGFRα/β, VEGFR1/2 and ligands, VEGF, PDGFB, NG2, IGF-1 and CD31 by soluble HC-HA/PTX3 **(****FIGS. 21A** and **21C-****211,** ** # p < 0.01) or soluble HA when compared to 3D MG **(****FIGS. 21A** and **21C****-21I,** ## p<0.01, n=3).

### Example 14: HC-HA/PTX3, but not HA and 3D Matrigel, promoted anti-angiogenesis in HUVEC can be averted by the reversal of P10 LNC

Previously, it has been shown that early passage P4 LNC express neurovascular phenotypes such as vascular pericyte markers (pericyte-EC) (e.g. FLK-1, CD34, CD31, α-SMA, PDGFRβ and NG2) with MSC tri-lineage differentiation and neuro crest marker (Pax6, p75^{NTR}, Musashi-1, Sox2, Msx-1, FoxD3). It has been demonstrated that soluble amniotic extract or HC-HA can suppress endothelial (HUVEC) viability that is CD44 independently and inhibit cell proliferation and suppress HUVEC tube formation (data not shown). Pericytes have been known to stabilize vessels and survival of endothelial cells. Co-culture of mesenchymal stem cells (MSC) with developing vascular endothelial cells reduce the rate of proliferation and apoptosis in endothelial cells. It remains whether the anti-angiogenic of the apoptosis of HUVEC by HC-HA/PTX3 can be averted by the reversal of late passage LNC.

### Experimental Design

5 × 10⁵ /ml HUVEC and P10 LNC (2:1) were seeded in ECGM supplemented with 2% FBS on Matrigel and treated with PBS or 25 µg/ml of HA or HC·HA/PTX3 for 16 h or longer. Fewer tube formations were found in HC·HA-treated cultures based on representative phase contrast micrographs. Total length of tube formations per field in 5 random 100× fields were recorded and compared to control PBS. It was anticipated that HC-HA/PTX3 inhibits tube formation of HUVEC but not HA or non-treated cells on 3D Matrigel at 16 hrs or longer.

5 × 10⁵ /ml HUVEC and/or P10 LNC (2: 1) were seeded in ECGM supplemented with 2% FBS on Matrigel and treated with PBS or 25 µg/ml of HA or HC·HA/PTX3 for 0h, 30 min, 1h, 4h, 24 h and 48h. Caspase-9 was found in cytoplasmic and is an initiator caspase that is part of intrinsic apoptosis pathway. Upon activation, it translocates to the mitochondria. Following mitochondrial disruption, Cytochrome C is released from mitochondria and interact with APAF-1 resulting in Pro-Caspase dimerization. The act of dimerization activates Pro-Caspase-9 leading to activation of Caspase-3. Thus the anti-angiogenesis effect of HC-HA/PTX3 in HUVEC or/and LNC through Caspase 9 apoptosis assay by a Caspase Colorimetric assay 9 (Abcam, ab65608) was examined. To perform the assay, lysis buffer was added to the samples. After incubation, assay was based on detection of chromophore p-nitroanilide (p-NA) after cleavage from the labeled substrate LEHD-P-NA. The p-NA light emission was quantified with a microplate reader at 400 nm. This assay allowed the earliest time of activation of caspase-9 in a time course study.

Because Annexin V is expressed in early stage of apoptotic cells on cell membrane (earlier than caspase-9), GFP-CERTIFIED^{®} Apoptosis/Necrosis detection kit using fluorescent probes were utilized to determine earliest time of expression of Annexin V (should be earlier than Caspase 9) by HC-HA/PTX3. In the presence of LNC, apoptosis in GFP-HUVEC was particularly in the aggregates cells.

### Results

HC-HA/PTX3, but not HA or 3D Matrigel, induced anti-angiogenesis in HUVEC apoptosis in the absence of LNC but increased HUVEC cell survival in the presence of LNC (data not shown).

### Example 15: HC-HA/PTX3, but not HA and 3D Matrigel, promoted quiescence vasculogenic niche in P10 LNC

Because the main driver of sprouting angiogenesis is the arrangement of endothelial cells in tip and stalk cells, it remains unclear whether P10 LNC alone that expressed aforementioned angiogenic progenitor markers, PDGFRβ, VEGR2, IGF-1 and CD31 by soluble HC-HA/PTX3, promotes angiogenesis sprouting on 3D Matrigel or require addition of vascular endothelial cells. It was anticipated the HC-HA/PTX3 but not HA or 3D MG would promote quiescence vasculogenic niche.

### Experimental Design

Single 5 × 10⁵ /ml P10 LNC, GFP-HUVEC or P10 LNC+GFP-HUVEC (1:2) were seeded on 8-wells chambers containing Endothelial Cell Growth Medium 2 (EGM2) supplemented with 10 ng/mL VEGF and 2% FBS with or without soluble HC-HA/PTX3 or soluble HA for 4h, 4, 13 and 30 days. Sprouting diameter on D13 was measured from the both invading edges. Measurements of mean values recorded.

The migration assay was performed in 24-well transwell plate (8 µm pore size, Costar, Kennebunk, ME) by adding Endothelial Cell Growth Medium 2 (EGM2) supplemented with 10 ng/mL VEGF and 2% FBS in the lower compartment while adding 0.1 ml of P10 LNC and GFP-HUVEC in the same media with PBS (vehicle control), HA (25 µg/mL), or HC-HA/PTX3 (25 µg /mL) to the upper compartment that coated with Matrigel. After incubation at 37 °C for 24 h, cells not migrating through the pores were removed by a cotton swab, while cells on the filter facing the lower compartment were fixed with 5% glutaraldehyde, stained with 1% crystal violet, and counted from six random microscopic fields for each control or treatment group. It was anticipated HA or non-treated cells would promote cell invasion but not in HC-HA/PTX3 on 3D Matrigel.

The Click-iT^{®} EdU Assay is an alternative to the BrdU assay. EdU (5-ethynyl-2'-deoxyuridine), is a nucleoside analog of thymidine and is incorporated into DNA during active DNA synthesis. 1 Detection is based on a click reaction,2-5 a copper-catalyzed covalent reaction between an azide and an alkyne.

EdU staining was conducted using Click-iTTM EdU imaging kit (Invitrogen, Carlsbad, CA) according to the manufacturer's protocol. Cell will be cytospin onto slide and fixed with 4% paraformaldehyde in phosphate buffer saline (PBS) for 15 min. After washing twice with 3% bovine serum albumin (BSA) in PBS the sections permeabilize with 0.5% Triton X-100 in PBS for 20 min. The sections were again washed twice with 3% BSA in PBS and then incubated with a Click-iTTM reaction cocktail containing Click-iTTM reaction buffer, CuSO4, Alexa Fluor^{®} 594 Azide, and reaction buffer additive for 30 min while protected from light. The sections were washed once more with 3% BSA in PBS. For subsequent DNA staining, sections were washed once with PBS and then incubated with 5 µg/mL Hoechst 33342 for 30 min. The slides were then washed twice with PBS and coverslip with Vectashield mounting media (Vector Laboratories Inc, Burlingame, CA). All steps were carried out at room temperature. Cell proliferation was anticipated to take place in both sprouting LNC and GFP-HUVEC cells on D10 in HC-HA/PTX3 treated group but not in HA or non-treated groups

### Results

P10 LNC, GFP-HUVEC or P10 LNC + GFP-HUVEC were seeded on 3D MG in EGM medium with or without soluble HA or soluble HC-HA/PTX3. Phase contrast microscopy showed represented cell morphology reunion aggregates at 4h, D4 and D13. **(****FIG. 45A****,** bar = 100 rim) The diameter of sprouting outgrowth was measured from the two sides of invading edges on D13. Normal distribution mean value of sprouting outgrowth diameter at 75% (dark grey column) and 50% (light grey column) compare to control LNC without treatment. (**P<0.01, n=20) **(****FIG. 45B****)** Normal distribution mean value of GFP-HUVEC diameter sprouting outgrowth at 75% (dark grey column,) and 50% (light grey column n=20) compare to control LNC without treatment. (**P<0.01, n=20) **(****FIG. 45C****).**

### Example 16: Activation of CD44ICD and non-canonical TGFβRI synergistically promotes HIF1α signaling by HC-HA/PTX3 and TGFβ1

HIF-1α is a master regulator of cellular processes including regulation of oxygen concentrations, aerobic glycolysis, cell migration, and inflammation. The effects of HC-HA/PTX3 and TGFβ1 on HIF1α signaling was determined.

Briefly, P3 human corneal fibroblasts (HCF) were seeded on plastic with or without immobilized HA, HC-HA/PTX3 complex in DMEM+10%FBS for 72 h, and then in DMEM+ITX for 24 h, and then treated with or without TGFβ1 for 24 h before being harvested for mRNA quantitation of HIF1α.

As seen in **FIG. 46****,** HC-HA/PTX3 upregulates HIF1α mRNA by 3-fold (third bar from left) and 5-fold when TGFβ1 (10 ng/ml) was also added for 24 hours (fourth bar from the left). **P<0.01 and ***P<0.001. N=3. The data suggests a synergistic increase of HIF1 α mRNA in HCF when treated with HC-HA/PTX3 and TGFβ1. Further, the data suggests HIF1α signaling is involved in CD44ICD signaling and non-canonical TGFβRI signaling.

## Claims

1. A fetal support tissue product for use in the therapeutic promotion of vasculogenesis and/or neurovasculogenesis of a tissue, wherein neurovasculogenesis comprises neurogenesis, wherein the tissue comprises endothelial cells and pericytes, wherein the fetal support tissue product comprises native heavy chain-hyaluronan/pentraxin 3 (HC-HA/PTX3) complex, reconstituted HC-HA/PTX3 complex, or a combination thereof, wherein the fetal support tissue product reprograms the pericytes to a first progenitor phenotype and reprograms the endothelial cells to a second progenitor phenotype.

2. The fetal support tissue product for use according to claim 1, wherein the tissue in need of vasculogenesis and/or neurovasculogenesis comprises an ulcer, a wound, a perforation, a burn, a surgical incision, an injury, a fistula, a degenerated tissue, a bone, a tendon, a nerve, a ligament, an intervertebral disc, cardiac tissue, cartilage, an ischemic tissue, ischemic condition comprising cardiac ischemia, ischemic colitis, mesenteric ischemia, brain ischemia, acute limb ischemia, cyanosis, gangrene, neuropathic, or a neurotrophic condition.

3. The fetal support tissue product for use according to claim 1, wherein the tissue further comprises neural crest progenitor cells.

4. The fetal support tissue product for use according to claim 1 or 2, wherein the use prevents necrosis of the tissue.

5. The fetal support tissue product for use according to claim 1, wherein the fetal support tissue product comprises umbilical cord that is substantially free of a vein or artery.

6. The fetal support tissue product for use according to any one of claims 1 to 3 and 5 wherein the use promotes apoptosis, necrosis, or a combination thereof.

7. The fetal support tissue product for use according to claim 6, wherein the fetal support tissue product comprises native HC-HA/PTX3 complex (nHC-HA/PTX3).

8. The fetal support tissue product for use according to any one of claims 1 to 4, wherein the use comprises use of reconstituted HC-HA/PTX3 complex (rcHC-HA/PTX3).

9. The fetal support tissue product for use according to any one of claims 1 to 4 or 8, wherein the rcHC-HA/PTX3 complex consists of high molecular weight (HMW) HA, HC1, HC2 and PTX3.

10. The fetal support tissue product for use according to any one of claims 1 to 9, wherein the fetal support tissue product, the nHC-HA/PTX3 complex or the rcHC-HA/PTX3 complex comprises a pharmaceutically acceptable excipient, carrier, or combination thereof.

11. The fetal support tissue product for use according to any one of claims 1 to 7 or 10, wherein the fetal support tissue product is from placenta, placental amniotic membrane, umbilical cord, umbilical cord amniotic membrane, chorion, amnion-chorion, amniotic stroma, amniotic jelly, Wharton's jelly, amniotic fluid, or a combination thereof.

12. The fetal support tissue product for use according to any one of claims 1 to 7 or 10, wherein the fetal support tissue product is isolated from a fetal support tissue that is frozen or previously frozen.

13. The fetal support tissue product for use according to any one of claims 1 to 7 or 10-12, wherein the fetal support tissue product is ground, pulverized, morselized, a graft, a sheet, micronized, a powder, a homogenate, or an extract.

14. The fetal support tissue product for use according to any one of claims 1 to 7 or 10-12, wherein the fetal support tissue product comprises umbilical cord amniotic membrane (UCAM).

15. The fetal support tissue product for use according to claim 14, wherein UCAM further comprises Wharton's jelly.

## Patentansprüche

1. Fötales Stützgewebeprodukt zur Verwendung bei der therapeutischen Förderung der Vaskulogenese und/oder Neurovaskulogenese eines Gewebes, wobei die Neurovaskulogenese Neurogenese umfasst, wobei das Gewebe Endothelzellen und Perizyten umfasst, wobei das fötale Stützgewebeprodukt einen nativen Hyaluronan/Pentraxin-3-(HC-HA/PTX3)-Komplex der schweren Kette, einen rekonstituierten HC-HA/PTX3-Komplex oder eine Kombination davon umfasst, wobei das fötale Stützgewebeprodukt die Perizyten auf einen ersten Vorläuferphänotyp umprogrammiert und die Endothelzellen auf einen zweiten Vorläuferphänotyp umprogrammiert.

2. Fötales Stützgewebeprodukt zur Verwendung gemäß Anspruch 1, wobei das Gewebe, das einer Vaskulogenese und/oder Neurovaskulogenese bedarf, ein Geschwür, eine Wunde, eine Perforation, eine Verbrennung, einen chirurgischen Schnitt, eine Verletzung, eine Fistel, ein degeneriertes Gewebe, einen Knochen, eine Sehne, einen Nerv, ein Band, eine Bandscheibe, Herzgewebe, Knorpel, ein ischämisches Gewebe, einen ischämischen Zustand, umfassend kardiale Ischämie, ischämische Kolitis, mesenteriale Ischämie, Ischämie des Gehirns, akute Ischämie der Gliedmaßen, Zyanose, Gangrän, neuropathische oder einen neurotrophen Zustand umfasst.

3. Fötales Stützgewebeprodukt zur Verwendung gemäß Anspruch 1, wobei das Gewebe ferner Neuralleistenvorläuferzellen umfasst.

4. Fötales Stützgewebeprodukt zur Verwendung gemäß Anspruch 1 oder 2, wobei die Verwendung Nekrose des Gewebes verhindert.

5. Fötales Stützgewebeprodukt zur Verwendung gemäß Anspruch 1, wobei das fötale Stützgewebeprodukt Nabelschnur umfasst, die im Wesentlichen frei von einer Vene oder Arterie ist.

6. Fötales Stützgewebeprodukt zur Verwendung gemäß einem der Ansprüche 1 bis 3 und 5, wobei die Verwendung Apoptose, Nekrose oder eine Kombination davon fördert.

7. Fötales Stützgewebeprodukt zur Verwendung gemäß Anspruch 6, wobei das fötale Stützgewebeprodukt nativen HC-HA/PTX3-Komplex (nHC-HA/PTX3) umfasst.

8. Fötales Stützgewebeprodukt zur Verwendung gemäß einem der Ansprüche 1 bis 4, wobei die Verwendung die Verwendung eines rekonstituierten HC-HA/PTX3-Komplexes (rcHC-HA/PTX3) umfasst.

9. Fötales Stützgewebeprodukt zur Verwendung gemäß einem der Ansprüche 1 bis 4 oder 8, wobei der rcHC-HA/PTX3-Komplex aus HA, HC1, HC2 und PTX3 mit hohem Molekulargewicht (HMW) besteht.

10. Fötales Stützgewebeprodukt zur Verwendung gemäß einem der Ansprüche 1 bis 9, wobei das fötale Stützgewebeprodukt, der nHC-HA/PTX3-Komplex oder der rcHC-HA/PTX3-Komplex einen pharmazeutisch unbedenklichen Hilfsstoff, Träger oder eine Kombination davon umfasst.

11. Fötales Stützgewebeprodukt zur Verwendung gemäß einem der Ansprüche 1 bis 7 oder 10, wobei das fötale Stützgewebeprodukt aus Plazenta, Plazenta-Amnionmembran, Nabelschnur, Nabelschnur-Amnionmembran, Chorion, Amnion-Chorion, Amnionstroma, Amnionsulze, Whartonsche Sulze, Fruchtwasser oder einer Kombination davon ist.

12. Fötales Stützgewebeprodukt zur Verwendung gemäß einem der Ansprüche 1 bis 7 oder 10, wobei das fötale Stützgewebeprodukt aus einem fötalen Stützgewebe isoliert ist, das gefroren ist oder zuvor gefroren war.

13. Fötales Stützgewebeprodukt zur Verwendung gemäß einem der Ansprüche 1 bis 7 oder 10-12, wobei das fötale Stützgewebeprodukt gemahlen, pulverisiert, zerkleinert, ein Transplantat, eine Folie, mikronisiert, ein Pulver, ein Homogenat oder ein Extrakt ist.

14. Fötales Stützgewebeprodukt zur Verwendung gemäß einem der Ansprüche 1 bis 7 oder 10-12, wobei das fötale Stützgewebeprodukt Nabelschnur-Amnionmembran (UCAM) umfasst.

15. Fötales Stützgewebeprodukt zur Verwendung gemäß Anspruch 14, wobei UCAM ferner Whartonsche Sulze umfasst.

## Revendications

1. Produit de tissu de support fœtal destiné à être utilisé dans la promotion thérapeutique de la vasculogenèse et/ou de la neurovasculogenèse d'un tissu, ladite neurovasculogenèse comprenant la neurogenèse, ledit tissu comprenant des cellules endothéliales et des péricytes, ledit produit de tissu de support fœtal comprenant un complexe chaîne lourde-hyaluronane/pentraxine 3 (HC-HA/PTX3) natif, un complexe HC-HA/PTX3 reconstitué ou une combinaison de ceux-ci, ledit produit de tissu de support fœtal reprogrammant les péricytes en un premier phénotype progéniteur et reprogrammant les cellules endothéliales en un second phénotype progéniteur.

2. Produit de tissu de support fœtal destiné à être utilisé selon la revendication 1, ledit tissu nécessitant une vasculogenèse et/ou une neurovasculogenèse comprenant un ulcère, une plaie, une perforation, une brûlure, une incision chirurgicale, une blessure, une fistule, un tissu dégénéré, un os, un tendon, un nerf, un ligament, un disque intervertébral, un tissu cardiaque, un cartilage, un tissu ischémique, un état ischémique comprenant une ischémie cardiaque, une colite ischémique, une ischémie mésentérique, une ischémie cérébrale, une ischémie aiguë des membres, une cyanose, une gangrène, une neuropathie ou un état neurotrophique.

3. Produit de tissu de support fœtal destiné à être utilisé selon la revendication 1, ledit tissu comprenant en outre des cellules progénitrices de crête neurale.

4. Produit de tissu de support fœtal destiné à être utilisé selon la revendication 1 ou 2, ladite utilisation empêchant la nécrose du tissu.

5. Produit de tissu de support fœtal destiné à être utilisé selon la revendication 1, ledit produit de tissu de support fœtal comprenant un cordon ombilical qui est sensiblement exempt d'une veine ou d'une artère.

6. Produit de tissu de support fœtal destiné à être utilisé selon l'une quelconque des revendications 1 à 3 et 5, ladite utilisation favorisant l'apoptose, la nécrose ou une combinaison de celles-ci.

7. Produit de tissu de support fœtal destiné à être utilisé selon la revendication 6, ledit produit de tissu de support fœtal comprenant un complexe HC-HA/PTX3 natif (nHC-HA/PTX3).

8. Produit de tissu de support fœtal destiné à être utilisé selon l'une quelconque des revendications 1 à 4, ladite utilisation comprenant l'utilisation d'un complexe HC-HA/PTX3 reconstitué (rcHC-HA/PTX3).

9. Produit de tissu de support fœtal destiné à être utilisé selon l'une quelconque des revendications 1 à 4 ou 8, ledit complexe rcHC-HA/PTX3 étant constitué de HA, HC1, HC2 et PTX3 de poids moléculaire élevé (HMW).

10. Produit de tissu de support fœtal destiné à être utilisé selon l'une quelconque des revendications 1 à 9, ledit produit de tissu de support fœtal, ledit complexe nHC-HA/PTX3 ou ledit complexe rcHC-HA/PTX3 comprenant un excipient, un vecteur ou une combinaison de ceux-ci pharmaceutiquement acceptable.

11. Produit de tissu de support fœtal destiné à être utilisé selon l'une quelconque des revendications 1 à 7 ou 10, ledit produit de tissu de support fœtal provenant du placenta, d'une membrane amniotique placentaire, d'un cordon ombilical, d'une membrane amniotique de cordon ombilical, d'un chorion, d'un amnios-chorion, d'un stroma amniotique, d'une gelée amniotique, d'une gelée de Wharton, d'un liquide amniotique ou d'une combinaison de ceux-ci.

12. Produit de tissu de support fœtal destiné à être utilisé selon l'une quelconque des revendications 1 à 7 ou 10, ledit produit de tissu de support fœtal étant isolé à partir d'un tissu de support fœtal qui est congelé ou préalablement congelé.

13. Produit de tissu de support fœtal destiné à être utilisé selon l'une quelconque des revendications 1 à 7 ou 10 à 12, ledit produit de tissu de support fœtal étant broyé, pulvérisé, morcelé, une greffe, une feuille, micronisé, une poudre, un homogénat ou un extrait.

14. Produit de tissu de support fœtal destiné à être utilisé selon l'une quelconque des revendications 1 à 7 ou 10 à 12, ledit produit de tissu de support fœtal comprenant une membrane amniotique de cordon ombilical (UCAM).

15. Produit de tissu de support fœtal destiné à être utilisé selon la revendication 14, ledit UCAM comprenant en outre de la gelée de Wharton.
